(19) Europäisches Patentamt
European Patent Office
Office européen des brevets

(11) Veröffentlichungsnummer: **0 031 796**
**B1**

(12) # EUROPÄISCHE PATENTSCHRIFT

(45) Veröffentlichungstag der Patentschrift: **23.01.85**

(21) Anmeldenummer: **80810393.1**

(22) Anmeldetag: **15.12.80**

(51) Int. Cl.[4]: **C 07 D 239/26**, C 07 D 263/56, C 08 K 5/34, C 08 K 5/35, D 06 L 3/12, C 07 C 69/618, C 07 C 121/52, C 07 F 9/65, C 07 D 231/12, C 07 D 471/04, C 07 D 498/04 // (C07D471/04, 249/00, 221/00), (C07D498/04, 263/00, 221/00)

(54) **4-Heterocyclyl-4'-vinyl-stilbene.**

(30) Priorität: **21.12.79 CH 11402/79**
**18.09.80 CH 7008/80**

(43) Veröffentlichungstag der Anmeldung: **08.07.81 Patentblatt 81/27**

(45) Bekanntmachung des Hinweises auf die Patenterteilung: **23.01.85 Patentblatt 85/4**

(84) Benannte Vertragsstaaten: **CH DE FR GB IT LI**

(56) Entgegenhaltungen:
**DE - A - 2 525 684**
**DE - A - 2 539 537**
**DE - A - 2 602 750**
**DE - A - 2 848 193**

**Ullmanns Encyklopädie der technischen Chemie, Bd. 17 (1979), S. 461-470**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Burdeska, Kurt, Dr., Paracelsusstrasse 64, CH-4058 Basel (CH)**
Erfinder: **Kabas, Guglielmo, Dr., Im Egg 36, CH-4147 Aesch (CH)**
Erfinder: **Weber, Kurt, Dr., Rennweg 98, CH-4052 Basel (CH)**

**EP 0 031 796 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung sind 4-Heterocyclyl-4'-vinyl-stilbene, Verfahren zu deren Herstellung sowie deren Verwendung zum optischen Aufhellen von natürlichen und synthetischen organischen Materialien.

In Ullmanns Encyklopädie der technischen Chemie Bd, 17 (1979), S, 461—470 sind Stilbenverbindungen beschrieben, welche keine endständige, negative Reste tragende Vinylgruppe aufweisen.

Es sind ferner aus der Literatur z. B. DE-A 2 602 750 4,4'-Divinylstilbene und aus den DE-A 2 525 684, 2 539 537 und 2 848 193 gewisse 4-Heterocyclyl-stilbene bekannt, welche unbefriedigende Aufzieheigenschaften besitzen.

Aufgabe der vorliegenden Erfindung war, neue optische Aufheller mit besseren Aufzieheigenschaften zu beschaffen.

Es wurde nun gefunden, daß Heterocyclylstilbene mit einem Vinylsubstituent an einem der terminalen Phenylreste ausgiebiger sind als bis jetzt bekannte Heterocyclylstilbene.

Die neuen 4-Heterocyclyl-4'-vinyl-stilbene entsprechen der Formel

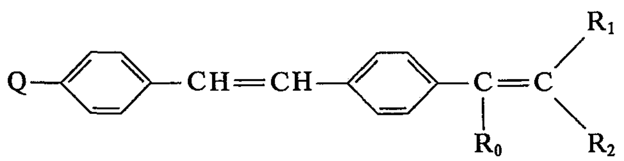

(1)

worin Q einen monocyclischen 5- oder 6gliedrigen unsubstituierten oder nicht-chromophor substituierten, 0, 1 oder 2 ankondensierte Benzolringe aufweisenden aromatischen heterocyclischen Ring oder einen bicyclischen 9gliedrigen aromatischen heterocyclischen Ring, $R_0$ Wasserstoff, unsubstituiertes oder nicht-chromophor substituiertes Alkyl, $R_1$ Wasserstoff, unsubstituiertes oder nicht-chromophor substituiertes Alkyl, Alkoxycarbonyl, Carbamoyl oder Sulfonamido; Alkenyl, Carboxy, Alkyl-, Aryl- oder Aralkylsulfonyl, Cyano, Sulfo oder Phosphonsäuredialkylester und $R_2$ Wasserstoff, unsubstituiertes oder nicht-chromophor substituiertes Alkyl oder Alkenyl bedeuten, wobei höchstens eines der Symbole $R_1$ und $R_2$ Wasserstoff bedeutet.

Nicht-chromophore Substituenten sind beispielsweise Halogen, gegebenenfalls substituiertes Alkyl, gegebenenfalls substituiertes Alkenyl, Aryl, Aralkyl, gegebenenfalls substituiertes Alkoxy, Alkoxycarbonyl, gegebenenfalls substituiertes Aminocarbonyl, Cyano, Alkylsulfonyl, Alkoxysulfonyl, gegebenenfalls substituiertes Aminosulfonyl, Acyl, Acylamino, Hydroxy, Aryloxy, Aralkyloxy, Alkenyloxy, Aryloxycarbonyl, Aralkyloxycarbonyl, Carboxy, Acyloxy oder Trifluormethyl.

Alkyl ist insbesondere $C_1$—$C_4$-Alkyl, das durch Hydroxy, $C_1$—$C_4$-Alkoxy, Cyano, Carboxy, $C_1$—$C_4$-Alkoxycarbonyl, Aminocarbonyl oder Chlor monosubstituiert sein kann.

Alkenyl ist insbesondere $C_2$—$C_5$-Alkenyl, das durch Hydroxy, $C_1$—$C_4$-Alkoxy, Cyano, Carboxy, $C_1$—$C_4$-Alkoxycarbonyl oder Chlor monosubstituiert sein kann.

Halogen ist insbesondere Fluor, Chlor oder Brom, vorzugsweise Chlor. Aryl ist insbesondere gegebenenfalls durch $C_1$—$C_4$-Alkyl, Chlor, Brom oder $C_1$—$C_4$-Alkoxy substituiertes Phenyl.

Aralkyl ist insbesondere Phenyl-$C_1$—$C_4$-Alkyl, das im Phenylkern noch durch Chlor, Methyl oder Methoxy substituiert sein kann.

Alkoxy ist insbesondere $C_1$—$C_4$-Alkoxy oder ein Rest der Formel

$$—(OCH_2—CH_2)_m—OR$$

wobei R Wasserstoff oder $C_1$—$C_4$-Alkyl und m eine ganze Zahl von 1 bis 20 bedeutet.

Cycloalkyloxy ist insbesondere Cyclopentyloxy und Cyclohexyloxy.

Acyl ist insbesondere $C_1$—$C_4$-Alkylcarbonyl, $C_1$—$C_4$-Alkylsulfonyl, gegebenenfalls durch Methyl, Methoxy oder Chlor substituiertes Benzoyl oder gegebenenfalls durch Methyl, Methoxy oder Chlor substituiertes Benzolsulfonyl.

Als Substituenten der Aminocarbonyl- und Aminosulfonylreste kommen insbesondere $C_1$—$C_4$-Alkyl, gegebenenfalls durch Methyl, Methoxy oder Chlor substituiertes Phenyl oder Phenyl-$C_1$—$C_4$-Alkyl in Betracht.

Im Rahmen der Verbindungen der Formel (1) sind von Interesse die Verbindungen der Formel

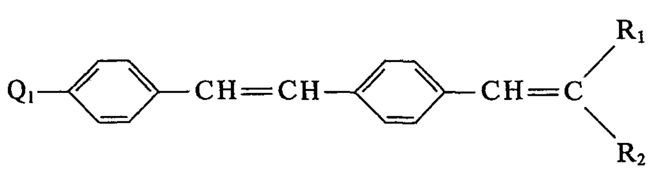

(2)

worin $R_1$ und $R_2$ die oben angegebene Bedeutung haben und $Q_1$ einen Rest der Formel

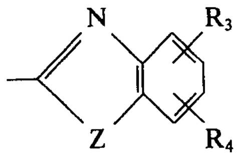
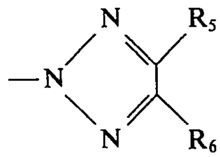
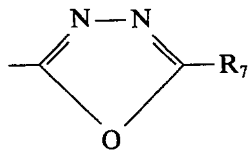
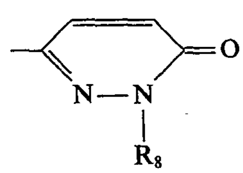
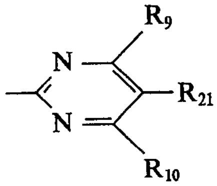
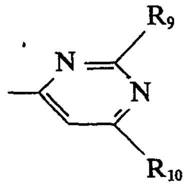
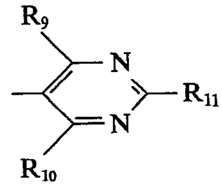
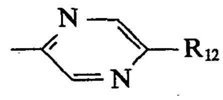
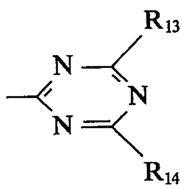
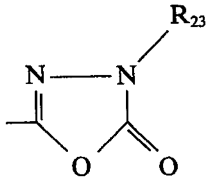
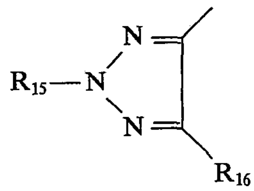
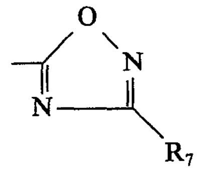
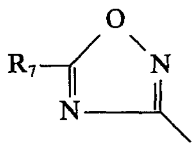
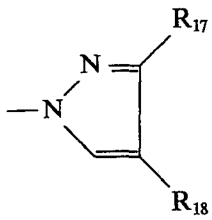
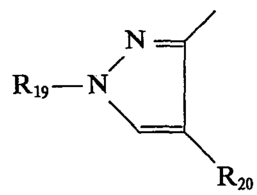
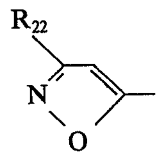
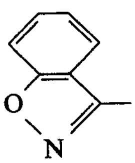
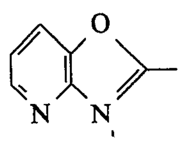
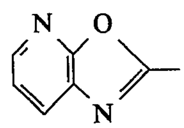
oder
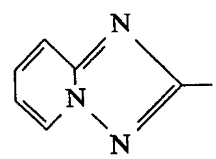

bedeuten, in welchen Resten

$R_3$ Wasserstoff, Chlor, $C_1$—$C_4$-Alkyl, Phenyl-$C_1$—$C_3$-Alkyl, Cyclohexyl, Phenyl, $C_1$—$C_4$-Alkoxy, $C_1$—$C_4$-Alkylsulfonyl, $C_1$—$C_4$-Alkoxycarbonyl, Cyano oder Carboxy oder zusammen mit $R_4$ einen gegebenenfalls durch 1 bis 4 Methylgruppen substituierten ankondensierten 1-Cyclopenten-, 1-Cyclohexen- oder Benzolring,

$R_4$ Wasserstoff, Chlor oder $C_1$—$C_4$-Alkyl oder zusammen mit $R_3$ einen gegebenenfalls durch 1 bis 4 Methylgruppen substituierten ankondensierten 1-Cyclopenten-, 1-Cyclohexen- oder Benzolring,

$R_5$ Wasserstoff, $C_1$—$C_4$-Alkyl, Cyano, COOR, worin R für $C_1$—$C_4$-Alkyl steht, Phenyl oder Styryl oder zusammen mit $R_6$ einen gegebenenfalls durch $C_1$—$C_4$-Alkyl, $C_1$—$C_4$-Alkoxy oder Chlor substituierten ankondensierten Benzolring oder einen ankondensierten Naphthalinring,

$R_6$ Wasserstoff, $C_1$—$C_4$-Alkyl, Phenyl oder zusammen mit $R_5$ einen gegebenenfalls durch $C_1$—$C_4$-Alkyl, $C_1$—$C_4$-Alkoxy oder Chlor substituierten ankondensierten Benzolring oder einen ankondensierten Naphthalinring,

$R_7$ unsubstituiertes oder nicht-chromophor substituiertes $C_1$—$C_4$-Alkyl, gegebenenfalls durch $C_1$—$C_4$-Alkyl, $C_1$—$C_4$-Alkoxy, $C_1$—$C_4$-Alkoxycarbonyl, Cyano oder Chlor substituiertes Phenyl, Styryl, Biphenylyl oder Naphthyl,

$R_8$ Wasserstoff, $C_1$—$C_4$-Alkyl oder unsubstituiertes oder durch Chlor oder Methyl substituiertes Phenyl,

$R_9$ und $R_{10}$ unabhängig voneinander Wasserstoff, $C_1$—$C_4$-Alkyl, unsubstituiertes oder durch Chlor oder Methyl substituiertes Phenyl, $C_1$—$C_4$-Alkoxy, $C_3$—$C_8$-Alkoxyalkoxy, unsubstituiertes oder durch Chlor oder Methyl substituiertes Phenoxy, Chlor, $C_1$—$C_4$-Alkylthio, Phenylthio, $C_1$—$C_4$-Alkylamino, Di-$C_1$—$C_4$-alkylamino, Morpholino, Piperidino, Piperazino, Pyrrolidino oder Anilino,

$R_{11}$ Wasserstoff, $C_1$—$C_4$-Alkyl oder unsubstituiertes oder durch Chlor oder Methyl substituiertes Phenyl,

$R_{12}$ $C_1$—$C_4$-Alkoxy, $C_3$—$C_8$-Alkoxyalkoxy, $C_1$—$C_4$-Alkylthio, unsubstituiertes oder durch Chlor oder Methyl substituiertes Phenoxy, Cycloalkoxy, $C_1$—$C_4$-Alkylthio, unsubstituiertes oder durch Chlor

oder Methyl substituiertes Phenylthio, $C_1-C_4$-Alkylamino, Di-$C_1-C_4$-alkylamino, Morpholino, Piperidino, Piperazino, Pyrrolidino oder Anilino,

$R_{13}$ und $R_{14}$ Wasserstoff, Halogen, $C_1-C_4$-Alkoxy, Phenyl, Aralkoxy, Cycloalkoxy, Aryloxy, $C_1-C_4$-Alkylmercapto, $C_1-C_4$-Alkylamino, Di-$C_1-C_4$-Alkylamino, Morpholino, Piperidino, Piperazino, Pyrrolidino, Arylamino oder $C_1-C_4$-Alkyl,

$R_{15}$ unsubstituiertes oder durch Chlor oder $C_1-C_4$-Alkyl substituiertes Phenyl,

$R_{16}$ Wasserstoff oder $C_1-C_4$-Alkyl,

$R_{17}$ unsubstituiertes oder durch Chlor oder $C_1-C_4$-Alkyl substituiertes Phenyl,

$R_{18}$ Wasserstoff oder $C_1-C_4$-Alkyl,

$R_{19}$ unsubstituiertes oder durch Chlor oder $C_1-C_4$-Alkyl substituiertes Phenyl,

$R_{20}$ Wasserstoff oder $C_1-C_4$-Alkyl,

$R_{21}$ Wasserstoff oder $C_1-C_4$-Alkyl,

$R_{22}$ unsubstituiertes oder durch $C_1-C_4$-Alkoxy substituiertes Phenyl,

$R_{23}$ Wasserstoff, $C_1-C_4$-Alkyl oder unsubstituiertes oder durch $C_1-C_4$-Alkyl, Halogen oder Cyano substituiertes Phenyl und

Z O, S oder NX bedeuten, worin X für Wasserstoff, $C_1-C_4$-Alkyl, Acetyl, Benzoyl oder Phenyl steht.

Bevorzugte Verbindungen entsprechen der Formel

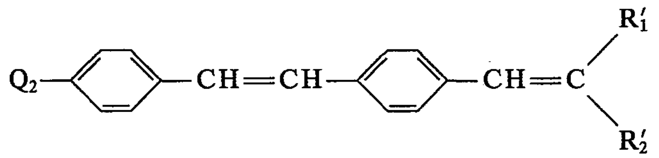

(3)

worin $R'_1$ Wasserstoff, $C_1-C_6$-Alkyl, durch $C_1-C_4$-Alkoxy oder $C_2-C_5$-Alkoxycarbonyl substituiertes $C_1-C_6$-Alkyl, $C_2-C_4$-Alkenyl, Cyano, $COOR°$ worin $R°$ für $C_1-C_4$-Alkyl, $C_1-C_4$-Alkoxy-$C_2-C_4$-alkyl, $C_1-C_4$-Alkoxy-$C_2-C_4$-alkoxy-$C_2-C_4$-alkyl, $C_1-C_4$-Alkoxy-$C_2-C_4$-alkoxy-$C_2-C_4$-alkoxy-$C_2-C_4$-alkyl, Tetrahydro-2-furyl-methyl, 1,4-Dioxa-2-cyclohexyl-methyl, Allyl, $C_1-C_4$-alkylamino-$C_1-C_4$-alkyl oder Di-$C_1-C_4$-alkylamino-$C_1-C_4$-alkyl steht,

$-CON(R')(R'')$, worin R' für Wasserstoff, $C_1-C_6$-Alkyl, durch Hydroxy, $C_1-C_4$-Alkoxy, $C_2-C_6$-Hydroxyalkoxy, $-SO_3M$ oder Mono- oder Di-$C_1-C_4$-alkylamino substituiertes $C_2-C_6$-Alkyl, Cyclohexyl, Benzyl oder Phenäthyl und R'' für Wasserstoff, $C_1-C_6$-Alkyl durch Hydroxy, $C_1-C_4$-Alkoxy oder $C_2-C_6$-Hydroxyalkoxy substituiertes $C_2-C_6$-Alkyl stehen,

$-SO_2N(R')(R'')$ worin R' und R'' die vorstehende Bedeutung haben, $-SO_3M$, $R_x-SO_2-$ worin $R_x$ für $C_1-C_6$-Alkyl, durch $C_1-C_4$-Alkoxy oder $C_1-C_4$-Alkoxy-$C_2-C_6$-alkoxy substituiertes $C_2-C_6$-Alkyl, Phenyl oder Benzyl steht, oder Phosphonsäuredialkylester,

$R'_2$ Wasserstoff oder $C_1-C_6$-Alkyl,

M Wasserstoff oder ein bei optischen Aufhellern übliches, nicht-chromophores Kation, und

$Q_2$ einen Rest der Formel

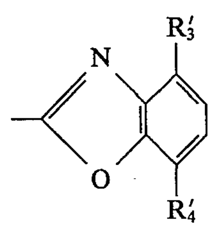
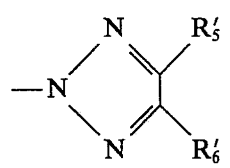
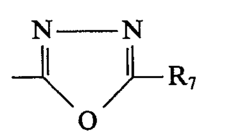
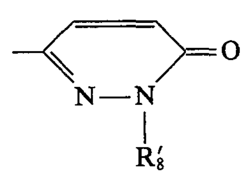

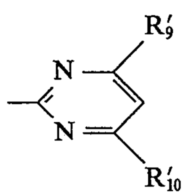
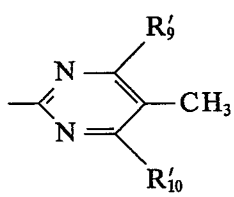
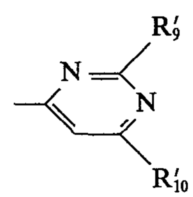
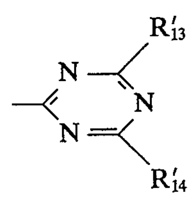

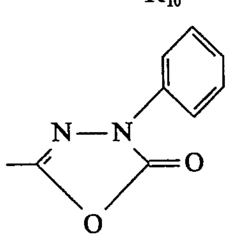
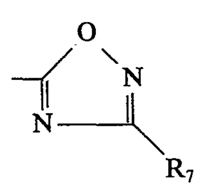
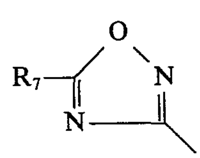
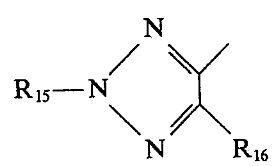

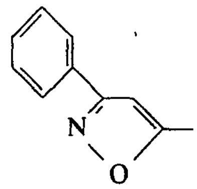 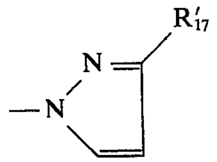 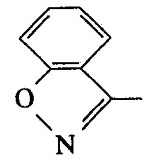 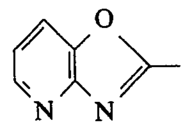 oder

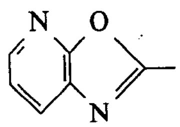

bedeuten, worin

$R_3'$ Wasserstoff, Methyl, Chlor oder $C_1$—$C_4$-Alkoxy,
$R_4'$ Wasserstoff, Chlor, $C_1$—$C_4$-Alkyl, Phenyl, $C_1$—$C_4$-Alkoxy oder Phenoxy,
$R_5'$ Wasserstoff, $C_1$—$C_4$-Alkyl, Phenyl oder Styryl oder zusammen mit $R_6'$ einen ankondensierten unsubstituierten oder durch $C_1$—$C_4$-Alkyl, $C_1$—$C_4$-Alkoxy oder Chlor substituierten Benzolring oder einen ankondensierten Naphthalinring,
$R_6'$ Wasserstoff, $C_1$—$C_4$-Alkyl, Phenyl oder zusammen mit $R_5'$ einen ankondensierten unsubstituierten oder durch $C_1$—$C_4$-Alkyl, $C_1$—$C_4$-Alkoxy oder Chlor substituierten Benzolring oder einen ankondensierten Naphthalinring,
$R_7$ unsubstituiertes oder nicht-chromophor substituiertes $C_1$—$C_4$-Alkyl, gegebenenfalls durch $C_1$—$C_4$-Alkyl, $C_1$—$C_4$-Alkoxy, $C_1$—$C_4$-Alkoxycarbonyl, Cyano oder Chlor substituiertes Phenyl, Styryl, Biphenylyl oder Naphthyl,
$R_8'$ unsubstituiertes $C_1$—$C_4$-Alkyl oder Phenyl,
$R_9'$ Wasserstoff, unsubstituiertes $C_1$—$C_4$-Alkyl, unsubstituiertes oder durch Chlor oder Methyl substituiertes Phenyl, Chlor, $C_1$—$C_4$-Alkoxy, $C_3$—$C_5$-Alkoxyalkoxy, unsubstituiertes oder durch Chlor oder Methyl substituiertes Phenoxy, $C_1$—$C_4$-Alkylthio oder Phenylthio,
$R_{10}'$ Wasserstoff, unsubstituiertes $C_1$—$C_4$-Alkyl, $C_1$—$C_4$-Alkoxy, unsubstituiertes oder durch Chlor oder Methyl substituiertes Phenyl, unsubstituiertes oder durch Chlor oder Methyl substituiertes Phenoxy, $C_1$—$C_4$-Alkylthio, Phenylthio oder Chlor,
$R_{13}'$ und $R_{14}'$ Wasserstoff, $C_1$—$C_4$-Alkylamino, Di-$C_1$—$C_4$-alkylamino, Phenyl, Morpholino, Piperidino, Phenylamino oder einen Rest $-(OCH_2-CH_2)_q-OY$ worin Y für Wasserstoff, $C_1$—$C_4$-Alkyl, Benzyl oder Phenyl und q für eine ganze Zahl von 0 bis 7 stehen,
$R_{15}$ unsubstituiertes oder durch Chlor oder $C_1$—$C_4$-Alkyl substituiertes Phenyl,
$R_{16}$ Wasserstoff oder $C_1$—$C_4$-Alkyl und
$R_{17}'$ $C_1$—$C_4$-Alkyl bedeuten.

Weitere bevorzugte Verbindungen der Formel (1) sind solche

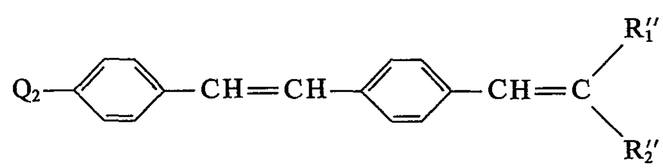 (4)

worin $R_1''$ Cyano, COOR worin R für $C_1$—$C_4$-Alkyl, $C_1$—$C_4$-Alkoxy-$C_2$—$C_4$-alkyl, $C_1$—$C_4$-Alkoxy-$C_2$—$C_4$-alkoxy-$C_2$—$C_4$-alkyl, $C_1$—$C_4$-Alkoxy-$C_2$—$C_4$-alkoxy-$C_2$—$C_4$-alkoxy-$C_2$—$C_4$-alkyl, 2,3,4,5-Tetrahydro-2-furyl-methyl, 1,4-Dioxa-2-cyclohexyl-methyl steht,
CON(R')(R''), worin R' für Wasserstoff, $C_1$—$C_6$-Alkyl, durch Hydroxy, $C_1$—$C_4$-Alkoxy, $C_2$—$C_6$-Hydroxyalkoxy, $-SO_3M$ oder Mono- oder Di-$C_1$—$C_4$-alkylamino substituiertes $C_2$—$C_6$-Alkyl, Cycloalkyl, Benzyl oder Phenäthyl und R'' für Wasserstoff, $C_1$—$C_4$-Alkyl, durch Hydroxy, $C_1$—$C_4$-Alkoxy oder $C_2$—$C_6$-Hydroxyalkoxy substituiertes $C_2$—$C_6$-Alkyl stehen, $R_x-SO_2-$ worin $R_x$ für $C_1$—$C_6$-Alkyl, durch $C_1$—$C_4$-Alkoxy oder $C_1$—$C_4$-Alkoxy-$C_2$—$C_6$-alkoxy substituiertes $C_2$—$C_6$-Alkyl, Phenyl oder Benzyl steht oder Phosphonsäuredialkylester,
$R_2''$ Wasserstoff oder $C_1$—$C_4$-Alkyl,
M Wasserstoff oder ein bei optischen Aufhellern übliches, nicht-chromophores Kation bedeuten und $Q_2$ die oben angegebene Bedeutung hat.

M steht vorteilhaft für Wasserstoff, ein Alkalimetallkation oder für ein unsubstituiertes oder substituiertes Ammoniumkation, z. B. Mono-, Di- oder Tri-($C_1$—$C_4$-alkyl)- oder ($C_2$—$C_4$-alkanol)-ammonium, wie z. B. Mono-, Di- oder Triäthanol- oder -isopropanolammonium, vorzugsweise Wasserstoff oder Natrium.

Besonders bevorzugte erfindungsgemäße Verbindungen entsprechen

A) der Formel

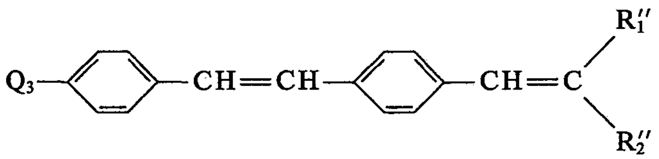

(5)

worin $R_1''$ und $R_2''$ die oben angegebene Bedeutung haben und $Q_3$ einen Rest der Formel

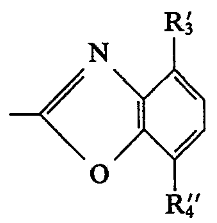

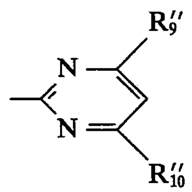

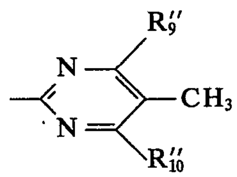

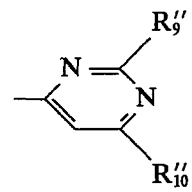

oder

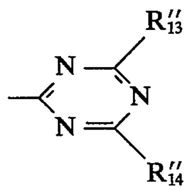

bedeuten, worin

$R_3'$ Wasserstoff, Methyl, Chlor oder $C_1$—$C_4$-Alkoxy,
$R_4''$ Wasserstoff, Methyl, Chlor oder $C_1$—$C_4$-Alkyl,
$R_9''$ Wasserstoff, Methyl, Phenyl, $C_1$—$C_3$-Alkoxy, Methoxyäthoxy oder Phenoxy,
$R_{10}''$ unsubstituiertes $C_1$- oder $C_2$-Alkyl, $C_1$—$C_3$-Alkoxy oder Phenoxy und
$R_{13}''$ und $R_{14}''$ je Wasserstoff oder einen Rest $-(OCH_2-CH_2)_r-OY'$ worin
Y' für $C_1$—$C_4$-Alkyl und r für eine ganze Zahl von 0 bis 2 stehen, bedeuten,

B) der Formel

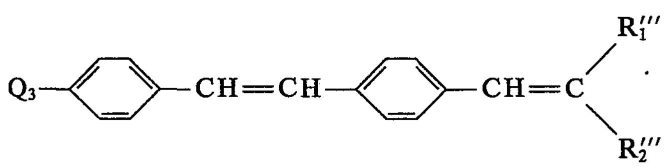

(6)

worin $R_1'''$ Cyano, COOR worin R für $C_1$—$C_4$-Alkyl, $C_1$—$C_4$-Alkoxy-$C_2$—$C_4$-alkyl, $C_1$—$C_4$-Alkoxy-$C_2$—$C_4$-alkoxy-$C_2$—$C_4$-alkyl, $C_1$—$C_4$-Alkoxy-$C_2$—$C_4$-alkoxy-$C_2$—$C_4$-alkoxy-$C_2$—$C_4$-alkyl, 2,3,4,5-Tetrahydro-2-furyl-methyl oder 1,4-Dioxa-2-cyclohexyl-methyl steht, oder $R_x-SO_2-$, worin $R_x$ für $C_1$—$C_4$-Alkyl, durch $C_1$—$C_4$-Alkoxy oder $C_1$—$C_4$-Alkoxy-$C_2$—$C_6$-Alkoxy substituiertes $C_2$—$C_6$-Alkyl, Phenyl oder Benzyl steht, und $R_2'''$ Wasserstoff oder $C_1$—$C_4$-Alkyl bedeutet, und $Q_3$ die oben angegebene Bedeutung hat, sowie

C) der Formel

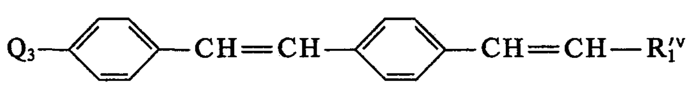

(7)

worin $R_1^{IV}$ Cyano oder COOR' bedeutet, worin R' $C_1$—$C_4$-Alkyl oder $C_1$—$C_4$-Alkoxy-$C_2$—$C_6$-alkyl ist und $Q_3$ die oben angegebene Bedeutung hat.

Die erfindungsgemäßen 4-Heterocyclyl-4'-vinyl-stilbene der Formel (1) können dadurch hergestellt werden, daß man in einem organischen Lösungsmittel und in Gegenwart basischer Kondensationsmittel eine Verbindung der Formel

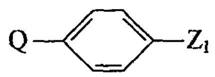
(8)

mit einer Verbindung der Formel

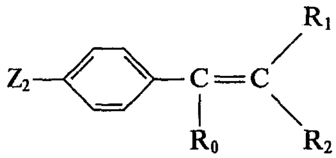
(9)

kondensiert, worin Q, $R_1$, $R_1$ und $R_2$ die unter Formel (1) angegebene Bedeutung haben und von $Z_1$ und $Z_2$ das eine die OHC-Gruppe und das andere eine Gruppierung der Formel

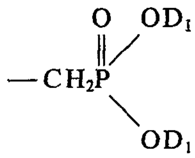 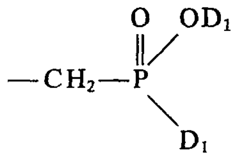 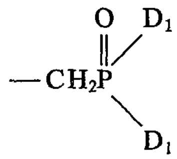 oder 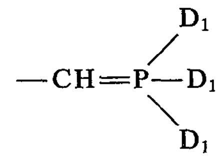

bedeuten, worin $D_1$ einen unsubstituierten oder substituierten Alkyl-, Aryl-, Cycloalkyl- oder Arylkylrest bedeutet.

Als Lösungsmittel verwendet man vorteilhafterweise indifferente Lösungsmittel, beispielsweise Kohlenwasserstoffe wie Toluol oder Xylol oder Alkohole wie Methanol, Äthanol, Isopropanol, Butanol, Glykol, Glykoläther wie 2-Methoxyäthanol, Hexanol, Cyclohexanol, Cyclooctanol, ferner Äther wie Diisopropyläther, Dioxan, Tetrahydrofuran, weiterhin Formamide oder N-Methylpyrrolidon. Besonders geeignet sind dipolare organische Lösungsmittel wie Dimethylformamid und Dimethylsulfoxid.

Als Kondensationsmittel kommen stark basische Verbindungen in Betracht wie Alkali- und Erdalkalimetallhydroxide, Alkali- und Erdalkaliamide und Alkali- und Erdalkalimetallalkoholate, beispielsweise Kaliumhydroxid, Natriumhydroxid, Kalium-tert.-butylat, Natriumamid oder Natriummethylat, ferner die Alkaliverbindungen des Dimethylsulfoxids und Alkalihydride sowie gegebenenfalls Alkalimetalldispersionen.

Man arbeitet vorzugsweise im Temperaturbereich von 0 bis 100°C. Die erfindungsgemäßen Verbindungen werden ebenfalls erhalten, wenn man anstelle von Phosphonoverbindungen (8) und (9) die entsprechenden quartären Phosphonium-Salze, beispielsweise die Triphenylphosphoniumsalze, einsetzt und diese über die Stufe der Phosphorylene mit den Aldehyden (9) bzw. (8) kondensiert.

An den Reaktionsprodukten der vorstehenden Verfahren können natürlich noch weitere an sich bekannte Umwandlungen vorgenommen werden wie Halogenierungen, funktionelle Abwandlungen von Carboxylgruppen, Einführung von Chlormethylgruppen oder Austausch von Halogenatomen gegen Cyangruppen.

Die Herstellung von Verbindungen der Formel (1) kann aber auch nach anderen an sich bekannten Verfahren erfolgen. So kann man eine Schiff'sche Base der Formel

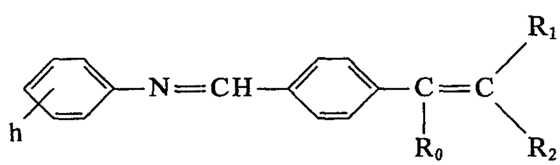
(10)

worin $R_0$, $R_1$ und $R_2$ die oben angegebene Bedeutung haben und h zweckmäßig für Wasserstoff oder Chlor steht, mit einer Methylverbindung der Formel

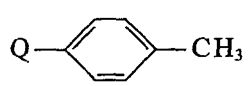
(11)

worin Q die oben angegebene Bedeutung hat, in Gegenwart einer stark basischen Alkaliverbindung in Dimethylformamid als Reaktionsmedium umsetzt. Unter stark basischen Alkaliverbindungen sollen hierbei solche Verbindungen der Alkalimetalle verstanden werden, die eine Basenstärke von mindestens etwa der des Lithiumhydroxids aufweisen. Es können hiernach Verbindungen des Lithiums, Natriums, Kaliums, Rubidiums oder Cäsiums vom Typ beispielsweise der Alkoholate, Hydroxide oder stark basische Ionenaustauscher sein. Vorteilhafterweise verwendet man Kaliumverbindungen der Zusammensetzung

$KOC_{m-1}H_{2m-1}$

worin m eine ganze Zahl von 1 bis 6 darstellt, wie z. B. Kaliumhydroxid oder Kaliumtertiär-butylat. Im Falle von Alkali-Alkoholaten ist in praktisch wasserfreiem Medium zu arbeiten, während bei Alkalihydroxiden Wassergehalte bis zu 25% (z. B. Kristallwassergehalte) erlaubt sind. Im Falle von Kaliumhydroxid hat sich ein Wassergehalt von bis zu etwa 10% als zweckmäßig erwiesen. Als Beispiele für andere verwendbare Alkaliverbindungen seien genannt: Natriummethylat, Natriumhydroxid, Lithiumhydroxid, Rubidiumhydroxid, Cäsiumhydroxid usw. Selbstverständlich ist es auch möglich, mit Gemischen solcher Basen zu arbeiten.

Die Verbindungen der Formel (11) worin Q einen Rest der Formel

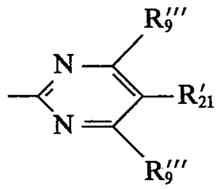

ist, worin $R_9'''$ für Wasserstoff, $C_3$—$C_4$-Alkyl oder $C_1$—$C_4$-Alkylthio und $R_{21}'$ für Wasserstoff oder Methyl stehen sind neu, und sind ebenfalls Gegenstand der vorliegenden Erfindung.

Zweckmäßig werden die Verbindungen (11) mit der Schiff'schen Base der Formel (10) in äquivalenten Mengen zur Umsetzung gebracht, sodaß von keiner Komponente ein wesentlicher Überschuß vorhanden ist. Von der Alkaliverbindung verwendet man mit Vorteil mindestens die äquivalente Menge, d. h. mindestens 1 Mol einer Verbindung mit z. B. einer KO-Gruppe auf ein Mol Schiff'sche Base. Bei der Verwendung von Kaliumhydroxid wird vorzugsweise die 4- bis 8fache Menge angewandt. Die Umsetzung kann generell bei Temperaturen im Bereich zwischen etwa 10 und 150°C durchgeführt werden. Werden bei der Reaktion als Kaliumverbindung Alkoholate verwendet, so ist im allgemeinen keine Wärmezufuhr notwendig. Man verfährt z. B. so, daß man die Schiff'sche Base der Formel (10) dem Gemisch aus der Verbindung der Formel (11) dem Lösungsmittel und dem Kaliumalkoholat, zweckmäßig unter Rühren und unter Ausschluß von Luft, bei einer Temperatur zwischen 15 und 30°C, zusetzt, worauf die Reaktion unter leichtem Temperaturanstieg ohne weiteres stattfindet. Bei der Anwendung von Kaliumhydroxid ist es häufig notwendig, bei höherer Temperatur zu arbeiten. Beispielsweise wird das Reaktionsgemisch langsam auf 30 bis 100°C erwärmt und dann während einiger Zeit, z. B. 1/2 bis 2 Stunden, bei dieser Temperatur gehalten. Aus dem Reaktionsgemisch können die Endstoffe nach üblichen, an sich bekannten Methoden isoliert werden.

Die Verbindungen der Formel (1) können auch gemäß der Heck'schen Reaktion, durch Umsetzung eines aromatischen Halogenides mit einem Acrylsäurederivat in Gegenwart von einem Palladiumderivat, z. B. Paladiumacetat als Katalysator [vgl. R. F. Heck und J.-P. Nolley, Jr., J. Org. Chem., 37, 2320—2322 (1972)] hergestellt werden.

Die erfindungsgemäßen Verbindungen zeigen in gelöstem oder feinverteiltem Zustande eine ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Hierfür seien beispielsweise, ohne daß durch die nachfolgende Übersicht irgendeine Beschränkung hierauf ausgedrückt werden soll, die folgenden Gruppen von organischen Materialien, sowie eine optische Aufhellung derselben in Betracht kommt, genannt:

I. Synthetische organische hochmolekulare Materialien

a) Polymerisationsprodukte auf Basis mindestens eine polymerisierbare Kohlenstoff-Kohlenstoff-Doppelbindung enthaltender organischer Verbindungen, d. h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von $\alpha,\beta$-ungesättigten Carbonsäuren oder Derivaten solcher Carbonsäuren, insbesondere von Acrylverbindungen (wie z. B. Acrylestern, Acrylsäure, Acrylnitril, Acrylamiden und deren Derivaten oder deren Methacryl-Analoga), von Olefin-Kohlenwasserstoffen (wie z. B. Äthylen, Propylen, Styrole oder Diene, ferner sogenannte ABS-Polymerisate), Polymerisate auf Basis von Vinyl- und Vinyliden-Verbindungen (wie z. B. Vinylchlorid, Vinylalkohol, Vinylidenchlorid),

b) Polymerisationsprodukte, die durch Ringöffnung erhältlich sind, z. B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- oder Mischkondensationsprodukte sowie Produkte der Nachbehandlung, wie beispielsweise Polyester, insbesondere gesättigte (z. B. Äthylenglykolterephthalsäure-Polyester) oder ungesättigte (z. B. Maleinsäure-Dialkohol-Polykondensate sowie deren Vernetzungsprodukte mit anpolymerisierbaren Vinylmonomeren), unverzweigte

sowie verzweigte (auch auf Basis höherwertiger Alkohole, wie z. B. Alkydharze) Polyester, Polyamide (z. B. Hexamethylendiaminadipat), Maleinatharze, Melaminharze, deren Vorkondensate und Analoga, Polycarbonate, Silikone,

d) Polyadditionsprodukte wie Polyurethane (vernetzt und unvernetzt), Epoxidharze,

II. Halbsynthetische organische Materialien, z. B. Celluloseester verschiedener Veresterungsgrade (sogenanntes 2 1/2-Acetat, Triacetate) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seide, natürliche Lackharze, Stärke, Casein.

Die optisch aufzuhellenden Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d. h. beispielsweise als vorwiegend dreidimensional ausgedehnte Körper wie Platten, Profile, Spritzgußformlinge, verschiedenartige Werkstücke, Schnitzel, Granulate oder Schaumstoffe, ferner als vorwiegend zweidimensional ausgebildete Körper wie Filme, Folien, Lacke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z. B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u. a. Bedeutung für die Behandlung von textilen organischen Materialien, insbesondere textilen Gewebe, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäß optisch aufzuhellen sind, geschieht dies mit Vorteil in wäßrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich vorteilhaft erweisen, in neutralem oder alkalischem oder saurem Bade zu arbeiten. Die Behandlung wird üblicherweise bei Temperaturen von etwa 20 bis 140°C, beispielsweise bei Siedetemperatur des Bades oder in deren Nähe (etwa 90°C), durchgeführt. Für die erfindungsgemäße Veredlung textiler Substrate kommen auch Lösungen oder Emulsionen in organischen Lösungsmitteln in Betracht, wie die in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäß vorliegender Erfindung können ferner den Materialien vor oder während deren Verformung zugesetzt bzw. einverleibt werden. So kann man sie beispielsweise bei der Herstellung von Filmen, Folien (z. B. Einwalzen in Polyvinylchlorid in der Hitze) oder Formkörpern der Preßmasse oder Spritzgußmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

- Zugabe zu den Ausgangssubstanzen (z. B. Monomeren) oder Zwischenprodukten (z. B. Vorkondensaten, Praepolymeren), d. h. vor oder während der Polymerisation, Polykondensation oder Polyaddition,
- Aufpudern auf Polymerisatschnitzel oder Granulate für Spinnmassen,
- Badfärbung von Polymerisatschnitzeln oder Granulaten für Spinnmassen,
- Dosierte Zugabe zu Spinnschmelzen oder Spinnlösungen,
- Applikation auf Spinnkabel vor dem Verstrecken.

Die neuen optischen Aufhellmittel gemäß vorliegender Erfindung können beispielsweise auch in folgenden Anwendungsformen eingesetzt werden:

a) Mischungen mit Farbstoffen (Nuancierung) oder Pigmenten (Farb- oder insbesondere z. B. Weißpigmente) oder als Zusatz zu Färbebädern, Druck-, Ätz- oder Reservepasten. Ferner auch zur Nachbehandlung von Färbungen, Drucken oder Ätzdrucken,

b) in Mischungen mit sogenannten »Carriern«, Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z. B. Stärke oder synthetischen Appreturen) sowie in Kombination mit den verschiedensten Textilveredlungsverfahren, insbesondere Kunstharzausrüstungen (z. B. Knitterfest-Ausrüstungen wie »wash-and-wear«, »permanent-press«, »no-iron«), ferner Flammfest-, Weichgriff-, Schmutzablöse (»anti-soiling«)- oder Antistatisch-Ausrüstungen oder antimikrobiellen Ausrüstungen,
d) Einarbeiten der optischen Aufhellmittel in polymere Trägermaterialien (Polymerisations-, Polykondensations- oder Polyadditionsprodukte) in gelöster oder dispergierter Form für Anwendung z. B. in Beschichtungs-, Imprägnier- oder Bindemitteln (Lösungen, Dispersionen, Emulsionen) für Textilien, Vliese, Papier, Leder,
e) als Zusätze zu sogenannten »master batches«,
f) als Zusätze zu den verschiedensten industriellen Produkten, um dieselben marktfähiger zu machen (z. B. Aspektverbesserung von Seifen, Waschmitteln, Pigmenten),
g) in Spinnbadpräparationen, d. h. als Zusätze zu Spinnbädern wie sie zur Gleitfähigkeitsverbesserung für die Weiterverarbeitung von Synthese-Fasern verwendet werden, oder aus einem speziellen Bad vor der Verstreckung der Faser,
h) als Szintillatoren, für verschiedene Zwecke photographischer Art, wie z. B. für elektrophotographische Reproduktion oder Supersensibilisierung,
i) je nach Substitution als Laser-Farbstoffe.

Die erfindungsgemäßen Verbindungen können auch in Kombination mit anderen optisch aufhellenden Verbindungen verwendet werden. Als zu mischende Verbindungen kommen z. B. die folgenden bekannten in Betracht: 1,4-Bis-(benzoxazol-2'-yl)-naphthalin, 4,4'-Bis-(äthoxycarbonyl-vinyl)-stilben, 4,4'-Bis-(cyan-vinyl)-stilben, 1,4-Bis-(2'-cyano-styryl)-benzol, 1,5-Bis-(benzoxazol-2'-yl)-thiophen, 1-Phenyl-4-(5',7'-dimethyl-benzoxazol-2'-yl)-stilben, 1,2-Bis-(5'-methyl-benzoxazol-2'-yl)-vinylen, 4-(benzoxazol-2'-yl)-4'-(3''-methyl-1'',2'',4''-oxadiazol-5''-yl)-stilbene und 2,4-Dimethoxytriazin-6-yl-pyren. Diese werden vorteilhaft mit Verbindungen der Formel

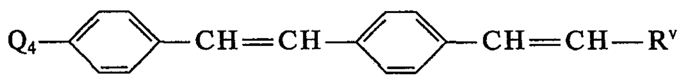 (12)

vermischt, worin $Q_4$ einen Benzoxazol-2-yl-, Pyrimidin-2-yl-, 4-Methyl-pyrimidin-2-yl oder 4,6-Dimethyl-pyrimidin-2-ylrest und $R^V$ $C_2$—$C_4$-Alkoxycarbonyl oder Cyano bedeuten.

Die so erstellbaren Aufhellermischungen enthalten die erfindungsgemäße Verbindung und die bekannte im Verhältnis 1 : 9 bis 9 : 1, vorzugsweise 1 : 2 oder 2 : 1.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellende Verbindungen in solcher Konzentration enthalten, daß der gewünschte Aufhelleffekt erreicht wird.

In gewissen Fällen werden die Aufheller durch eine Nachbehandlung zur vollen Wirkung gebracht. Diese kann beispielsweise eine chemische (z. B. Säure-Behandlung), eine thermische (z. B. Hitze) oder eine kombinierte chemisch/thermische Behandlung darstellen. So verfährt man beispielsweise bei der optischen Aufhellung einer Reihe von Fasersubstanzen, z. B. von Polyesterfasern, mit dem erfindungsgemäßen Aufhellern zweckmäßig in der Weise, daß man diese Fasern mit den wäßrigen Dispersionen (gegebenenfalls auch Lösungen) der Aufhellmittel bei Temperaturen unter 75°C, z. B. bei Raumtemperatur, inprägniert und einer trockenen Wärmebehandlung bei Temperaturen über 100°C unterwirft, wobei es sich im allgemeinen empfiehlt, das Fasermaterial vorher noch bei mäßig erhöhter Temperatur, z. B. bei mindestens 60 bis etwa 130°C zu trocknen. Die Wärmebehandlung in trockenem Zustande erfolgt dann vorteilhaft bei Temperaturen zwischen 120 und 225°C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z. B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,0005 und 0,5 Gewichtsprozent von Interesse.

Aus verschiedenen Gründen ist es oft zweckmäßig, die Aufheller nicht als solche, d. h. rein einzusetzen, sondern vermischt mit den verschiedensten Hilfs- und Coupiermitteln, wie z. B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kalimpyrophosphat und Natrium- oder Kaliumtripolyphosphaten oder Alkalimetallsilicaten.

Die neuen optischen Aufhellmittel eignen sich auch besonders als Zusätze für Waschbäder oder zu Gewerbe- und Haushaltwaschmitteln, wobei sie in verschiedener Weise zugesetzt werden können.

Zu Waschbädern werden sie zweckmäßig in Form ihrer Lösungen in Wasser oder organischen Lösungsmitteln oder auch in feiner Verteilung als wäßrige Dispersion zugegeben. Zu Haushalt- oder gewerblichen Waschmittel werden sie vorteilhaft in irgendeiner Phase des Herstellungsprozesses der Waschmittel, z. B. der sogenannten »slurry« vor dem Zerstäuben dem Waschpulver oder bei der Vorbereitung flüssiger Waschmittelkombinationen, zugesetzt. Die Zuagbe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seifen in Form von Schnitzeln und Pulver, Synthetika, lösliche Salze von Sulfonsäurehalbestern höherer Fettalkohole, höher und/oder mehrfach alkylsubstituierten Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw. in Frage. Als Aufbaustoffe, sogenannte »Builders«, kommen z. B. Alkalipoly- und -polymetaphosphate, Alkalipyrophosphate, Alkalisalze der Carboxymethylcellulose und andere »Soilredepositionsinhibitoren«, ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren, in Betracht. Ferner können in den Waschmitteln beispielsweise enthalten sein: antistatische Mittel, rückfettende Hautschutzmittel wie Lanolin, Enzyme, Antimikrobika, Parfüme und Farbstoffe.

Die neuen optischen Aufheller haben den besonderen Vorteil, daß sie auch bei Gegenwart von Aktivchlorspendern, wie z. B. Hypochlorit, wirksam sind und ohne wesentliche Einbuße der Effekte in Waschbädern mit nichtionogenen Waschmitteln, z. B. Alkylphenolpolyglykoläthern, verwendet werden können.

Die erfindungsgemäßen Verbindungen werden in Mengen von 0,005 bis 1 % oder mehr, bezogen auf das Gewicht des flüssigen oder pulverförmigen, fertigen Waschmittels, zugesetzt. Waschflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt:

Die angegebenen Textilien werden während 1 bis 30 Minuten bei 20 bis 100°C in einem Waschbad behandelt, das 1 bis 10 g/kg eines aufgebauten, zusammengesetzten Waschmittels und 0,05 bis 1 % bezogen auf das Waschmittelgesetz, der beanspruchten Aufhellmittel enthält. Das Flottenverhältnis kann 1 : 3 bis 1 : 50 betragen. Nach dem Waschen wird wie üblich gespült und getrocknet. Das Waschbad kann als Bleichzusatz 0,2 g/l Aktivchlor (z. B. als Hypochlorit) oder 0,1 bis 2 g/l Natriumperborat enthalten.

In den Beispielen sind Prozente immer Gewichtsprozente. Schmelz- und Siedepunkte sind, sofern nicht anders vermerkt, unkorrigiert.

## Beispiel 1

27,5 g 2-(4-Diäthoxy-phosphorylmethylphenyl)-4,6-dimethoxy-pyrimidin und 14,3 g 4-Formylzimtsäuremethylester werden in 150 ml Dimethylformamid gelöst und innerhalb von 30 Minuten in kleinen Portionen mit 4,9 g Natriummethylat versetzt. Das Reaktionsgemisch wird nach 30 Minuten bei Raumtemperatur und dann noch 2 1/2 Stunden bei 40–45°C gerührt. Nach dem Abkühlen auf Raumtemperatur wird mit Ameisensäure sauer gestellt und in 800 ml Wasser und Eis eingerührt. Das ausgefallene Produkt wird abfiltriert, mit Wasser und Methanol gewaschen und bei 80°C im Vakuum getrocknet. Man erhält 28 g der Verbindung der Formel

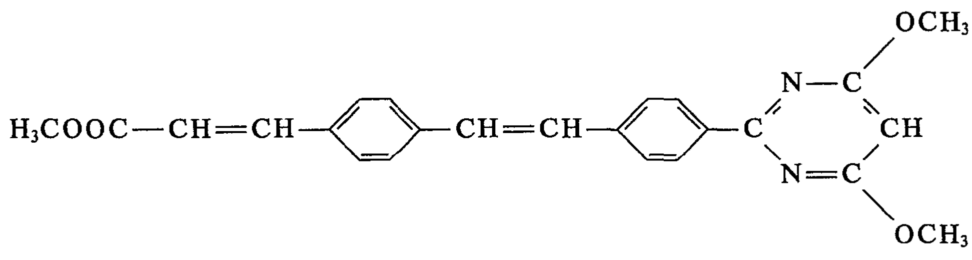

(101)

Das Produkt kristallisiert aus Toluol unter Zusatz von Bleichmitteln in gelben Kristallen vom Schmelzpunkt 169–170°C.

Verfährt man in analoger Weise, dann erhält man aus den entsprechenden Ausgangsstoffen die in Tabelle I aufgeführten Verbindungen der Formel

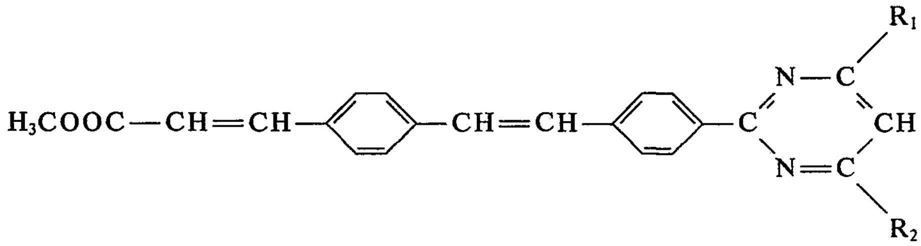 (102)

Tabelle I

| Verbindung Nr. | $R_1 = R_2$ | Schmelzpunkt °C |
|---|---|---|
| 103 | $—OCH_3$ | 167–170 |
| 104 | $—OC_2H_5$ | 192–193 |
| 105 | $—OC_3H_7$-n | 190–192 |
| 106 | 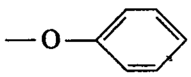 | 216–127 |
| 107 | $—SC_2H_5$ | 172–173 |
| 108 | $—CH_3$ | 193–194 |

Die als Ausgangsprodukte für die Synthese der Phosphonate benötigten in 4,6-Stellung substituierten 2-(4-Brommethylphenyl)-pyrimidine werden auf folgende Weise hergestellt.

102,3 g p-Tolylamidinhydrochlorid und 99,3 g Malonsäurediäthylester werden in 520 ml wasserfreiem Äthanol angeschlämmt. Unter gutem Rühren und Kühlung läßt man nun 323,7 g einer 30%igen Natriummethylatlösung einfließen. Danach wird zum Rückfluß erhitzt und 4 bis 5 Stunden am Rückfluß gerührt. Nach Abdestillation des Lösungsmittels wird der Rückstand in 1000 ml Wasser aufgenommen, auf 80°C erhitzt und die etwas trübe Lösung über Kieselsäure filtriert. Nach dem Abkühlen wird mit 15%iger Salzsäure angesäuert. Der dicke Kristallbrei wird abfiltriert, mit Wasser gewaschen und bei 100°C getrocknet. Man erhält 100–110 g der Verbindung der Formel

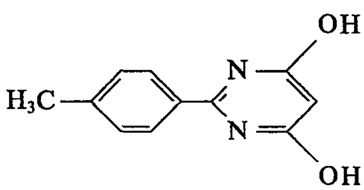 (109)

Das Produkt hat einen Schmelzpunkt von 314°C (Zersetzung).

72,6 g der Dihydroxy-Verbindung werden mit 72,6 g N,N-Dimethylanilin und 363 g Phosphoroxychlorid zum Sieden erhitzt und eine Stunde am Rückfluß gerührt. Nach Abdestillation des überschüssigen Phosphoroxychlorids wird das zurückbleibende Produkt, zur Entfernung des noch anhaftenden Phosphoroxychlorids, mit Eiswasser behandelt, danach mit Eiswasser fein gemahlen, abfiltriert, mit Eiswasser gewaschen und bei 40–50°C im Vakuum getrocknet. Die Ausbeute an der Verbindung der Formel

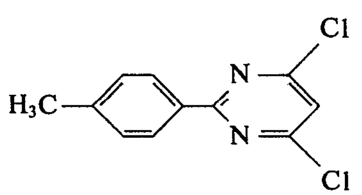 (110)

beträgt 85,9 g. Das Produkt hat einen Schmelzpunkt von 86–87°C.

156,1 g einer 30,5%igen Natriummethylatlösung werden mit 700 ml wasserfreiem Methanol verrührt. In die Lösung werden nun innerhalb von 10 Minuten unter leichter Kühlung 95,64 g der Verbindung (110) eingetragen. Danach wird auf Rückfluß erhitzt und 4 Stunden am Sieden gehalten.

Nach Abdestillieren des Lösungsmittels wird das zurückbleibende Produkt in 1000 ml Wasser eingetragen. Zur Entfernung des entstandenen Natriumchlorids wird mit Wasser fein gemahlen. Danach abfiltriert, mit Wasser gewaschen und an der Luft getrocknet. Man erhält so 90,4 g der Verbindung der Formel

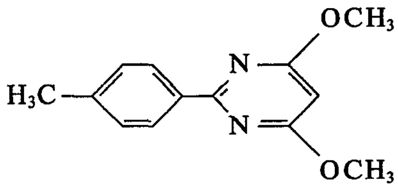

(111)

mit einem Schmelzpunkt von 61–62°C.

Auf analoge Weise werden die in Tabelle II aufgeführten Pyrimidine der Formel

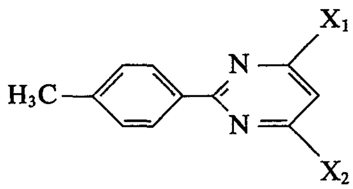

(112)

hergestellt:

Tabelle II

| Verbindung Nr. | $X_1$ | $X_2$ | Schmelzpunkt °C |
|---|---|---|---|
| 113 | $—OC_2H_5$ | $—OC_2H_5$ | 71–71 |
| 114 | 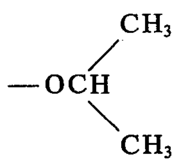 | 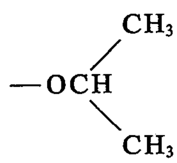 | Kp 123 5,332 Pa |
| 115 | 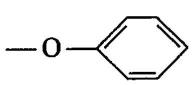 | 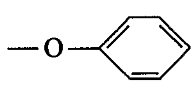 | 125–126 |
| 116 | $—OCH_2—CH_2—OCH_3$ | $—OCH_2—CH_2—OCH_3$ | schwachgelbes Öl |
| 117 | 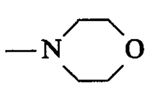 | 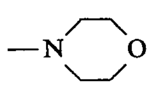 | 186–187 |
| 118 | $—NH—CH_3$ | $—OCH_2—CH_2—OCH_3$ | 65–66 |
| 119 | 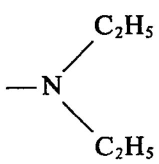 | $—OCH_2—CH_2—OCH_3$ | schwachgelbes Öl |
| 120 | $—NH—CH_3$ | Cl | 107 |

Fortsetzung

| Verbindung Nr. | $X_1$ | $X_2$ | Schmelzpunkt °C |
|---|---|---|---|
| 121 | 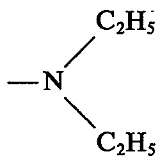 | Cl | 74–75 |
| 122 | $—OC_3H_7$ | $—OC_3H_7$ | 62 |
| 123 | $—SC_2H_5$ | $—SC_2H_5$ | 55–56 |
| 124 | $—OC_4H_9$ | $—OC_4H_9$ | Kp 158–161<br>13, 332 Pa |

115,2 g 4,6-Dimethoxy-2-(4-methylphenyl)-pyrimidin werden mit 500 ml Tetrachlormethan auf 70°C erhitzt. In die Lösung wird danach bei 70—75°C ein Gemisch aus 0,5 g Dibenzoylperoxid, 1 g Azosiobutyronitril und 90,8 g N-Bromsuccinimid unter gleichzeitiger Bestrahlung mit einer 500 Watt Lampe innerhalb von 30 Minuten eingetragen.

Zur Vervollständigung der Reaktion wird noch 2 1/2 Stunden am Rückfluß erhitzt. Danach wird das Succinimid bei 65°C abfiltriert und das Filtrat zur Trockne eingedampft. Man erhält 144 g Rohprodukt der Formel

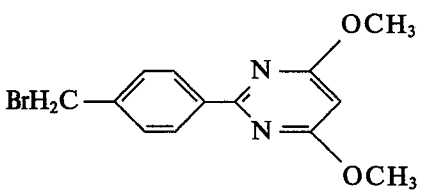

(125)

Das Produkt kann durch Umkristallisation aus Äthanol/Äthylenglykolmonomethyläther (1 : 1) gereinigt werden. (Schmelzpunkt 132—134°C).

In analoger Weise werden die in Tabelle III aufgeführten Brommethylverbindungen der Formel

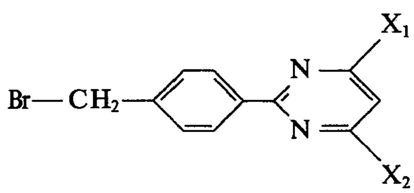

(126)

hergestellt:

Tabelle III

| Verbindung Nr. | $X_1$ | $X_2$ | Schmelzpunkt °C |
|---|---|---|---|
| 127 | $—OC_2H_5$ | $—OC_2H_5$ | 95–96 |
| 128 | $—OC_3H_7$ | $—OC_3H_7$ | 68–70 |
| 129 | 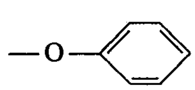 | 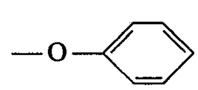 | 134–136 |

Fortsetzung

| Verbindung Nr. | $X_1$ | $X_2$ | Schmelzpunkt °C |
|---|---|---|---|
| 130 | $-CH_3$ | $-CH_3$ | 156–157 |
| 131 | $-SC_2H_5$ | $-SC_2H_5$ | gelbes Öl |
| 132 | $-OC_4H_9$ | $-OC_4H_9$ | schwachgelbes Öl |

116,4 g 2-(4-Brommethylphenyl)-4,6-dimethoxy-pyrimidin werden mit 400 ml Triäthylphosphit innerhalb von 2 1/2 Stunden auf 150—155°C erhitzt und noch 3 1/2 Stunden bei 150—155°C gerührt. Nach Abdestillation des überschüssigen Triäthylphosphits erhält man 128 g eines schwachgelben Öls vom $Kp_{13,332\ Pa}$ 207—210°C, das zu farblosen Kristallen vom Smp. 74—76°C auskristallisiert.
In analoger Weise wurden auch die folgenden Phosphonate der Formel

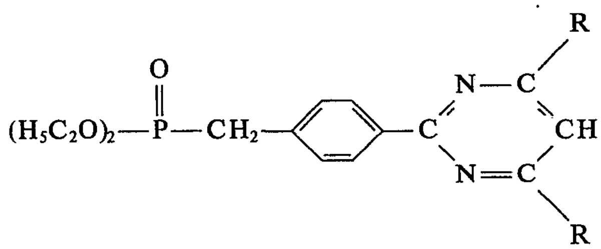

(133)

hergestellt:

Tabelle IV

| Verbindung Nr. | R | Schmelzpunkt °C |
|---|---|---|
| 134 | $-CH_3$ | 67–69 |
| 135 | $-OC_2H_5$ | schwachgelbes Öl |
| 136 | $-OC_3H_7$ | 77–79 |
| 137 | 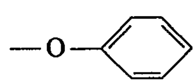 | farbloses, halbkristallines Produkt |
| 138 | $-SC_2H_5$ | schwachgelbes Öl |
| 139 | $-OC_4H_9$ | schwachgelbes Öl |

Beispiel 2

36,63 g 2-(4-Diäthoxy-phosphoryl-methylphenyl)-4,6-dimethoxy-pyrimidin und 17,62 g 4-Formylzimtsäure werden in 220 ml Dimethylformamid auf 40–45°C erhitzt. In die Lösung läßt man nun innerhalb von 20 Minuten 35,4 g einer 30,5%igen Natriummethylat-Lösung einfließen. Das dick ausgefallene Reaktionsprodukt wird noch 3 Stunden bei 40 bis 45°C gerührt, auf Raumtemperatur abgekühlt und mit 500 ml Wasser verdünnt. Nach dem Ansäuern mit 15%iger Salzsäure wird das ausgefallene Produkt abfiltriert, mit Wasser gewaschen und im Vakuum bei 80°C getrocknet. Man erhält 36,4 g der Verbindung der Formel

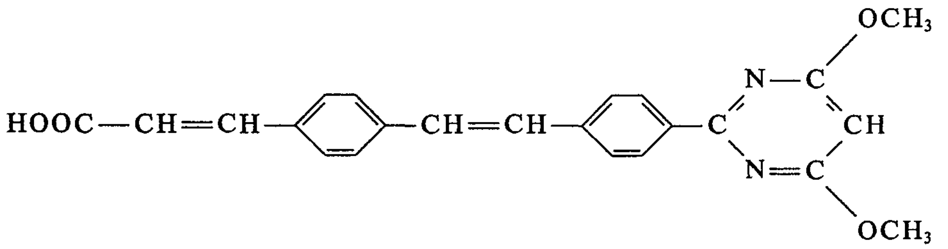

(201)

Das Produkt kristallisiert aus wenig Dimethylformamid in gelben Kristallen (Smp. 278—279°C).

Beispiel 3

13,6 g der Säure der Formel (201) werden mit 120 ml Toluol und wenig Dimethylformamid auf 70—75°C erhitzt. Danach läßt man in die Suspension 3,5 ml Thionylchlorid innerhalb von 15 Minuten einfließen. Es entsteht eine Lösung, die bis zur Beendigung der Chlorwasserstoffentwicklung zum Sieden erhitzt wird. Danach werden 30 ml Toluol und der Überschuß an Thionylchlorid abdestilliert. Nach dem Abkühlen auf 80°C läßt man in die Lösung 50 ml Isopropanol einfließen. Es wird erneut zum Sieden erhitzt und bis zur Beendigung der Chlorwasserstoffentwicklung am Sieden gehalten. Der nach dem Abdestillieren des Lösungsmittels erhaltene Rückstand wird zuerst mit Methanol behandelt und danach aus Cyclohexan, in Gegenwart von Bleicherde umkristallisiert. Man erhält 8,2 g der Verbindung der Formel

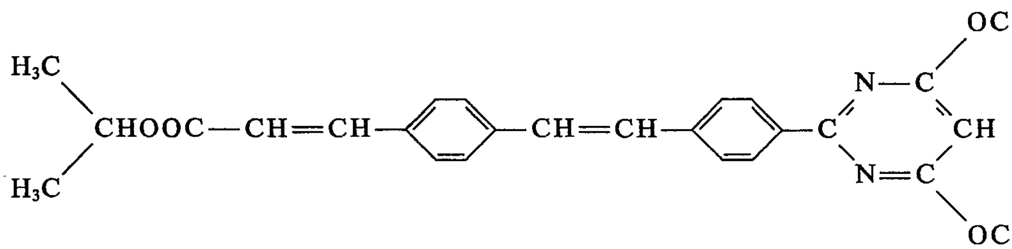

(301)

vom Smp. 126—127°C.

Beispiel 4

16,1 g der nach Beispiel 1 hergestellten Verbindung der Formel (101) werden mit 130 ml Äthylenglykolmonomethyläther auf 110°C erhitzt. In die entstandene Lösung gibt man eine Spatelspitze Lithiumamid. Danach wird auf Rückfluß erhitzt und 1 1/2 Stunden am Rückfluß gehalten. Nach Abdestillation des Lösungsmittels wird das anfallende Produkt mit Methanol behandelt, abfiltriert, mit Methanol gewaschen und getrocknet. Man erhält 14,9 g der Verbindung der Formel

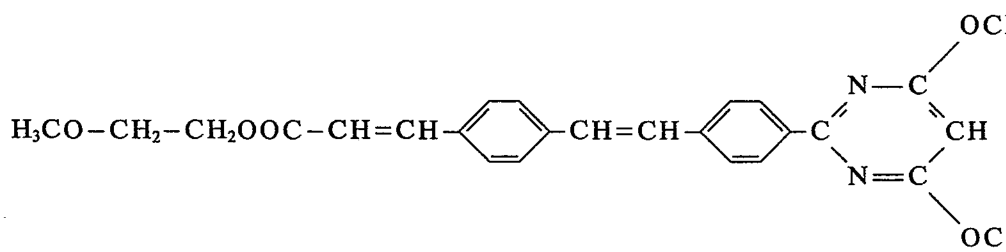

(401)

Das Produkt kristallisiert aus einem Gemisch von Cyclohexan/Toluol oder aus Tetrachloräthylen in gelben Kristallen vom Smp. 110—111°C.

Beispiel 5

13,5 g 1-Methyl-3-(4-diäthoxyphosphorylmethylphenyl)-pyridazinon und 7,6 g 4-Formyl-zimtsäuremethylester werden in 100 ml Dimethylformamid gelöst. In die Lösung werden nun innerhalb von 10 Minuten 2,6 g festes Natriummethylat eingetragen. Man läßt noch für 20 Minuten bei Raumtemperatur ausrühren und erhitzt dann für 2 Stunden auf 40—45°C. Das Reaktionsgemisch wird dann in 800 ml Wasser eingerührt, mit Ameisensäure angesäuert, abfiltriert, mit Wasser und mit Methanol gewaschen und im Vakuum bei 80°C getrocknet. Man erhält 10,9 g der Verbindung der Formel

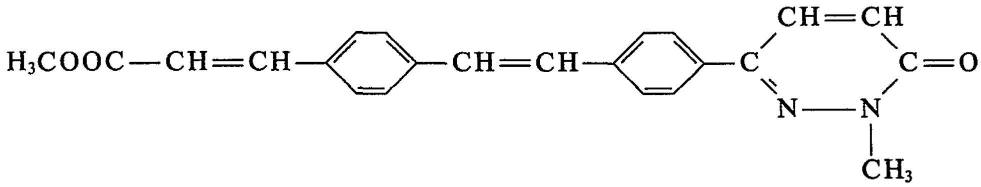

(501)

Das Produkt kristallisiert aus einem Gemisch von o-Dichlorbenzol/Chlorbenzol im Verhältnis 1 : 1 in gelben Kristallen vom Schmelzpunkt 248–250°C.

Das als Ausgangsprodukt benötigte 1-Methyl-3-(4-diäthoxyphosphorylmethylphenyl)-pyridazinon wird auf folgende Weise hergestellt:

110 g 1-Methyl-3-(4-methylphenyl)-pyridazinon und 700 ml Tetrachlormethan werden auf 70°C erhitzt. In die entstandene Lösung wird nun innerhalb von 40 Minuten bei 70–75°C ein Gemisch aus 3,5 g Dibenzoylperoxyd und 103,3 g N-Bromsuccinimid unter gleichzeitiger Bestrahlung mit einer 500 Watt Lampe eingetragen. Anschließend wird noch für weitere 3 Stunden bei Rückflußtemperatur gerührt. Danach wird das Succinimid bei 65°C abfiltriert, der Filterkuchen mit 100 ml heißem Tetrachlormethan nachgewaschen und das Filtrat zur Trockne eingedampft. Der Rückstand wird noch warm mit 100 ml Methanol verrührt, wobei Kristallisation erfolgt. Nach dem Abkühlen wird das Produkt abfiltriert und mit wenig kaltem Methanol gewaschen. Man erhält nach dem Trocknen im Vakuum bei 80°C 115,2 g der Verbindung der Formel

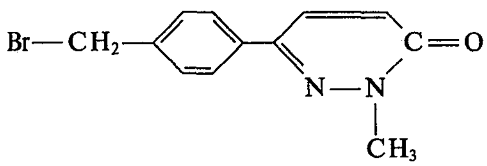

(502)

Das Produkt kristallisiert aus Methanol in farblosen Kristallen vom Schmelzpunkt 135–136°C.

Das 1-Methyl-3-(4-diäthoxyphosphorylmethyl-phenyl)-pyridazinon der Formel

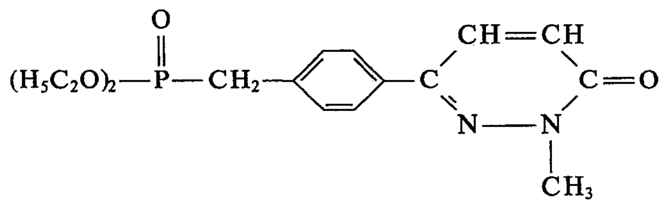

(503)

wird durch Umsatz des 1-Methyl-3-(4-brommethyl-phenyl)-pyridazinons mit Triäthylphosphit wie in Beispiel 1 beschrieben, hergestellt. Es bildet farblose Kristalle vom Smp. 91–92°C.

## Beispiel 6

In analoger Weise wie im Beispiel 5 beschrieben, wird durch Umsatz von 2-Methyl-4-methoxy-6-(4-diäthoxy-phosphorylmethyl-phenyl)-pyrimidin mit 4-Formylzimtsäure die Verbindung der Formel

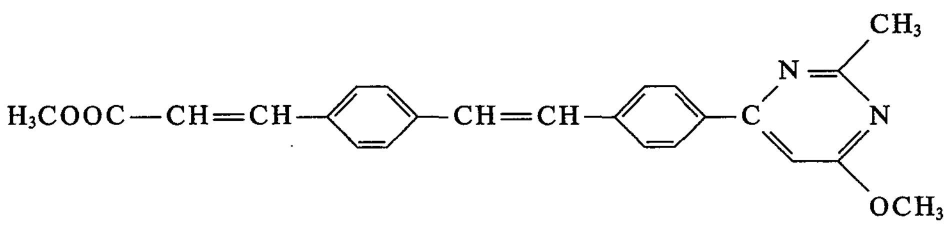

(601)

hergestellt, vom Smp. 240–241°C.

Das für die Synthese benötigte 2-Methyl-4-methoxy-6-(4-diäthoxyphosphorylmethyl-phenyl)-pyrimidin wird wie im Beispiel 1 beschrieben durch Umsatz von 2-Methyl-4-methoxy-6-(4-brommethyl-phenyl)-pyrimidin mit Triäthylphosphit hergestellt. Es liegt in Form von farblosen Kristallen vom Smp. 82–83°C vor.

Das 2-Methyl-4-methoxy-6-(4-brommethylphenyl)-pyrimidin wird wie folgt hergestellt:

70,9 g salzsaures Acetamidin und 154,65 g p-Tolylacetessigester werden in 450 ml wasserfreiem Methanol auf 60°C erhitzt. Bei dieser Temperatur läßt man nun innerhalb von einer Stunde 265,6 g einer 30,5%igen Natriummethylatlösung zufließen. Die entstandene Suspension wird noch für weitere 3 Stunden am Rückfluß erhitzt. Nach dem Abkühlen wird das Reaktionsgemisch in 1500 ml Eis und

Wasser eingerührt, wobei eine gelbe Lösung entsteht. Nach dem Ansäuren mit 45 ml Eisessig bildet sich ein dicker, farbloser Kristallbrei. Es wird filtriert, mit Wasser säurefrei gewaschen und bei 70—80°C im Vakuum getrocknet. Man erhält 95 g Rohprodukt der Formel

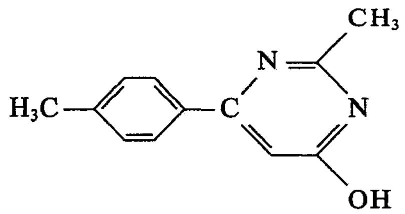

(602)

Die Verbindung läßt sich durch Umkristallisation aus Chlorbenzol reinigen (Schmelzpunkt 278—279°C).

76 g des 2-Methyl-4-hydroxy-6-(p-tolyl)-pyrimidin werden unter Kühlung und gutem Rühren innerhalb von 10 Minuten in ein Gemisch bestehend aus 171 g Phosphoroxychlorid und 19 g Triäthylamin eingetragen. Es entsteht ein dicker Brei, der nun innerhalb von 30 Minuten auf 100—105°C erhitzt wird, wobei eine Lösung entsteht, die noch eine Stunde bei 100—105°C gerührt wird. Nach dem Abkühlen auf 50°C läßt man die Reaktionslösung unter schnellem Rühren in ein Gemisch aus 1000 ml Wasser und Eis einfließen. Das ausgefallene Produkt wird noch 20 Minuten eiskalt gerührt, dann abfiltriert, mit eiskaltem Wasser gewaschen und an der Luft getrocknet. Man erhält 86,5 g Rohprodukt der Formel

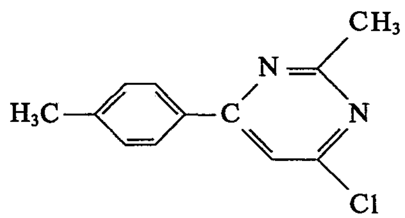

(603)

Die Reinigung erfolgt durch Lösen in 400 ml Hexan, Abfiltration von wenig ungelöstem braunen Beiprodukt und Eindampfen der gelben Hexan-Lösung im Vakuum. Es resultiert ein gelbes Öl, das nach dem Abkühlen zu schwachgelben Kristallen erstarrt. Ausbeute 81 g.

76,51 g des Rohproduktes werden ohne weitere Reinigung in 350 ml wasserfreiem Methanol, enthaltend 69,3 g einer 30%igen Natriummethylatlösung eingetragen, auf Rückfluß erhitzt und 4 Stunden bei Rückflußtemperatur gerührt. Danach wird die Reaktionsmischung zur Trockne eingedampft, der entstandene Kristallbrei mit 1000 ml Eiswasser behandelt, das Produkt abfiltriert mit Wasser gewaschen und bei 40—50°C im Vakuum getrocknet. Man erhält 75,1 g Rohprodukt der Formel

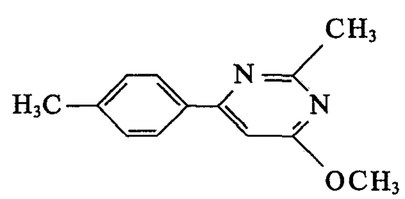

(604)

Das Produkt kristallisiert aus Methanol in farblosen Kristallen vom Schmelzpunkt 78—79°C.

Das Produkt wurde nun wie unter Beispiel 1 mit N-Bromsuccinimid in der Seitenkette bromiert (Schmelzpunkt des 2-Methyl-4-methoxy-6-(4-brommethylphenyl)-pyrimidin 93—94°C).

## Beispiel 7

Auf dem gleichen Weg wird auch durch Umsatz von 4-Formylzimtsäuremethylester mit dem 2,4-Dimethoxy-6-(4-dimethoxy-phosphorylmethyl-phenyl)-1,3,5-triazin die Verbindung der Formel

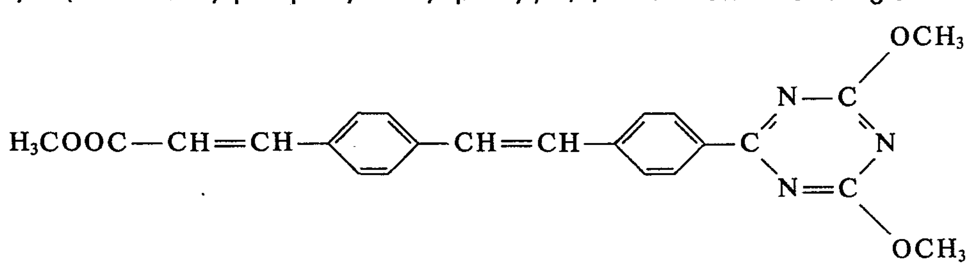

(701)

hergestellt: Smp. 201—202°C.

## Beispiel 8

8,06 g der Verbindung der Formel (701) werden mit 150 ml Äthylenglykolmonomethyläther und 0,1 g Lithiumamid 1 1/2 Stunden am Rückfluß erhitzt. Nach dem Abdampfen des Lösungsmittels erhält man 7,4 g der Verbindung der Formel

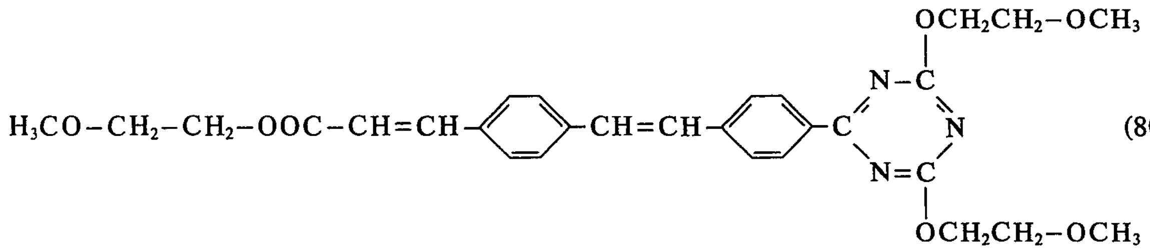

(801)

Das Produkt kristallisiert aus einem Gemisch von Toluol/Cyclohexan im Verhältnis 1 : 1 und in Gegenwart von Bleicherde in gelben Kristallen vom Smp. 103—104°C.

## Beispiel 9

13,4 g 2-(4-Diäthoxy-phosphorylmethylphenyl)-4,6-dimethyl-pyrimidin und 6,3 g 4-Formylzimtsäurenitril werden in 100 ml Dimethylformamid gelöst. Danach trägt man in die Lösung 2,58 g Natriummethylat innerhalb von 15 Minuten ein. Es wird anschließend noch eine Stunde bei Raumtemperatur, und dann noch 2 Stunden bei 40—45°C nachgerührt. Das Reaktionsgemisch wird in 800 ml Eiswasser eingetragen, mit Ameisensäure sauer gestellt, abfiltriert und mit Wasser und Methanol gewaschen. Nach dem Trocknen erhält man 10,8 g der Verbindung der Formel

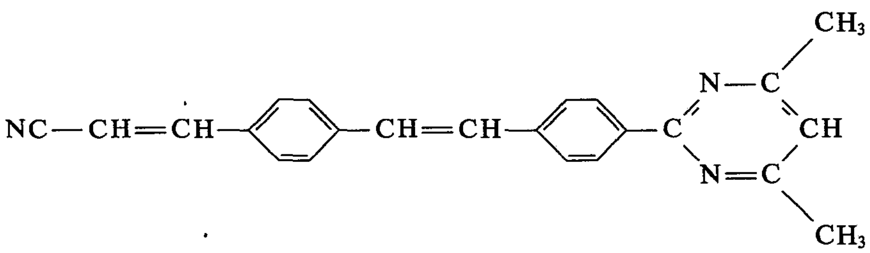

(901)

Das Produkt kristallisiert aus Xylol unter Zusatz von Bleicherde in gelben Kristallen vom Smp. 234—235°C.

Verfährt man in analoger Weise, dann erhält man aus den entsprechenden Ausgangsstoffen die in Tabelle V aufgeführten Verbindungen der Formel

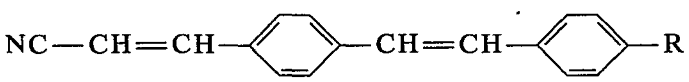

(902)

Tabelle V

| Verbindung Nr. | R | Schmelzpunkt °C |
|---|---|---|
| 903 | 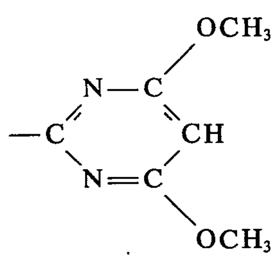 | 218–219 |

Fortsetzung

| Verbindung Nr. | R | Schmelzpunkt °C |
|---|---|---|
| 904 | 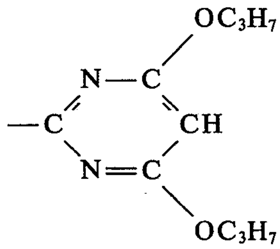 | 188–189 |
| 905 | 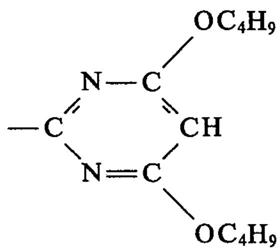 | 161–162 |
| 906 | 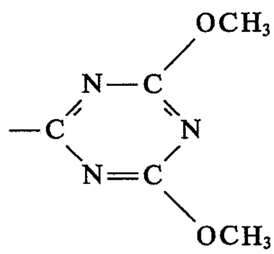 | 266–267 |
| 907 | 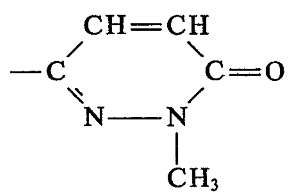 | 209–211 |
| 908 | 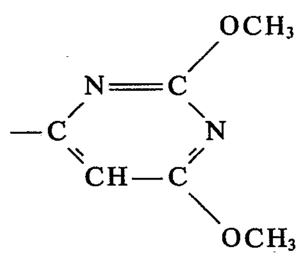 | 226–227 |
| 909 | 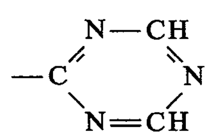 | |

Beispiel 10

13,13 g 4,6-Dichlor-2-(4-diäthoxy-phosphorylmethyl-phenyl)-pyrimidin und 5,5 g 4-Formylzimtsäurenitril werden in 100 ml Dimethylformamid gelöst. In die entstandene Lösung läßt man danach innerhalb von 25 Minuten eine Lösung von 3,4 g Natrium in 65 ml Äthylenglykolmonomethyläther einfließen. Das Reaktionsgemisch wird auf 40°C erhitzt, 2 Stunden bei dieser Temperatur gerührt, anschließend in 800 ml Wasser und Eis eingerührt, mit Ameisensäure sauer gestellt, abfiltriert mit Wasser gewaschen und getrocknet. Man erhält 15,4 g der Verbindung der Formel

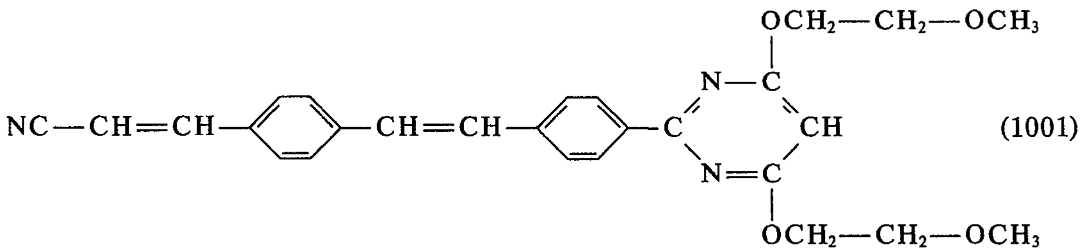

(1001)

Die Verbindung wird säulenchromatographisch an Kieselgel gereinigt. (Lösungs- und Laufmittel Toluol/Essigester 80 : 20). Man erhält 8,2 g der Verbindung der Formel (1001) vom Smp. 141–142°C.

Das für die Synthese benötigte 4,6-Dichlor-2-(4-diäthoxy-phosphorylmethylphenyl)-pyrimidin (Smp. 111–112°C) wird durch Umsatz von 4,6-Dichlor-2-(4-brommethylphenyl)-pyrimidin (Smp. 155–156°C) mit Triäthylphosphit hergestellt.

Beispiel 11

13,7 g 2-[(4-Dibutoxy-phosphoryl-methyl)phenyl]-5-(p-tolyl)-1,3,4-oxadiazol, 5,7 g 4-Formylzimtsäuremethylester und 80 ml Dimethylformamid werden innerhalb von 10 Minuten bei Raumtemperatur mit 1,95 g Natriummethylat versetzt. Die Suspension wird noch 30 Minuten bei Raumtemperatur und dann noch 2 1/2 Stunden bei 40–45°C gerührt. Nach dem Ansäuern mit Ameisensäure läßt man die Suspension in 750 ml Wasser und Eis einfließen. Das ausgefallene Produkt wird abfiltriert, mit Wasser und dann mit Methanol gewaschen und getrocknet. Man erhält 8,3 g der Verbindung der Formel

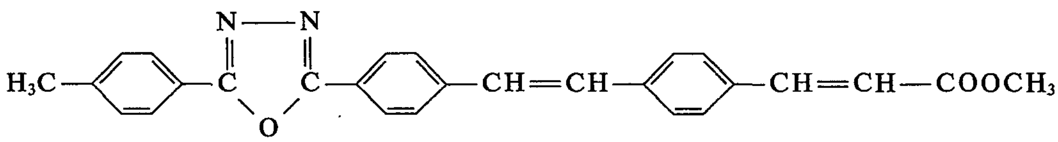

(1101)

Das Produkt kristallisiert aus einem Gemisch aus Xylol und Chlorbenzol im Verhältnis 4 : 6 und in Gegenwart von Bleicherde in grüngelben Kristallen vom Schmelzpunkt 266–268°C.

Verwendet man anstelle der 13,7 g 2-[(4-Dibutoxyphosphorylmethyl)-phenyl]-5-(p-tolyl)-1,3,4-oxadiazol äquimolare Mengen 2-[4-Dibutoxy-phosphoryl-methyl)phenyl]-5-(phenyl)-1,3,4-oxadiazol, dann erhält man ein Produkt vom Schmelzpunkt 227–228°C der Formel

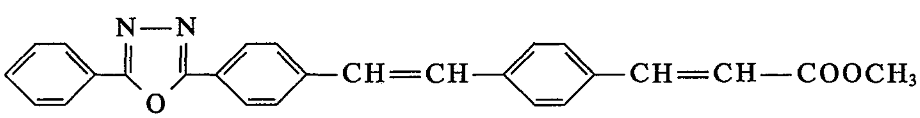

(1102)

Beispiel 12

Zu einer Lösung von 19,4 g 2-[(4-Diäthoxyphosphorylmethyl)phenyl]-4-phenyl-1,3,4-oxadiazol-2-on (vergleiche EP 9 095) und 9,5 g 4-Formyl-zimtsäuremethylester in 70 ml Dimethylformamid tropft man bei 35°C, innerhalb 15 Minuten 11 g einer 30%igen Natriummethylatlösung zu. Die Temperatur steigt bis auf 40°C. Das Reaktionsgemisch wird hierauf 4 Stunden bei 40°C verrührt, auf ein Gemisch, bestehend aus 160 ml Methanol und 250 ml Wasser gegossen und die wäßrige Suspension mit Eisessig auf pH 7 gestellt. Der Niederschlag wird abgenutscht, mit Wasser gewaschen und getrocknet. Nach mehrmaliger Umkristallisation aus Chlorbenzol werden unter Zuhilfenahme von Bleicherde 14,4 g, entsprechend 68% der Theorie, der Verbindung der Formel

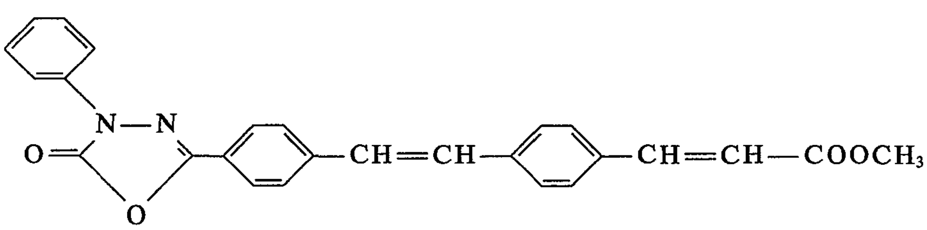

(1201)

als gelbliches Pulver vom Smp. 225—226°C erhalten.

In analoger Weise, ausgehend von den entsprechenden Ausgangsstoffen, wird auch folgende Verbindung vom Smp. 237—238°C erhalten.

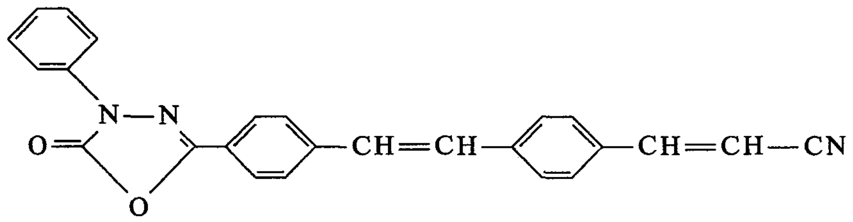

(1201)

Beispiel 13

Zu einer Lösung von 9,3 g 3-Methyl-1-(4-formylphenyl)-pyrazol und 24 g 4-(Diäthoxyphosphorylmethylphenyl)-zimtsäureäthylester in 100 ml Dimethylformamid trägt man bei Raumtemperatur innerhalb 10 Minuten 3,3 g festes Natriummethylat ein. Die Temperatur steigt bis auf 45°C. Das Reaktionsgemisch wird hierauf 2 Stunden bei 45°C verrührt, auf 500 ml Wasser gegosssen und die wäßrige Suspension mit Essigsäure auf pH 7 gestellt. Der Niederschlag wird abgenutscht, mit Wasser gewaschen und getrocknet. Nach mehrmaliger Umkristallisation aus Chlorbenzol werden unter Zuhilfenahme von Bleicherde 9,7 g, entsprechend 54% der Theorie, der Verbindung der Formel

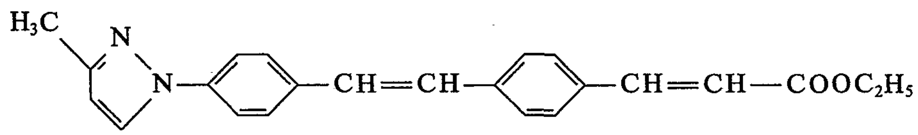

(1301)

als gelbliches Pulver vom Smp. 236—238°C.

In analoger Weise, ausgehend von den entsprechenden Ausgangsstoffen wird auch die folgende Verbindung vom Smp. 249—250°C erhalten.

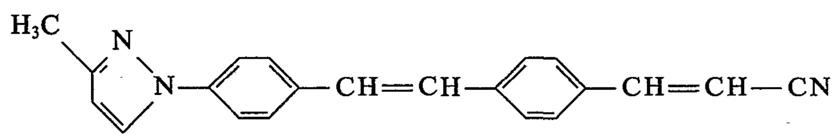

(1302)

Das verwendete Pyrazol der Formel

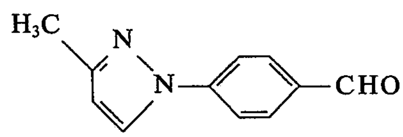

(1303)

wird wie folgt erhalten:

Zu einer Suspension von 465 g 4-Carboxyphenylhydrazinhydrochlorid (81%ig) in 3400 ml Äthanol, die 253 g Pyridin enthalten, tropft man bei Raumtemperatur 264 g 1-Acetyl-2,2-dimethoxyäthan zu. Das Reaktionsgemisch wird hierauf 1 Stunde am Rückfluß gehalten. Nun tropft man eine Lösung von 73 g Chlorwasserstoff in 240 ml Äthanol zu. Das Gemisch wird weitere 18 Stunden bei 80°C gerührt, mit einer Lösung von 240 g Natriumhydroxid in 1500 ml Wasser versetzt und das Äthanol abdestilliert. Das ausgefallene Natriumsalz des 3-Methyl-1-(4-carboxyphenyl)-pyrazols wird in 1000 ml Wasser heiß gelöst und durch Zugabe von verdünnter Salzsäure ausgefällt. Der Niederschlag wird abgenutscht mit Wasser gewaschen und getrocknet. Nach Umkristallisation aus Methanol werden 361 g, entsprechend 89% der Theorie, an 3-Methyl-1-(4-carboxyphenyl)-pyrazol vom Smp. 249—250°C erhalten.

Eine Suspension von 101 g dieser Säure in 500 ml Hexan und 1 ml Dimethylformamid wird auf 60°C erhitzt. Danach läßt man in die Suspension 71 g Thionylchlorid innerhalb 30 Minuten einfließen. Das Reaktionsgemisch wird nun 18 Stunden am Rückfluß gerührt. Nun werden 100 ml Toluol zugegeben und erneut zum Sieden erhitzt. Die nun klare Lösung wird filtriert und nach dem Abkühlen auf 10°C das ausgefallene 3-Methyl-1-(4-chlorformylphenyl)-pyrazol abgenutscht und getrocknet. Ausbeute 90 g (82% der Theorie) als helle Kristalle vom Smp. 110—111°C.

88 g 3-Methyl-1-(4-chlorformylphenyl)-pyrazol werden in 900 ml Xylol unter Zusatz von 0,23 g Thioharnstoff und 9 g Pd/$BaSO_4$ 5% bei 140°C nach Rosenmund mit Wasserstoff hydriert. Nach dem Abkühlen auf 80°C und Abfiltration des Katalysators wird die xylolische Lösung zur Trockne eingedampft und das ausgefallene Produkt aus einem Gemisch von Hexan und Toluol umkristallisiert. Man erhält 68,3 g an 3-Methyl-1-(4-formylphenyl)-pyrazol als hellgelbe Kristalle vom Smp. 96—98°C.

88 g durch Bromieren von 4-Methylzimtsäurenitril mit N-Bromsuccinimid in Tetrachlorkohlenstoff hergestelltes 4-Brommethylzimtsäurenitril vom Smp. 105—107 °C werden mit 256 g Triäthylphosphit vermischt und das Gemisch langsam unter Rühren auf 150 °C erwärmt und dann 3 Stunden bei dieser Temperatur gerührt. Anschließend wird unter Vakuum das überschüssige Triäthylphosphit entfernt. Man erhält das rohe Phosphonat der Formel

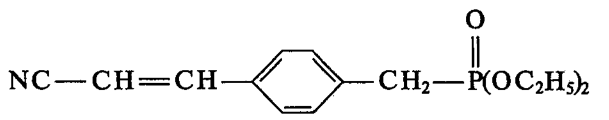

(1304)

als hellbraunes Öl.

Beispiel 14

13,78 g 1-(4-Diäthoxy-phosphorylmethylphenyl)-3,5-diphenyltriazin und 4,72 g 4-Formylzimtsäurenitril werden in 100 ml Dimethylformamid gelöst und innerhalb von 15 Minuten mit 2,1 g festem Natriummethylat versetzt. Die Suspension wird 30 Minuten bei Raumtemperatur und zur Vervollständigung der Reaktion, noch 2 Stunden bei 40—50 °C gerührt. Nach dem Abkühlen auf Raumtemperatur wird mit Ameisensäure schwach sauer gestellt und in 800 ml Eiswasser eingerührt. Das ausgefallene gelbe Produkt wird abfiltriert, mit Wasser und Methanol gewaschen und bei 80 °C im Vakuum getrocknet. Man erhält 12 g der Verbindung der Formel

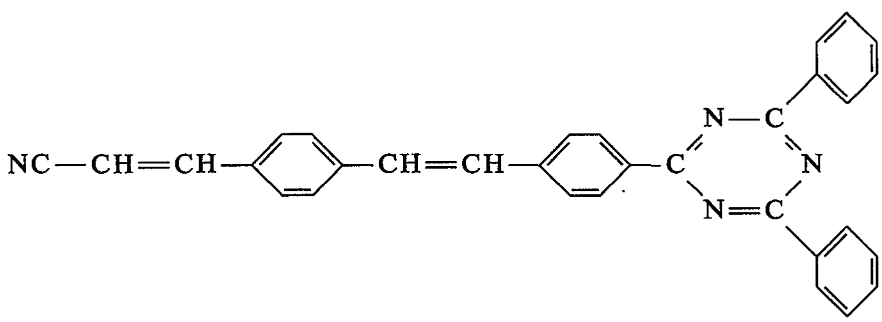

(1401)

Das Produkt kristallisiert aus o-Dichlorbenzol unter Zusatz von Bleicherde in gelben Kristallen vom Schmelzpunkt 313—314 °C.

Verfährt man in analoger Weise, dann erhält man aus den entsprechenden Ausgangsverbindungen die Verbindung der Formel

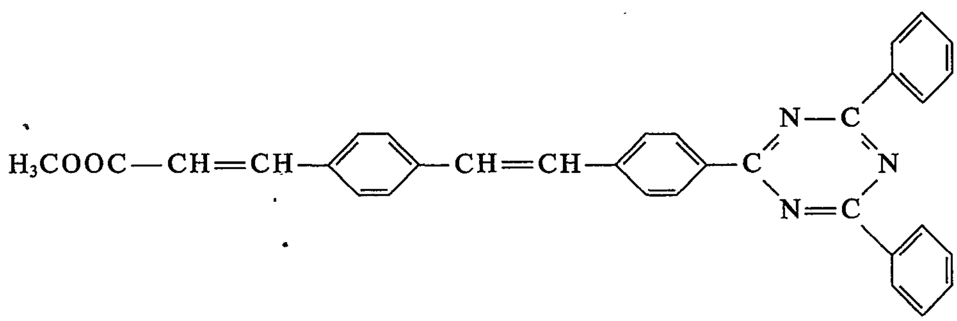

(1402)

Die Verbindung kristallisiert aus Xylol, in Gegenwart von Bleicherde in gelben Kristallen vom Schmelzpunkt 315—316 °C.

Das für die Synthese benötigte 1-(4-Diäthoxy-phosphorylmethylphenyl)-3,5-diphenyl-triazin (Schmelzpunkt 150—151 °C) wird auf folgendem Weg hergestellt:

80,84 g 1-(p-Tolyl)-3,5-diphenyl-triazin und 400 ml Äthylenchlorid werden auf 75—80 °C erhitzt. In die Lösung werden innerhalb von 30 Minuten ein Gemisch von 37,87 g 1,3-Dibrom-5,5-dimethylhydantoin und 1 g Azoisobutyronitril eingetragen. Anschließend wird noch 5 Stunden am Rückfluß erhitzt. Das nach Abdestillation des Lösungsmittels angefallene Produkt der Formel

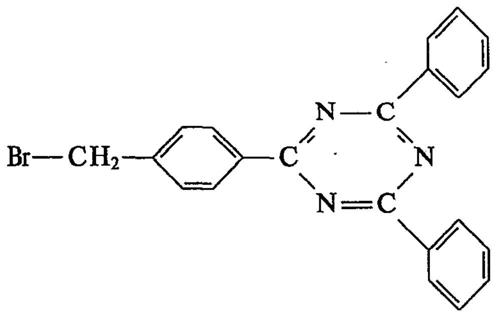
(1403)

wird mit Wasser fein gemahlen, abfiltriert, mit Wasser und Methanol gewaschen und getrocknet. Ausbeute 95,3 g. Das Produkt kristallisiert aus Äthylenglykolmonomethyläther in farblosen Kristallen vom Schmelzpunkt 201–203°C.

90,5 g 1-(4-Brommethylphenyl)-3,5-diphenyltriazin und 300 ml Triäthylphosphit werden innerhalb von 6 Stunden auf 155°C erhitzt und anschließend noch 1 Stunde bei 155°C gerührt. Der nach Abdestillation des überschüssigen Triäthylphosphits hinterbleibende ölige Rückstand wird mit Petroläther behandelt, wobei das Produkt kristallisiert. Die Ausbeute beträgt 107 g.

Das Produkt kristallisiert aus wenig Methanol in farblosen Kristallen vom Schmelzpunkt 150–151°C.

## Beispiel 15

Zu einer Lösung von 17,5 g 2-(4-Diäthoxyphosphorylmethylphenyl)-4-methoxy-6-methyl-pyrimidin und 9,5 g 4-Formyl-zimtsäuremethylester in 100 ml Dimethylformamid tropft man, bei Raumtemperatur, innerhalb 16 Minuten 11 g einer 30%igen Natriummethylat-Lösung zu. Die Temperatur steigt bis auf 40°C. Das Reaktionsgemisch wird hierauf 2 Stunden bei 45°C verrührt, auf einem Gemisch bestehend aus 160 ml Methanol und 250 ml Wasser gegossen und die wäßrige Suspension mit Essigsäure auf pH 7 gestellt. Der Niederschlag wird abgenutscht, mit Wasser gewaschen und getrocknet. Nach mehrmaliger Umkristallisation aus Toluol/Ligroin (1 : 1) werden unter Zuhilfenahme von Bleicherde 12,1 g, entsprechend 62% der Theorie, der Verbindung der Formel

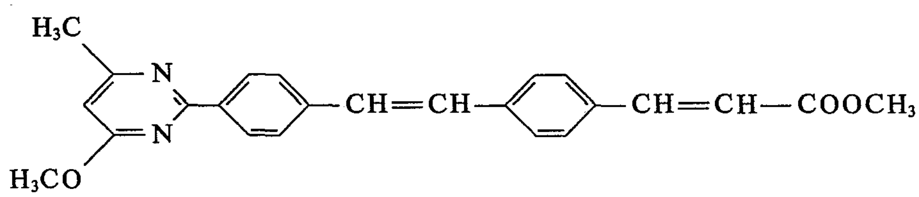
(1501)

als gelbliches Pulver vom Smp. 175–176°C erhalten.

In analoger Weise, ausgehend aus den entsprechenden Ausgangsstoffen werden die in der Tabelle VI aufgeführten Verbindungen der Formel

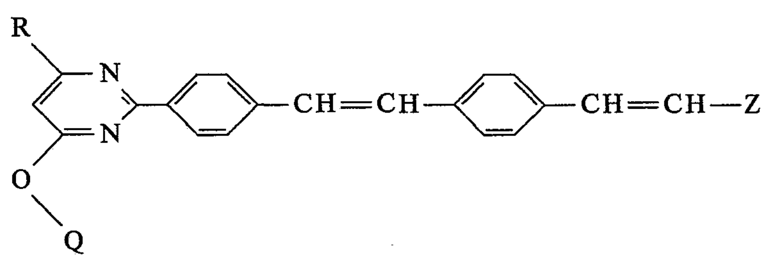
(1502)

hergestellt:

Tabelle VI

| Verbindung Nr. | R | Q | Z | Schmelzpunkt |
|---|---|---|---|---|
| 1503 | $CH_3$ | $C_6H_5$ | $-COOCH_3$ | 182–184 |
| 1504 | $C_6H_5$ | $CH_3$ | $-COOCH_3$ | 179–181 |

Fortsetzung

| Verbindung Nr. | R | Q | Z | Schmelzpunkt |
|---|---|---|---|---|
| 1505 | $C_6H_5$ | $CH_3$ | —CN | 192–194 |
| 1506 | $CH_3$ | $C_6H_5$ | —CN | 246–248 |
| 1507 | $CH_3$ | $CH_3$ | —CN | 196–197 |

Das verwendete 2-(4-Diäthoxyphosphorylmethylphenyl)-4-methoxy-6-methyl-pyrimidin der Formel

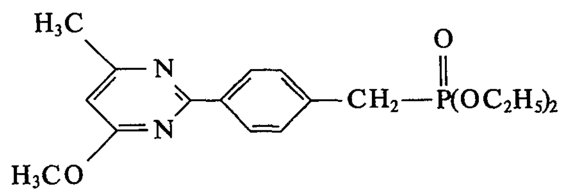

(1508)

wird wie folgt hergestellt.

Zu einer Lösung von 85 g p-Tolylamidinhydrochlorid und 80,5 g Acetessigester in 250 ml Methanol wird bei 60°C eine Lösung von 54 g Natriummethylat in 130 ml Methanol zugetropft. Das Reaktionsgemisch wird hierauf 4 Stunden am Rückfluß gehalten, auf 1000 ml Wasser gegossen und mit Essigsäure auf pH 6 bis 7 gestellt. Der Niederschlag wird abgenutscht, mit Wasser gewaschen und getrocknet. Man erhält 94,4 g (94% der Theorie) 4-Hydroxy-6-methyl-2-p-tolylpyrimidin vom Schmelzpunkt 206–207°C.

Verwendet man anstelle der 80,5 g Acetessigester äquivalente Mengen an Benzoylessigsäure-äthylester, so erhält man bei sonst gleicher Arbeitsweise das 4-Hydroxy-6-phenyl-2-p-tolylpyrimidin vom Schmelzpunkt 282–283°C.

Es werden 183 g Phosphoroxychlorid bei 5 °C vorgelegt und bei dieser Temperatur werden 20 g Triäthylamin zugetropft. Zu diesem Gemisch werden bei 10–15°C 80 g 4-Hydroxy-6-methyl-2-p-tolylpyrimidin eingetragen. Das Reaktionsgemisch wird hierauf innerhalb 30 Minuten auf 100°C erhitzt, 1 Stunde lang bei dieser Temperatur gerührt, abgekühlt und auf Eiswasser gegossen. Nach dem Absaugen, Neutralwaschen und Trocknen erhält man 85,5 g (98% der Theorie) 4-Chlor-6-methyl-2-p-tolylpyrimidin vom Schmelzpunkt 103–104°C.

Verwendet man anstelle der 80 g 4-Hydroxy-6-methyl-2-p-tolylpyrimidin äquivalente Mengen an 4-Hydroxy-6-phenyl-2-p-tolylpyrimidin so erhält man bei sonst gleicher Arbeitsweise das 4-Chlor-6-phenyl-2-p-tolylpyrimidin vom Schmelzpunkt 86–87°C.

Zu einer Suspension von 76,5 g 4-Chlor-6-methyl-2-p-tolylpyrimidin in 200 ml Methanol wird eine Lösung von 11,5 g Natrium in 100 ml Methanol eingetragen und das Reaktionsgemisch 2 Stunden am Rückfluß erhitzt und auf Wasser gegossen. Nach dem Absaugen, Waschen mit Wasser und Trocknen erhält man 72,9 g (97% der Theorie) 4-Methoxy-6-methyl-2-p-tolylpyrimidin vom Schmelzpunkt 66–67°C.

Verwendet man anstelle der 76 g 4-Chlor-6-methyl-2-p-tolylpyrimidin äquivalente Mengen 4-Chlor-6-phenyl-2-p-tolylpyrimidin, so erhält man bei sonst gleicher Arbeitsweise das 4-Methoxy-6-phenyl-2-p-tolylpyrimidin vom Schmelzpunkt 99–100°C.

Zu einer Lösung von 10,7 g 4-Methoxy-6-methyl-2-p-tolylpyrimidin und 0,2 g Dibenzoylperoxid in 100 ml wasserfreiem Tetrachlormethan gibt man bei 70°C portionenweise innerhalb von 30 Minuten eine Mischung aus 9,4 g N-Bromsuccinimid und 0,2 g Azoisobutyronitril und hält anschließend 2 Stunden am Rückfluß. Nach dem Abkühlen wird das Succinimid abgenutscht, das Nutschgut mit 200 ml Tetrachlormethan nachgewaschen und das Filtrat eingedampft. Nach Umkristallisation aus n-Hexan werden 11 g (75% der Theorie) 2-(4-Brommethylphenyl)-4-methoxy-6-methyl-pyrimidin vom Schmelzpunkt 98–99°C erhalten.

Verwendet man anstelle der 10,7 g 4-Methoxy-6-methyl-2-p-tolylpyrimidin eine äquivalente Menge 4-Methoxy-6-phenyl-2-p-tolylpyrimidin, so erhält man bei sonst gleicher Arbeitsweise das 2-(4-Brommethylphenyl)-4-methoxy-6-phenylpyrimidin vom Schmelzpunkt 98–100°C.

Ein Gemisch, bestehend aus 250 g 2-(4-Brommethylphenyl)-4-methoxy-6-methylpyrimidin und 540 g Triäthylphosphit wird langsam unter Rühren auf 150°C erwärmt und dann 5 Stunden bei dieser Temperatur gerührt. Anschließend wird unter Vakuum das überschüssige Triäthylphosphit entfernt. Man erhält das rohe Phosphat als hellbraunes Öl.

Analog wird auch das Phosphat der Formel

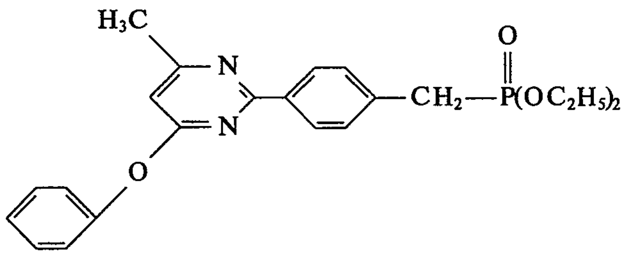

(1509)

hergestellt, das als hellbraunes Öl anfällt.

Das dazu benötigte 2-p-Tolyl-4-methyl-6-phenoxypyrimidin wird wie folgt hergestellt:

Ein Gemisch, bestehend aus 250 g Phenol und 29,7 g Natriummethylat wird erhitzt unter gleichzeitigem Abdestillieren des gebildeten Methanols bis eine Innentemperatur von 160°C erreicht ist. Anschließend wird die Schmelze auf 120°C abgekühlt, mit 109 g 4-Chlor-6-methyl-2-p-tolylpyrimidin versetzt und 3 Stunden bei 120°C gerührt. Nach dem Abkühlen wird das überschüssige Phenol mit Wasserdampf entfernt, der Rückstand abgesaugt, mit Wasser gewaschen und getrocknet. Nach Umkristallisation aus Methanol werden 119,4 g (86,5% der Theorie) 4-Methyl-6-phenoxy-2-p-tolylpyrimidin vom Schmelzpunkt 90–91°C erhalten.

Die Herstellung von 2-(4-Brommethylphenyl)-4-methyl-6-phenoxypyrimidin vom Schmelzpunkt 126–127°C erfolgt wie oben beschrieben mit N-Bromsuccinimid in einer 76%igen Ausbeute. Die Herstellung des 2-(4-Diäthoxyphosphorylmethylphenyl)-4-methoxy-6-phenoxypyrimidin erfolgt ebenfalls in analoger Weise.

Die verwendete Diäthoxyphosphorylmethyl-Verbindung der Formel

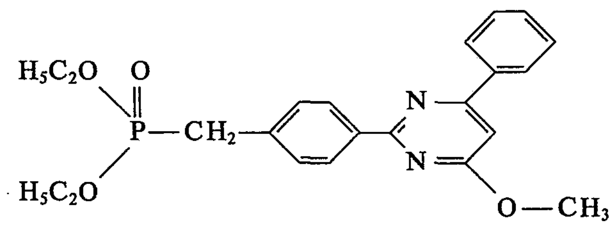

(1510)

wird auf folgende Weise hergestellt:

Ein Gemisch, bestehend aus 89 g 2-(4-Brommethylphenyl)-4-methoxy-6-phenylpyrimidin und 166 g Triäthylphosphit wird langsam unter Rühren auf 150°C erwärmt und dann 5 Stunden bei dieser Temperatur gerührt. Anschließend wird der größte Teil des überschüssigen Triäthylphosphits im Vakuum abdestilliert und der Rückstand über Kieselgel chromatographiert. Ausbeute 79,3 g (77% der Theorie) als farblose Kristalle vom Schmelzpunkt 73–75°C.

## Beispiel 16

Zu einer Lösung von 16 g 2-(4-Diäthoxyphosphorylmethylphenyl)-4-methylpyrimidin und 7,85 g 4-Formyl-zimtsäurenitril in 100 ml Dimethylformamid tropft man bei Raumtemperatur, innerhalb 15 Minuten 11 g einer 30%igen Natriummethylat-Lösung zu. Die Temperatur steigt bis auf 40°C. Das Reaktionsgemisch wird hierauf 4 Stunden bei 45°C verrührt, auf ein Gemisch bestehend aus 160 ml Methanol und 250 ml Wasser gegossen und die wäßrige Suspension mit Essigsäure auf pH 7 gestellt. Der Niederschlag wird abgenutscht, mit Wasser gewaschen und getrocknet. Nach mehrmaliger Umkristallisation aus Toluol werden unter Zuhilfenahme von Bleicherde 10,2 g, entsprechend 63% der Theorie, der Verbindung der Formel

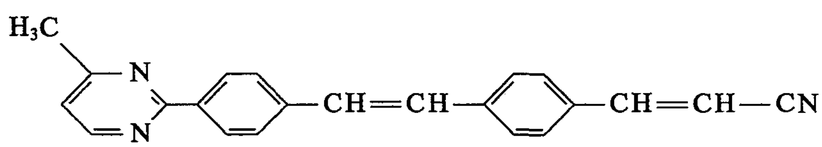

(1601)

als gelbliches Pulver vom Smp. 246–248°C erhalten.

In analoger Weise, ausgehend aus den entsprechenden Ausgangsstoffen wird auch die folgende Verbindung vom Smp. 207–208°C erhalten

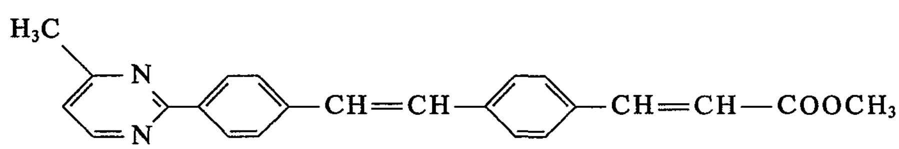

(1602)

Das verwendete Phosphonat der Formel

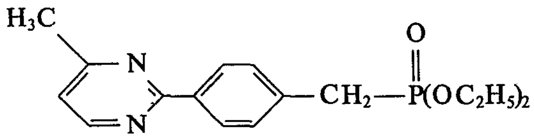

(1603)

wird wie folgt erhalten.

Zu einer Lösung von 170 g p-Tolylamidinhydrochlorid und 150 g 1-Acetyl-2,2-dimethoxyäthan in 500 ml Methanol wird bei 50°C eine Lösung von 108 g Natriummethylat in 260 ml Methanol zugetropft. Das Reaktionsgemisch wird hierauf 4 Stunden bei 50°C gerührt, auf 2000 ml Wasser gegossen und mit Essigsäure auf pH 7 gestellt. Der Niederschlag wird abgenutscht, mit Wasser gewaschen und getrocknet. Man erhält 167 g (91 % der Theorie) von 2-(4-Methylphenyl)-4-methylpyrimidin vom Smp. 80–81 °C.

Durch Umsetzung dieses 2-(4-Methylphenyl)-4-methylpyrimidins mit N-Bromsuccinimid wie in Beispiel 15 beschrieben, erhält man, nach Umkristallisation aus Ligroin das 2-(4-Brommethylphenyl)-4-methyl-pyrimidin vom Smp. 104–106°C.

Ein Gemisch, bestehend aus 105 g 2-(4-Brommethylphenyl)-4-methylpyrimidin und 265 g Triäthylphosphit wird langsam unter Rühren auf 150°C erwärmt, unter gleichzeitiger Abdestillation des gebildeten Äthylbromids, und dann 5 Stunden bei dieser Temperatur gerührt. Nach Abdestillation des überschüssigen Triäthylphosphits erhält man 125 g des Phosphats als hellbraunes dickes Öl.

## Beispiel 17

17,8 g der nach Beispiel 16 hergestellten Verbindung der Formel (1602) werden mit 80 ml Äthylenglykolmonomethyläther und 0,1 g Lithiumamid auf 110°C erhitzt. Danach wird die Lösung 6 Stunden bei dieser Temperatur gerührt, das Lösungsmittel abdestilliert und das anfallende Produkt mit Methanol behandelt, abfiltriert, mit Methanol gewaschen und getrocknet. Nach mehrmaliger Umkristallisation aus Ligroin werden unter Zuhilfenahme von Bleicherde 13,9 g, entsprechend 69,4% der Theorie, der Verbindung der Formel

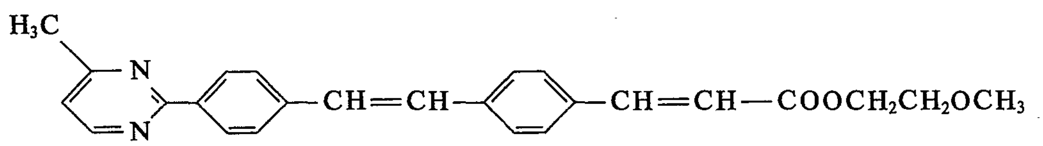

(1701)

als gelbliches Pulver vom Smp. 139–141 °C erhalten.

In analoger Weise, ausgehend von den entsprechenden Ausgangsstoffen, wurden auch folgende Verbindungen erhalten.

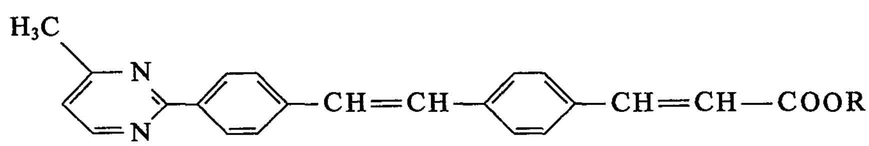

(1702)

| Verbindung Nr. | R | Smp. °C |
|---|---|---|
| (1703) | $-C_2H_5$ | 170–171 |
| (1704) | $-C_3H_7$ | 158–160 |
| (1705) | $-CH_2CH_2-O-CH_2CH_3$ | 159–161 |
| (1706) | $-CH_2CH_2-O-CH_2CH_2-O-CH_3$ | 149–151 |
| (1707) | $-CH_2CH_2-(OCH_2CH_2)_2-O-CH_3$ | 114–116 |

Fortsetzung

| Verbindung Nr. | R | Smp. °C |
|---|---|---|
| (1708) | 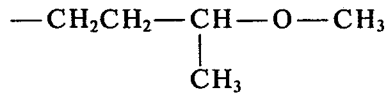 | 151–153 |
| (1709) | 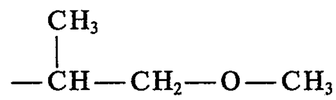 | 153–156 |
| (1710) | 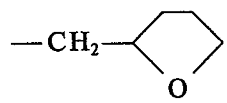 | 140–142 |
| (1711) | 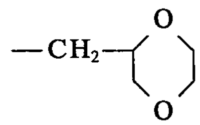 | 169–171 |

Beispiel 18

Zu 200 ml Toluol und 100 ml Pyridin in einer Rückflußapparatur, die mit einer Barrett-Falle zur Entfernung des aus den Rückflußdämpfen auskondensierten Wassers ausgestattet ist, werden 148 g Terephthalaldehyd, 85 g Cyanessigsäure und 3 g Ammoniumacetat gegeben. Das Reaktionsgemisch wird erwärmt und unter Rühren beim mäßigen Rückflußsieden gehalten, bis kein Wasser mehr gebildet wird (etwa 2–3 Stunden).

Nach Zugabe von 200 ml Wasser wird das Gemisch der Wasserdampfdestillation unterworfen, wodurch Toluol und andere flüchtige Stoffe entfernt werden. Anschließend wird der Rückstand mit einer Lösung von 300 g Natriumbisulfit in 900 ml Wasser bei 50°C kräftig durchgerührt, auf Raumtemperatur abgekühlt und filtriert. Das Filtrat wird mit 600 ml einer 30%igen wäßrigen Formaldehyd-Lösung versetzt und der abgeschiedene Aldehyd in Toluol aufgenommen. Die toluolische Lösung wird über Magnesiumsulfat getrocknet und nach dem Verdampfen des Toluols wird das 4-Formylzimtsäurenitril destilliert.

Man erhält 84,5 g des E-Z-Isomerengemisches vom Kp. 132–155°C 13,332 Pa aus dem man die E-Form vom Schmelzpunkt 118–119°C durch Kristallisieren aus Methanol erhält.

Beispiel 19

6,1 g 4-(Diäthoxy-phosphoryl-methyl)biphenyl und 4,1 g 4-Formyl-zimtsäuremethylester werden bei 40°C in 40 ml Dimethylformamid unter Rühren gelöst. Dann werden 4,25 ml methanolische Natrium-methylatlösung (Gehalt: 30,7%) zugetropft, wobei die Temperatur durch Kühlung mit Eiswasser unter 45°C gehalten wird. Dann wird 2 1/2 Stunden bei 40–45°C nachgerührt, die entstandene Suspension auf 10°C abgekühlt, zuerst 67 ml Methanol, dann 94 ml Wasser zugetropft, mit 0,5 ml Essigsäure 50% neutralisiert, nach dem Abkühlen auf 5°C das auskristallierende Produkt abgenutscht, mit etwa 200 ml Methanol/Wasser gewaschen und unter Vakuum bei 70–80°C getrocknet. Nach dem Umkristallisieren aus 200 ml Chlorbenzol unter Zuhilfenahme von Bleicherde erhält man 1,5 g der Verbindung der Formel

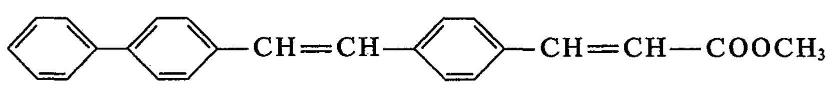 (1901)

als gelbliches Pulver. Schmelzpunkt 264–277°C.

Beispiel 20

12,25 g 2-(4-Diäthoxy-phosphorylmethylphenyl)-pyrimidin und 6,3 g 4-Formylzimtsäurenitril werden in 100 ml Dimethylformamid gelöst. Die Lösung wird anschließend innerhalb von 20 Minuten mit 2,59 g Natriummethylat versetzt. Es wird noch 30 Minuten bei Raumtemperatur und anschließend, zur Vervollständigung der Reaktion, noch weitere 2 1/2 Stunden bei 40–41 °C gerührt. Danach wird das Reaktionsgemisch in 800 ml Eis und Wasser eingerührt, mit Ameisensäure schwach sauer gestellt, abfiltriert, mit Wasser und Methanol gewaschen und getrocknet. Man erhält 9,4 g der Verbindung der Formel

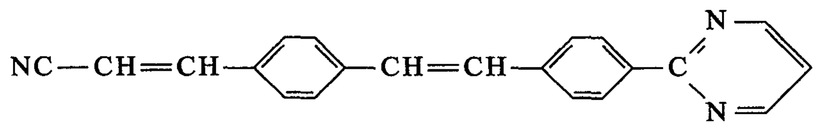

(2001)

Die Verbindung kristallisiert aus Chlorbenzol, in Gegenwart von Bleicherde, in schwach grüngelben Kristallen vom Smp. 290–291 °C.

Verwendet man in obiger Vorschrift an Stelle von 12,25 g 2-(4-Diäthoxy-phosphorylmethylphenyl)-pyrimidin die äquivalente Menge an 2-(4-Diäthoxy-phosphorylmethylphenyl)-1,3,5-triazin und verfährt ansonsten wie beschrieben, so erhält man 9,3 g der Verbindung der Formel

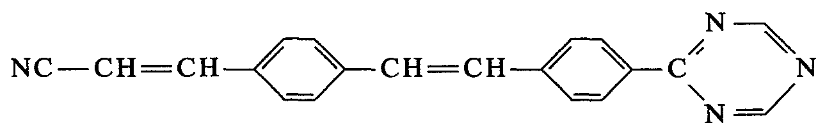

(2001 a)

die aus Dimethylformamid in grüngelben Kristallen mit einem Schmelzpunkt von 248–249 °C kristallisiert.

Verwendet man in obiger Vorschrift an Stelle von 6,3 g 4-Formylzimtsäurenitril die äquivalente Menge an 4-Formylzimtsäuremethylester, dann erhält man bei sonst gleicher Arbeitsweise 9,3 g der Verbindung der Formel

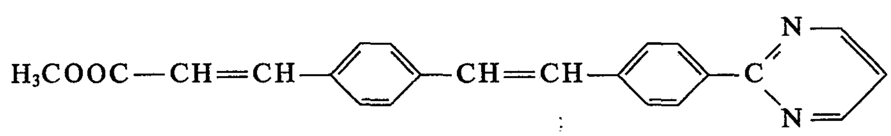

(2002)

mit einem Schmelzpunkt von 249–250°C.

13,7 g der Verbindung der Formel (2002) werden mit 200 ml Äthylenglykolmonomethyläther und 0,1 g Lithiumamid während 4 1/2 Stunden am Rückfluß erhitzt. Nach Abdestillation des Lösungsmittels erhält man 11,3 g der Verbindung der Formel

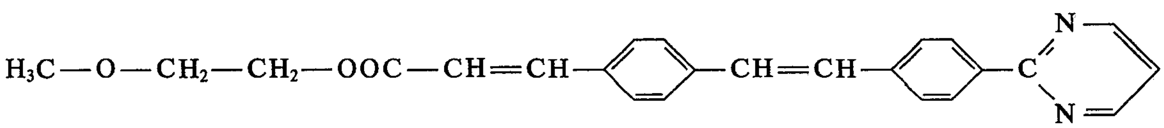

(2003)

welche aus Xylol, in grünlichgelben Kristallen vom Smp. 240°C auskristallisiert.

Das für die Synthese benötigte 2-(4-Diäthoxy-phosphorylmethylphenyl)-pyrimidin wird auf folgendem Weg hergestellt:

95,64 g 2-p-Tolyl-4,4-dichlorpyrimidin werden in 1800 ml wasserfreiem Äthanol unter Zusatz von 72,2 g wasserfreiem Natriumacetat und 10 g Pd/C 5% mit Wasserstoff bei Raumtemperatur hydriert. Nach Abfiltration des Katalysators wird die äthanolische Lösung zur Trockne eingedampft und das angefallene feste Produkt mit Wasser behandelt. Nach dem Trocknen erhält man 61,9 g der Verbindung der Formel

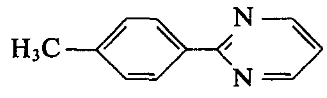

(2004)

als farblose Kristalle vom Schmelzpunkt 88–89°C.

61,3 g 2-p-Tolyl-pyrimidin werden wie im Beispiel 1 beschrieben mit N-Bromsuccinimid in der Seitenkette bromiert. Man erhält 90,3 g der Verbindung der Formel

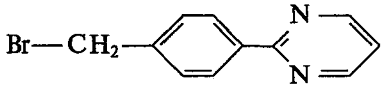

(2005)

welche aus Methanol in farblosen Kristallen vom Schmelzpunkt 103–104°C kristallisiert.

88,3 g 2-(4-Brommethylphenyl)-pyrimidin der Formel (2005) werden mit 350 ml Triäthylphosphit auf 145–150°C erhitzt und 4 Stunden bei dieser Temperatur gerührt. Nach Abdestillation des überschüssigen Triäthylphosphits im Vakuum erhält man 108 g der Verbindung der Formel

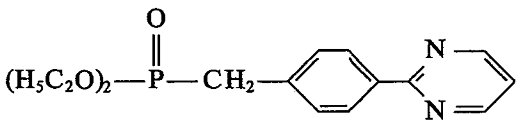

(2006)

als schwachgelbes Öl, das nach einigen Tagen zu Kristallen erstarrt.

Beispiel 21

12,24 g 2-(4-Diäthoxy-phosphorylmethylphenyl)-pyrimidin und 6,85 g 2-Methyl-3-(4-formylphenyl)-2-propennitril (Schmelzpunkt 62–64°C) werden in 80 ml Dimethylformamid gelöst und innerhalb von 10 Minuten mit 2,8 g festem Natriummethylat versetzt. Zur Vervollständigung der Reaktion wird das Reaktionsgemisch noch 2 1/2 Stunden bei 40–45°C gerührt. Danach wird das Gemisch in 800 ml Eiswasser eingerührt, mit Ameisensäure angesäuert, abfiltriert, mit Wasser und Methanol gewaschen und getrocknet. Man erhält 10,7 g der Verbindung der Formel

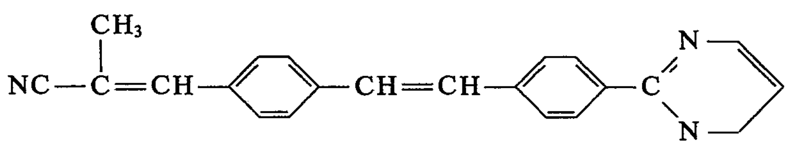

(2101)

Das Produkt kristallisiert aus Tetrachloräthan in Gegenwart von Bleicherde in gelben Kristallen vom Smp. 213–214°C.

Das verwendete 2-Methyl-3-(4-formylphenyl)-2-propennitril wird wie folgt erhalten:

Zu einer Suspension von 134 g Terephthalaldehyd in 500 ml Methanol werden bei 20–25°C 360 g einer 30%igen Natriummethylat-Lösung in Methanol zugetropft. Zur entstandenen Reaktionslösung werden nun bei 20–25°C 191 g 2-(Diäthylphosphoryl)-propionitril [hergestellt gemäß M. L. Raggio und D. S. Watt, J. Org. Chem. 41, 1873 (1976)] langsam zugetropft. Das Reaktionsgemisch wird 6 Stunden bei Zimmertemperatur gerührt, auf 1000 ml Wasser gegossen und die wäßrige Suspension mit Essigsäure auf pH 7 gestellt. Das ausgefallene Produkt wird, nach dem Trocknen, über Kieselgel chromatographiert. Ausbeute 35 g (20,5% der Theorie) als farblose Kristalle vom Smp. 62–64°C.

Verwendet man anstelle des 2-(Diäthylphosphoryl)-propionitrils eine äquivalente Menge an 2-(Diäthylphosphoryl)-propionsäureäthylester so erhält man die Verbindung

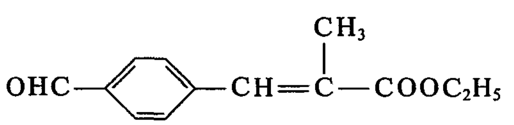

(2102)

vom Sdp. 127–131°C/7 Pa.

Beispiel 22

8,5 g 2-(4-Methylphenyl)-pyrimidin und 15 g 4-(2-Chlor-phenylimino-methyl)-zimtsäuremethylester werden bei 20–22°C in 100 ml Dimethylformamid unter Rühren gelöst. Nun werden 5,4 g Natriummethylat eingetragen, wobei sich das Reaktionsgemisch dunkelbraun färbt und die Temperatur vorübergehend bis auf 27°C ansteigt. Dann wird das Reaktionsgemisch 24 Stunden bei 20–22°C nachgerührt, auf 400 ml Wasser gegossen und das gelb ausgefallene Produkt abgenutscht, mit Wasser gewaschen und getrocknet.

Nach dem Chromatographieren über Kieselgel erhält man die Verbindung der Formel

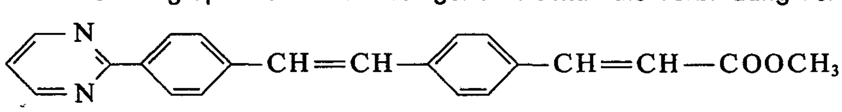

(2201)

vom Smp. 249–250°C.

Der verwendete 4-(2-Chlorphenylimino-methyl)-zimtsäuremethylester der Formel

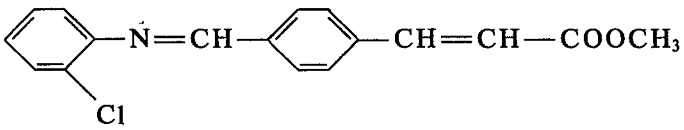 (2202)

wird wie folgt erhalten.

Zu einer Lösung von 19 g 4-Formylzimtsäuremethylester in 100 ml Methanol bei 60°C werden 14 g 2-Chloranilin eingetragen und die entstandene gelbe Lösung wird 2 Stunden lang am Rückfluß nachgerührt. Nach dem Abkühlen auf 5°C wird der entstandene Niederschlag abgenutscht, mit etwas Methanol gewaschen und bei 50°C getrocknet. Nach dem Umkristallisieren aus einem Gemisch, bestehend aus 120 ml Ligroin und 20 ml Toluol erhält man 22,3 g (74,5% der Theorie) der Schiff'schen Base als gelbe Kristalle vom Smp. 89–90°C.

## Beispiel 23

Zu einer Lösung von 8,8 g 2-(4'-Bromstilben-4-yl)-4-methyl-pyrimidin, 9,2 g Tri-n-butylamin und 5,3 g Acrylnitril in 20 ml Toluol werden bei 80°C 0,6 g Tri-o-tolyl-phosphin, gefolgt von 0,11 g Palladiumacetat zugegeben. Das Reaktionsgemisch wird hierauf 4 Stunden bei 90–95°C verrührt, mit 20 ml Toluol verdünnt, auf 20°C abgekühlt und der Niederschlag abgenutscht, mit Methanol gewaschen und getrocknet.

Nach Umkristallisation aus Toluol werden unter Zuhilfenahme von Bleicherde 2,1 g, entsprechend 26% der Theorie, der Verbindung der Formel

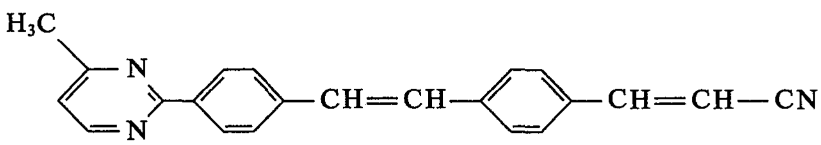 (2301)

als gelbliches Pulver vom Smp. 246–248°C.

Verwendet man anstelle der 5,3 g Acrylnitril eine äquivalente Menge an Acrylsäureäthylester, so erhält man bei sonst gleicher Arbeitsweise die folgende Verbindung vom Smp. 170–171°C.

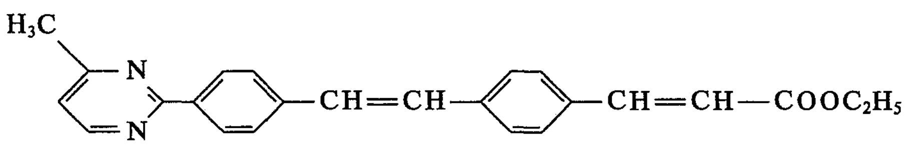 (2302)

Das verwendete 2-(4'-Bromstilben-4-yl)-4-methyl-pyrimidin wird wie folgt erhalten.

Zu einer Lösung von 69 g 2-(4-Diäthoxyphosphorylmethylphenyl)-4-methylpyrimidin und 39 g 4-Brombenzaldehyd in 100 ml Dimethylformamid tropft man bei 40°C 43,5 g einer 30%igen Natriummethylat-Lösung in Methanol zu. Das Reaktionsgemisch wird hierauf 4 Stunden bei 45°C verrührt, auf ein Gemisch, bestehend aus 200 ml Methanol und 200 ml Wasser gegossen und die wäßrige Suspension mit Essigsäure auf pH 7 gestellt. Der Niederschlag wird abgenutscht, mit Wasser gewaschen und getrocknet. Nach dem Umkristallisieren aus einem Gemisch, bestehend aus 250 ml Ligroin und 200 ml Toluol werden 52,2 g, entsprechend 74,5% der Theorie, der Bromverbindung vom Smp. 171–172°C erhalten.

## Beispiel 24

4,7 g 4-(Diäthoxy-phosphoryl-methyl)-benzoesäuremethylester und 3,0 g 4-Formylzimtsäuremethylester werden in 30 ml Dimethylformamid unter Zugabe von 3,4 g einer methanolischen Natriummethylat-Lösung (Gehalt 30%) wie in Beispiel 19 beschrieben kondensiert und nach Zugabe von 50 ml Methanol und 70 ml Wasser nach Neutralisation mit 0,4 ml Essigsäure 50% wie beschrieben isoliert und getrocknet. Nach Umkristallisation aus 400 ml Chlorbenzol unter Zuhilfenahme von Bleicherde erhält man 2,3 g der Verbindung der Formel

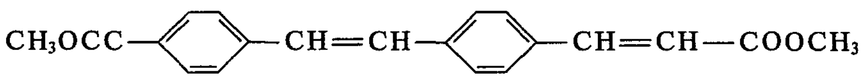 (2401)

als grünstichig gelbes Kristallpulver. Schmelzpunkt: 226–227°C.

Beispiel 25

5,2 g des Phosphonates der Formel

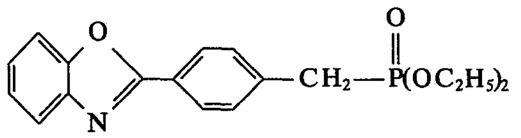 (2501)

und 3,0 g 4-Formylzimtsäuremethylester werden in 30 ml Dimethylformamid unter Zugabe von 3,2 ml einer methanolischen Natriummethylat-Lösung (Gehalt: 30,7%) wie in Beispiel 19 beschrieben, kondensiert und isoliert. Nach zweimaliger Umkristallisation aus je 300 ml Chlorbenzol erhält man 3,0 g der Verbindung der Formel

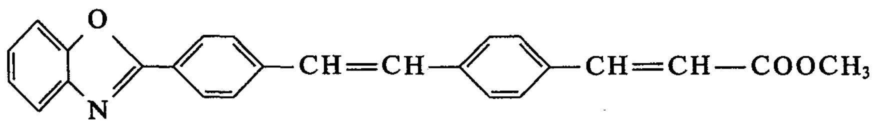 (2502)

als hellgelbes, grünstichiges Kristallpulver. Schmelzpunkt: 244—245°C.

Analog wie in den vorstehenden Beispielen beschrieben, erhält man die Verbindungen der Formel

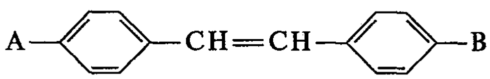 (2503)

Tabelle VII

| Verbindung Nr. | A | B | Schmelzpunkt °C |
|---|---|---|---|
| (2504) | 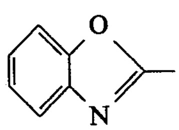 | $-CH{=}CH-COOC_2H_5$ | 290–291 |
| (2505) | 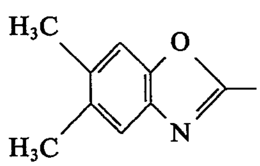 | $-CH{=}CH-COOCH_3$ | 230–231 |
| (2506) | 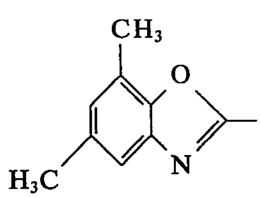 | $-CH{=}CH-COOCH_3$ | 184–185 |
| (2507) | 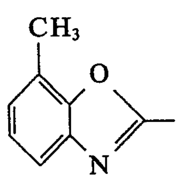 | $-CH{=}CH-COOC_2H_5$ | 183–184 |
| (2508) | 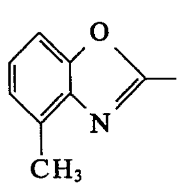 | $-CH{=}CH-COOC_2H_5$ | 197–198 |

Fortsetzung

| Verbindung Nr. | A | B | Schmelzpunkt °C |
|---|---|---|---|
| (2509) | 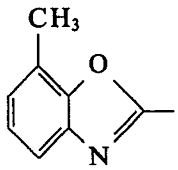 | $-CH{=}CH-COOCH_3$ | 200–244 |
| (2510) | 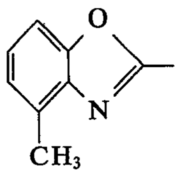 | $-CH{=}CH-COOCH_3$ | 217–238 |
| (2511) | 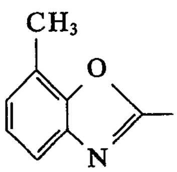 | $-CH{=}CH-CN$ | 217–218 |
| (2512) | 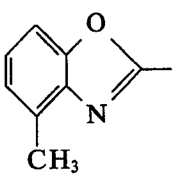 | $-CH{=}CH-CN$ | 236–237 |
| (2513) | 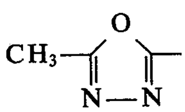 | $-CH{=}CH-COOC_2H_5$ | 192–194 |
| (2514) | 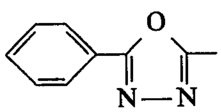 | $-CH{=}CH-COOC_2H_5$ | 223–226 |
| (2515) | 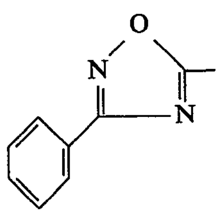 | $-CH{=}CH-COOC_2H_5$ | 231–233 |
| (2516) | 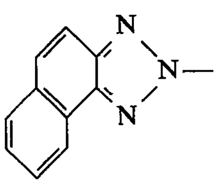 | $-CH{=}CH-COOC_2H_5$ | 242–243 |
| (2517) | 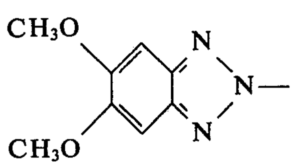 | $-CH{=}CH-COOC_2H_5$ | 301–302 |

Fortsetzung

| Verbindung Nr. | A | B | Schmelzpunkt °C |
|---|---|---|---|
| (2518) | 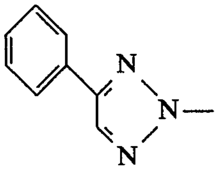 | $-CH{=}CH-COOC_2H_5$ | 166–169 |
| (2519) | 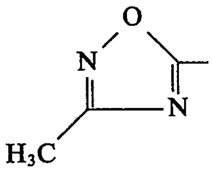 | $-CH{=}CH-COOC_2H_5$ | 174-175 |
| (2520) | 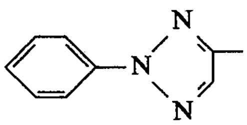 | $-CH{=}CH-COOC_2H_5$ | 166–167 |
| (2521) | 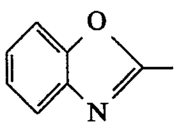 | $-CH{=}CH-CN$ | 259–260 |
| (2522) | 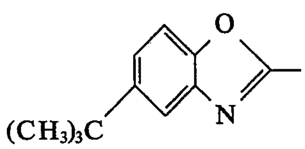 | $-CH{=}CH-COOC_2H_5$ | 243–244 |
| (2523) | 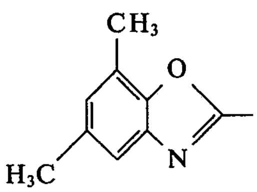 | $-CH{=}CH-CN$ | 232–233 |
| (2524) | 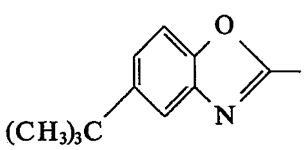 | $-CH{=}CH-CN$ | 240–241 |
| (2525) | 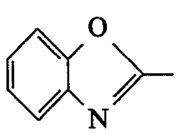 | 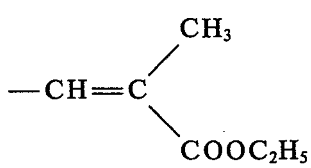 | 241–242 |
| (2526) | 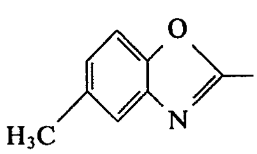 | 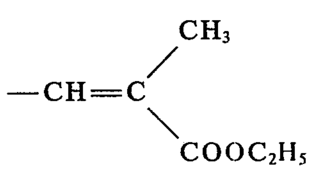 | 282–283 |

Fortsetzung

| Verbindung Nr. | A | B | Schmelzpunkt °C |
|---|---|---|---|
| (2527) | 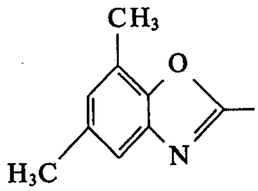 | 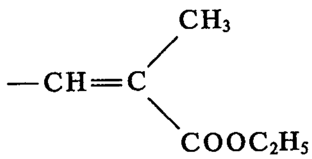 | 147–148 |
| (2528) | 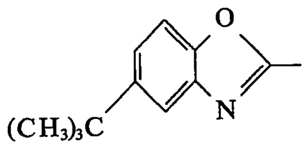 | 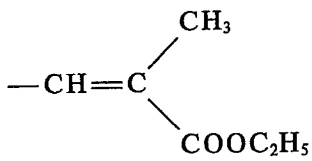 | 178–179 |
| (2529) | 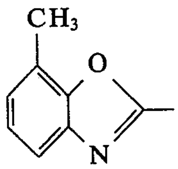 | 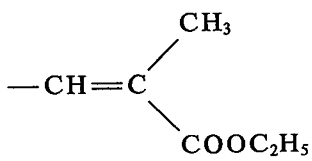 | 121–122 |
| (2530) | 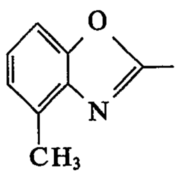 | 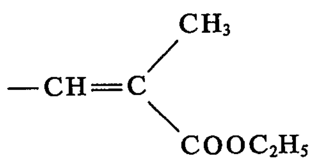 | 144–146 |
| (2531) | 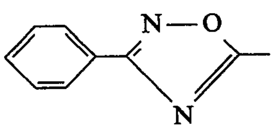 | $-CH=CH-CN$ | 219–220 |
| (2532) | 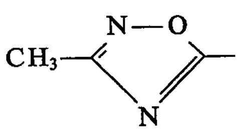 | $-CH=CH-CN$ | 166–167 |
| (2533) | 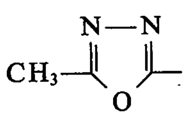 | $-CH=CH-COOCH_3$ | 224–225 |
| (2534) | 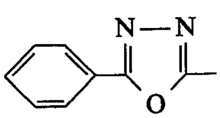 | $-CH=CH-CN$ | 241–242 |
| (2535) | 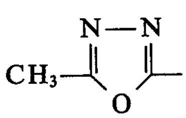 | $-CH=CH-CN$ | 214–215 |
| (2536) | 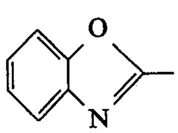 | 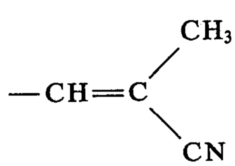 | 223–224 |

Fortsetzung

| Verbindung Nr. | A | B | Schmelzpunkt °C |
|---|---|---|---|
| (2537) | 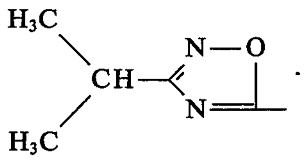 | $-CH=CH-CN$ | 177–178 |
| (2538) | 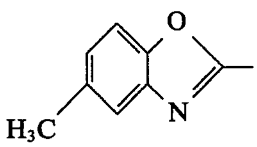 | 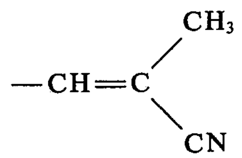 | 248–249 |
| (2539) | 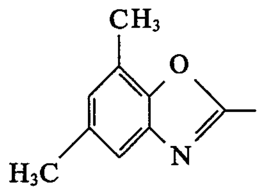 | 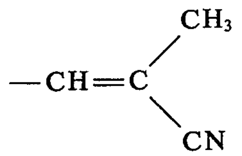 | 183–184 |
| (2540) | 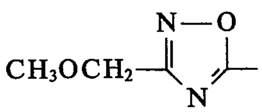 | $-CH=CH-CN$ | 163–165 |
| (2541) | 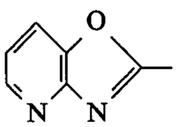 | $-CH=CH-COOCH_3$ | 274–275 |
| (2542) | 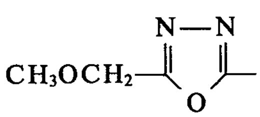 | $-CH=CH-COOCH_3$ | 165–166 |
| (2543) | 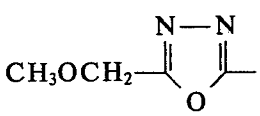 | $-CH=CH-COOCH_2CH_3$ | 180–181 |
| (2544) | 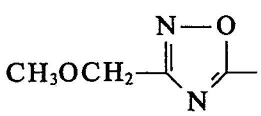 | $-CH=CH-COOCH_3$ | 162–163 |
| (2545) | 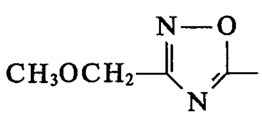 | $-CH=CH-COOCH_2CH_3$ | 181–182 |
| (2546) | 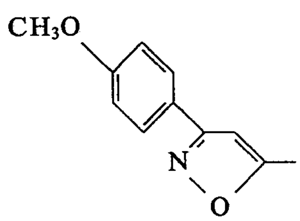 | $-CH=CH-COOCH_3$ | 340–347 |

Fortsetzung

| Verbindung Nr. | A | B | Schmelzpunkt °C |
|---|---|---|---|
| (2547) | 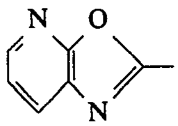 | $-CH{=}CH-COOCH_3$ | 288–289 |
| (2548) | 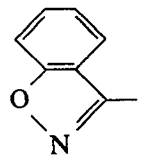 | $-CH{=}CH-COOCH_3$ | 222–223 |
| (2549) | 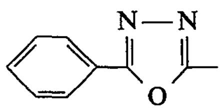 | 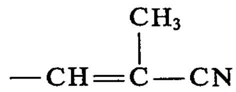 | 226–227 |
| (2550) | 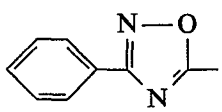 | 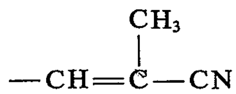 | 218–219 |
| (2551) | 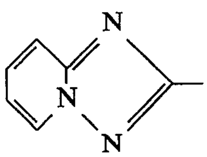 | $-CH{=}CH-COOCH_3$ | 277–278 |

Der für die Herstellung der Verbindung Nr. (2551) verwendete 4-Diäthoxyphosphorylmethylphenylzimtsäuremethylester (Schmelzpunkt 78–80°C) kann analog hergestellt werden, wie in der DE-OS 2 602 750 für den 4-Diäthoxyphosphorylmethylzimtsäureäthylester beschrieben.

## Beispiel 26

11,9 g der Verbindung (2504) werden in 500 ml Methylcellosolve® unter Rühren bei 110°C gelöst. Nach Zugabe einer Lösung von 1,8 g Kaliumhydroxid in 10 ml Wasser wird 30 Minuten bei 110–115°C gerührt, anschließend 3,5 ml konz. Salzsäure zugegeben, auf 5°C abgekühlt und das ausgefallene Produkt abgenutscht. Der feuchte Filterkuchen wird dann mit 100 ml Wasser verrührt, das Produkt wieder abgenutscht, mit Wasser gewaschen und unter Vakuum bei 60°C getrocknet. Man erhält so 10,2 g der Verbindung der Formel

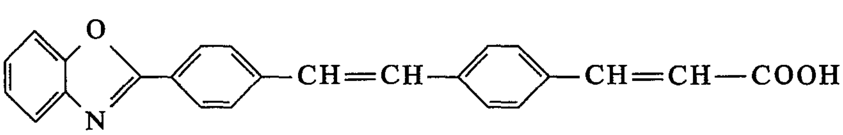 (2601)

als gelbes Kristallpulver. Schmelzpunkt: 330–332°C.

3,7 g der Verbindung der Formel (2601) werden in 100 ml Toluol mit 0,8 ml Thionylchlorid während einer Stunde bei 80–85°C gerührt, wobei nach je 15 Minuten noch 3 mal je 0,8 ml Thionylchlorid zugegeben werden. Dann wird noch 5 Stunden bei 105–110°C nachgerührt, auf 5°C abgekühlt, das auskristallisierte Produkt abgenutscht, mit Hexan gewaschen und unter Vakuum getrocknet. Man erhält so 3,6 g der Verbindung der Formel

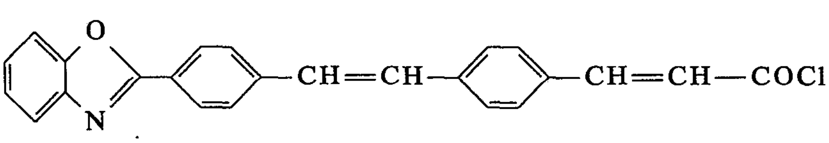 (2602)

als gelbes Pulver. Schmelzpunkt: 292–293°C (unter Zersetzung).

3,6 g der Verbindung der Formel (2602) werden in 100 ml Diäthylenglykolmonomethyläther während 1 1/2 Stunden bei 140°C gerührt. Nach dem Abkühlen auf Raumtemperatur wird das auskristallisierte Produkt abgenutscht, mit Methanol gewaschen und unter Vakuum bei 60°C getrocknet. Man erhält so 3 g der Verbindung der Formel

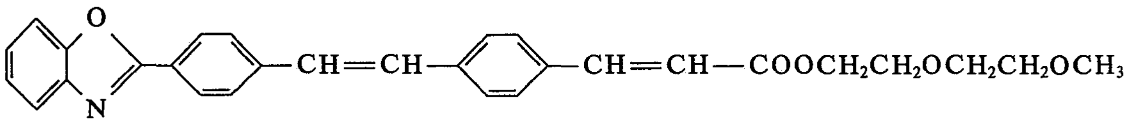

(2603)

welche nach dem Umkristallisieren aus 100 ml Xylol und 100 ml Nonan und nachher aus 500 ml Nonan unter Zuhilfenahme von Bleicherde als gelbes Kristallpulver vom Schmelzpunkt 258–262°C vorliegt.

## Beispiel 27

Etwa 23 mg Natrium werden in 200 ml Tetrahydrofurfurylalkohol gelöst. Dann werden unter Rühren 3,9 g der Verbindung (2504) eingetragen, auf 120–125°C erhitzt und während einer Stunde bei dieser Temperatur gerührt. Die leicht trübe Lösung wird heiß klärfiltriert, das Filtrat auf Raumtemperatur abgekühlt, das auskristallisierte Produkt abgenutscht und unter Vakuum bei 60°C getrocknet. Man erhält so 4,5 g der Verbindung der Formel

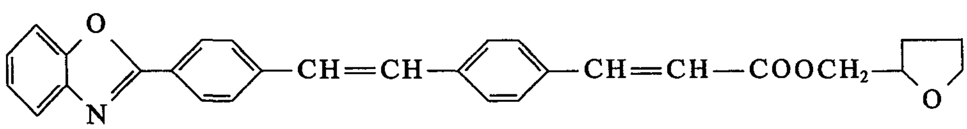

(2701)

welche durch Lösen in 200 ml siedendem Chlorbenzol, Behandlung mit Bleicherde, und Zugabe von 200 ml Hexan bzw. aus 220 ml Nonan umkristallisiert wird. Das so erhaltene hellgelbe Kristallpulver hat einen Schmelzpunkt von 260–262°C. Auf ähnliche Weise können die folgenden Verbindungen erhalten werden

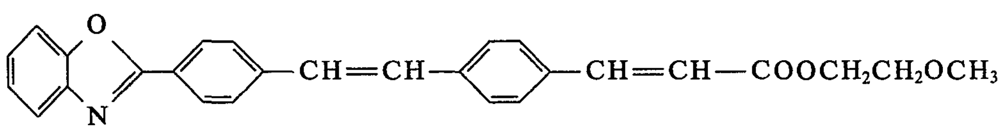

(2702)

Schmelzpunkt: 182°→fest→284°C (es handelt sich hierbei um den Übergang in eine andere Kristallmodifikation).

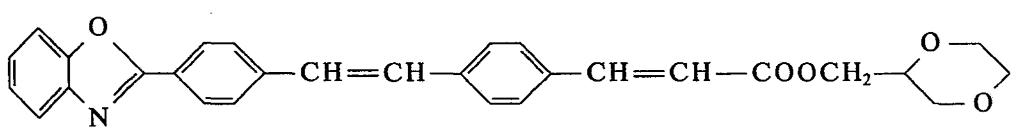

(2703)

Schmelzpunkt: 276–278°C.

## Beispiel 28

6,5 g der Verbindung der Formel

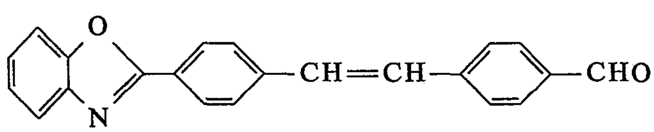

(2801)

werden in 400 ml Dimethylformamid unter Rühren bei 50°C gelöst. Dann werden 4,6 g des Phosphonates der Formel

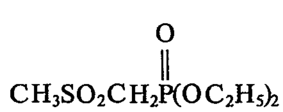

eingetragen und hierauf innert 10 Minuten 4 g einer methanolischen Natrium-methylatlösung (Gehalt 30%) zugetropft. Es wird noch 2 Stunden bei 38—40°C nachgerührt, 400 ml Methanol zugegeben, auf 5°C abgekühlt, das auskristallisierte Produkt abgenutscht und unter Vakuum getrocknet. Nach viermaliger Umkristallisation aus Chlorbenzol unter Zuhilfenahme von Bleicherde erhält man 2,6 g der Verbindung der Formel

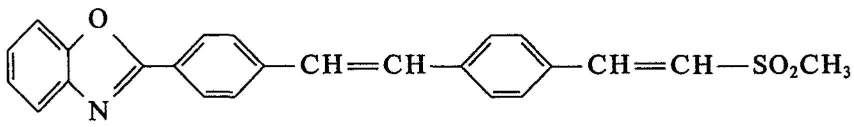
(2802)

als hellgelbes Kristallpulver. Schmelzpunkt: 288—289°C.

Beispiel 29

6,5 g der Verbindung der Formel (2801) werden bei 50°C unter Rühren in 400 ml Dimethylformamid gelöst. Dann werden 5,8 g des Phosphonates der Formel

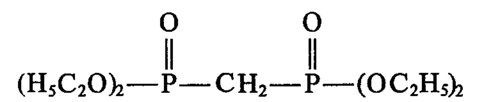

eingetragen und hierauf eine Lösung von 0,5 g Natrium in 15 ml wasserfreiem Äthylalkohol zugetropft. Es wird noch 2 Stunden bei 38—40°C nachgerührt, 400 ml Äthanol zugegeben, auf 5°C abgekühlt, wenig unlösliches Produkt durch Filtration entfernt, mit 800 ml Wasser verdünnt, das ausgefallene Produkt abgenutscht und unter Vakuum getrocknet. Nach Umkristallisation aus 1000 ml Nonan unter Zuhilfenahme von Bleicherde erhält man 3,5 g der Verbindung der Formel

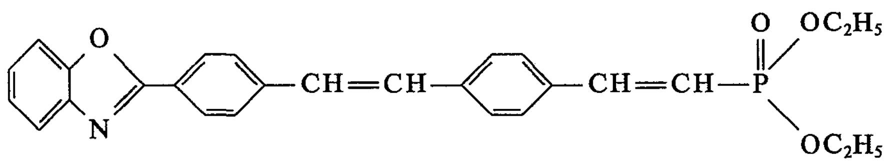
(2901)

als gelbes Kristallpulver. Schmelzpunkt: 252—253°C.

Herstellung der verwendeten Ausgangsprodukte

Die verwendeten Phosphonate sind bekannt oder können in an sich bekannter Weise wie vor- oder nachstehend oder beispielsweise in den DE-OS 2 816 511 oder 2 848 149 beschrieben, erhalten werden.

A. 64,8 g 2-(p-Tolyl)-naphth[1',2':4,5]-1,2,3-triazol werden unter Rühren bei 70—75°C in 1000 ml Tetrachlorkohlenstoff gelöst. Dann werden unter Einstrahlung von UV-Licht innert 21 Minuten 49,0 g N-Bromsuccinimid und 1,3 g Dibenzoylperoxid portionenweise eingetragen und noch 5 Stunden bei 70—75°C nachgerührt. Nach Abkühlen auf 4°C wird das auskristallisierte Produkt abgenutscht, mit 250 ml Tetrachlorkohlenstoff gewaschen, gut abgepreßt, in 500 ml heißem Wasser suspendiert, wieder abgenutscht, mit 7 Liter heißem Wasser gewaschen und unter Vakuum bei 90—100°C getrocknet. Man erhält so 42,8 g der Verbindung der Formel

(a) 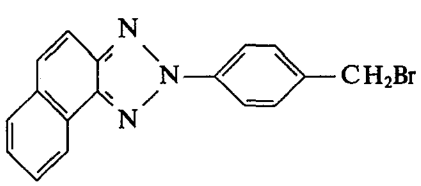

Schmelzpunkt: 225—227°C.

189,5 g dieser Verbindung werden unter Rühren bei 140—145°C innert einer Stunde in 400 ml Triäthylphosphit eingetragen und noch 6 Stunden bei 145—150°C nachgerührt. Aus dem Reaktionsgemisch wird das überschüssige Triäthylphosphit und das entstandene Äthyl-diäthylphosphonat unter Wasserstrahlvakuum abdestilliert. Als Rückstand erhält man so 209,5 g der Verbindung der Formel

(b) 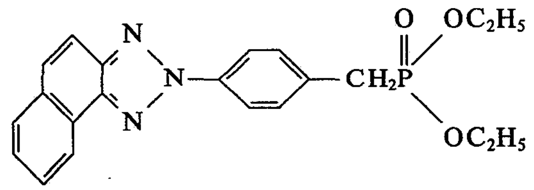

als viskoses Öl, welches nach ca. 3 Monaten durchkristallisiert.

B. 11 g Acetamidoxim-Hydrochlorid werden 200 ml N-Methylpyrrolidon unter Rühren bei Raumtemperatur suspendiert. Nach Zugabe von 8,4 g Natriumbicarbonat wird auf 50°C erhitzt und 30 Minuten ohne weiteres Erwärmen nachgerührt. Dann werden weitere 8,4 g Natriumbicarbonat und anschließend 18,9 g 4-Chlormethylbenzoylchlorid zugegeben, wobei die Temperatur wieder auf 49°C ansteigt. Innert 10 Minuten wird dann auf 160—165°C erhitzt, 5 Minuten bei dieser Temperatur gehalten, Abkühlen gelassen, in 1000 ml Wasser gegossen, das ausgefallene Produkt abgenutscht, mit Wasser gewaschen und unter Vakuum getrocknet. Man erhält so 13,3 g der Verbindung der Formel

(c) 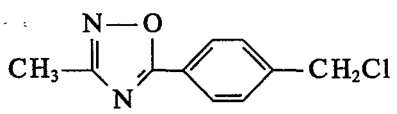

Schmelzpunkt: 89—91°C.

23,9 g dieser Verbindung werden in 37,5 g Tributylphosphit unter Stickstoff während 10 Minuten auf 210—220°C (Badtemperatur) erhitzt. Dann wird unter Vakuum das überschüssige Tributylphosphit abdestilliert. Man erhält so 38,4 g der Verbindung der Formel

(d) 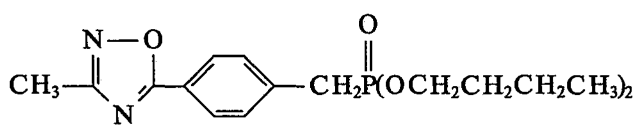

als rotbraunes Öl.

C. 66,7 g Acethydrazid werden in 5000 ml Dioxan bei Raumtemperatur unter Rühren vorgelegt. Nach Zugabe von 54 g Magnesiumoxid werden 170,1 g 4-Chlormethylbenzoylchlorid langsam zugegeben. Dann wird 18 Stunden bei 40—45°C gerührt, abgenutscht, der Rückstand viermal mit je 4 Liter Dioxan extrahiert und das so erhaltene Produkt aus 1000 ml Chlorbenzol umkristallisiert. Man erhält so 123 g der Verbindung der Formel

(e) 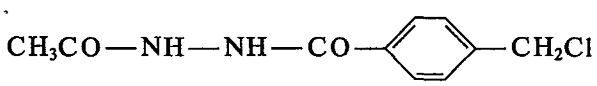

Schmelzpunkt: 174—175°C.

45,2 g dieser Verbindung werden mit 30,7 g Phosphoroxychlorid in 450 ml Cyclohexan unter Rühren während 2 Stunden unter Rückfluß gekocht. Dann werden noch 18,3 ml Phosphoroxychlorid und 250 ml Toluol zugegeben und weitere 3 Stunden unter Rückfluß gekocht. Nach Zugabe von weiteren 250 ml Toluol wird noch 2 Stunden unter Rückfluß gekocht, von etwas klebrigem Rückstand dekantiert und unter Vakuum zur Trockne eingedampft. Man erhält so 32,5 g der Verbindung der Formel

(f) 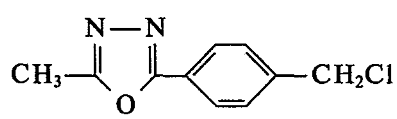

Schmelzpunkt: 73—75°C. 32,5 g dieser Verbindung werden in 78 g Tributylphosphit unter Stickstoff während 30 Minuten auf 200°C erhitzt. Nach dem Abdestillieren des überschüssigen Tributylphosphits unter Vakuum erhält man 57,0 g der Verbindung der Formel

(g) 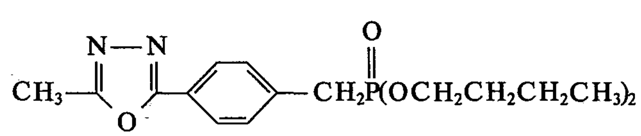

als braunes Öl, welches nach einiger Zeit erstarrt.

Auf analoge Weise wie vorstehend beschrieben, können die in der folgenden Tabelle aufgeführten Verbindungen der Formel

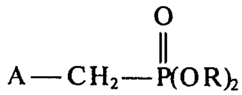

erhalten werden.

| Nr. | A | R | Schmelzpunkt °C |
|---|---|---|---|
| h | 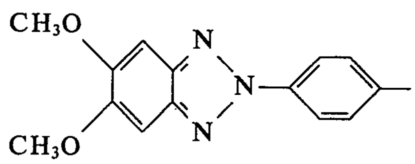 | $-C_2H_5$ | 154–156 |
| i | 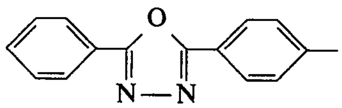 | $-C_2H_5$ | 119–121 |
| j | 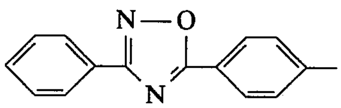 | $-C_2H_5$ | ca. 60 (unscharf) |
| k | 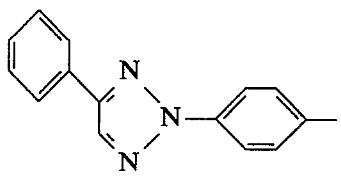 | $-C_2H_5$ | Öl |
| l | 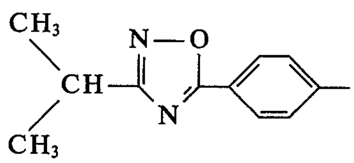 | $-C_4H_9(n)$ | Öl |
| m | 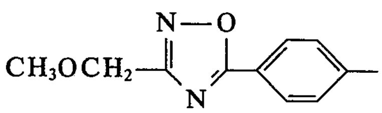 | $-C_2H_5$ | flüssig |
| n | 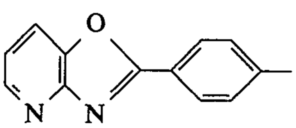 | $-C_2H_5$ | 124–125 |
| o | 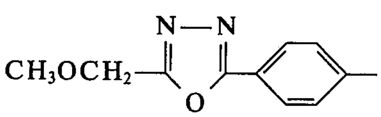 | $-C_4H_9(n)$ | flüssig |
| p | 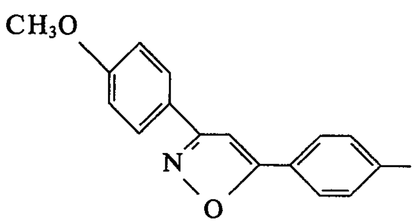 | $-C_2H_5$ | 120–121 |

Fortsetzung

| Nr. | A | R | Schmelzpunkt °C |
|---|---|---|---|
| q | 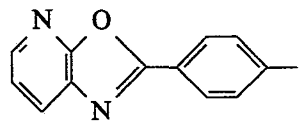 | $-CH_3$ | schmierig, z. T. fest |
| r | 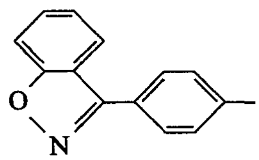 | $-C_2H_5$ | schmierig |

Beispiel 30

Zu einer Lösung von 16 g 2-(4-Diäthoxyphosphorylmethylphenyl)-5-methylpyrimidin und 9,5 g 4-Formyl-zimtsäuremethylester in 100 ml Dimethylformamid tropft man bei Raumtemperatur innerhalb 15 Minuten 11 g einer 30%igen Natriummethylat-Lösung in Methanol zu. Die Temperatur steigt bis auf 40°C. Das Reaktionsgemisch wird hierauf 2 Stunden bei 40°C verrührt, auf ein Gemisch, bestehend aus 160 ml Methanol und 250 ml Wasser gegossen und die wäßrige Suspension mit Essigsäure auf pH 7 gestellt. Der Niederschlag wird abgenutscht, mit Wasser gewaschen und getrocknet. Nach mehrmaliger Umkristallisation aus o-Dichlorbenzol werden unter Zuhilfenahme von Bleicherde 9,5 g, entsprechend 53% der Theorie, der Verbindung der Formel

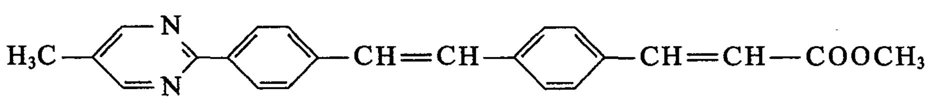 (3001)

als gelbliches Pulver vom Smp. 270–272°C erhalten.

Das verwendete Phosphonat der Formel

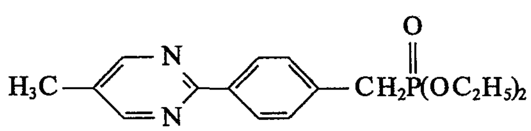 (3002)

wird wie folgt erhalten:

Zu einer Lösung von 170 g p-Tolylamidinhydrochlorid und 125 g 3-Äthoxy-2-methyl-acrolein in 500 ml Methanol werden bei 50°C 200 g einer 30%igen Natriummethylat-Lösung in Methanol innerhalb von 45 Minuten zugetropft. Das Reaktionsgemisch wir hierauf 4 Stunden bei 50°C gerührt, auf 2000 ml Wasser gegossen und der Niederschlag abgenutscht, mit Wasser gewaschen und getrocknet. Man erhält 153 g (83% der Theorie) von 2-(4-Methylphenyl)-5-methylpyrimidin vom Smp. 122–123°C.

Durch Umsetzung dieses 2-(4-Methylphenyl)-5-methylpyrimidin mit N-Bromsuccinimid, wie in Beispiel 15 beschrieben, erhält man, nach Umkristallisation aus Ligroin das 2-(4-Brommethylphenyl)-5-methylpyrimidin vom Smp. 129–131°C.

Ein Gemisch, bestehend aus 184 g 2-(4-Brommethylphenyl)-5-methylpyrimidin und 465 g Triäthylphosphit wird langsam unter Rühren auf 150°C erwärmt, unter gleichzeitiger Abdestillation des gebildeten Äthylbromids, und dann 5 Stunden bei dieser Temperatur gerührt. Nach Abdestillation des überschüssigen Triäthylphosphits erhält man 220 g des Phosphonates als hellbraunes dickes Öl.

Beispiel 31

Ein Polyestergewebe (Terylen Typ 540) wird auf einen Färbeapparat bei einem Flottenverhältnis von 1 : 20 mit einem wäßrigen Bad behandelt, welches 0,1 %, (bezogen auf das Warengewicht) der Verbindung der Formel (101), (108), (401), (1701) oder (1501) und 1 g/Liter des Kondensationsproduktes von 35 Mol Äthylenoxid mit 1 Mol Stearylalkohol enthält. Man erwärmt nun das Bad innerhalb 30 Minuten von 40 auf 120°C, behält es während 30 Minuten auf dieser Temperatur und kühlt es dann innerhalb 15 Minuten auf 15°C ab. Das Gewebe wird anschließend in fließendem deionisiertem Wasser gespült und bei 70°C getrocknet. Das so behandelte Polyestergewebe weist einen hohen Aufhelleffekt auf.

Beispiel 32

Man foulardiert bei Raumtemperatur ein Polyestergewebe (Terylen Typ 540) mit einer Flotte, welche pro Liter 1 g der Verbindung der Formel (2502), (2508), (901), (2302) oder (1601) und 1 ml des Kondensationsproduktes von 8—9 Mol Äthylenoxid mit 1 Mol p-tert.-Octylphenol enthält. Der Abquetscheffekt beträgt 80%. Anschließend wird 10 Minuten bei 80°C getrocknet und dann 30 Sekunden bei 200°C thermofixiert. Das so behandelte Gewebe weist einen hohen Aufhelleffekt auf.

Beispiel 33

Ein Polyester-Baumwoll-Mischgewebe wird auf einem Färbeapparat bei einem Flottenverhältnis von 1 : 20 mit einem wäßrigen Bad behandelt, welches 0,1 %, bezogen auf das Warengewicht, der Verbindung der Formel (104), (901), (907), (904) oder (1507) und 1 g/Liter des Kondensationsproduktes von 35 Mol Äthylenoxid mit 1 Mol Stearylalkohol enthält. Man erwärmt nun das Bad innerhalb 30 Minuten von 40 auf 97°C, behält es während 30 Minuten auf dieser Temperatur und kühlt es dann innerhalb 15 Minuten auf 15°C ab. Das Gewebe wird anschließend in fließendem, deionisiertem Wasser gespült und bei 70°C getrocknet. Das so behandelte Polyester-Baumwoll-Mischgewebe zeichnet sich durch einen hohen Aufhelleffekt aus.

Beispiel 34

Ein Polyamid-6,6-Webtricot wird auf einem Färbeapparat bei einem Flottenverhältnis von 1 : 20 mit einem wäßrigen Bad behandelt, welches 0,2%, bezogen auf das Warengewicht, der Verbindung (601), (901), (1601) oder (2507) und 3 g/Liter eines Gemisches von 60 Gewichtsteile Natriumhydrosulfit und 40 Gewichtsteile Natriumpyrophosphat enthält. Man erwärmt das Bad innerhalb 30 Minuten von 40 auf 130°C, behält es während 30 Minuten auf dieser Temperatur und kühlt dann innerhalb 15 Minuten auf 15°C ab. Das Gewebe wird anschließend in fließendem deionisiertem Wasser gespült und bei 60°C getrocknet. Das so behandelte Polyamidgewebe weist einen hohen Aufhelleffekt auf.

Beispiel 35

Ein Triacetatgewebe wird auf einem Färbeapparat bei einem Flottenverhältnis von 1 : 20 mit einem wäßrigen Bad behandelt, welches 0,1 %, bezogen auf das Warengewicht, der Verbindung der Formel (301), (501), (601) oder (901) und 1 g/Liter des Kondensationsproduktes von 35 Mol Äthylenoxid mit 1 Mol Stearylalkohol enthält. Man erwärmt nun das Bad innerhalb 30 Minuten von 40 auf 97°C, behält es während 30 Minuten auf dieser Temperatur und kühlt es dann innerhalb 15 Minuten auf 30°C ab. Das Gewebe wird anschließend in fließendem, deionisiertem Wasser gespült und bei 60°C getrocknet. Das so behandelte Triacetatgewebe weist einen hohen Aufhelleffekt auf.

Beispiel 36

Ein Acetatsatingewebe wird auf einem Färbeapparat bei einem Flottenverhältnis von 1 : 20 mit einem wäßrigen Bad behandelt, welches 0,1 %, bezogen auf das Warengewicht, der Verbindung der Formel (903), (1001), (1507), (1602) oder (2513), 1 g/Liter des Kondensationsprodukt von 35 Mol Äthylenoxid mit 1 Mol Stearylalkohol und 0,5 ml/Liter Essigsäure 80% enthält. Man erwärmt nun das Bad innerhalb 30 Minuten von 40 auf 80°C, behält es während 30 Minuten bei dieser Temperatur und kühlt es dann innerhalb 15 Minuten auf 20°C ab. Das Gewebe wird anschließend in fließendem, deionisiertem Wasser gespült und bei 60°C getrocknet. Das so behandelte Acetatsatin-Gewebe zeichnet sich durch einen hohen Aufhelleffekt aus.

Beispiel 37

1000 g Polyestergranulat vom Typ Äthylenglykolterephthalat, enthaltend 0,5% $TiO_2$ (Anatastyp), werden mit 0,5 g einer Verbindung der Formel (101), (106), (901) oder (904) in einem Rhönradmischer gemischt und das so behandelte Granulat in einer Extruderspinnanlage bei 280°C zu einem Multifilament versponnen. Die entstandenen Fäden zeigen einen ausgezeichneten Aufhelleffekt von guter Lichtechtheit.

Beispiel 38

100 Teile Polystyrol, enthaltend ca. 1,5% $TiO_2$ (Rutiltyp), werden mit 0,05 Teilen einer Verbindung (1506), (1505), (1601) oder (2515) trocken vermischt und auf einem Extruder bei 180°C zu einem aufgehellten Granulat verarbeitet. Das Granulat wird mit Hilfe einer Kolbenspritzgußmaschine zu Plättchen verformt. Die so erhaltenen Plättchen weisen einen starken Aufhelleffekt von guter Lichtechtheit auf.

Beispiel 39

Eine innige Mischung aus 65 Teilen Polyvinylchlorid (Suspensiontyp), 32 Teilen Dioctylphthalat, 3 Teilen eines epoxidierten Sojabohnenöls, 1,5 Teilen eines Stabilisators, 0,5 Teilen eines Costabilisators, 5 Teilen $TiO_2$ (Rutiltyp) und 0,05 Teilen einer Verbindung der Formel (105), (106), (701), (902) oder (1504) werden auf einem Kalander bei 150°C in einer Folie ausgewalzt. Die erhaltene Folie weist einen starken Aufhelleffekt von guter Lichtechtheit auf.

Beispiel 40

Ein Polyestergewebe (Terylen® 540) wird auf einem Färbeapparat bei einem Flottenverhältnis 1 : 20 mit einem wäßrigen Bad behandelt, welches

0,1 %, bezogen auf das Gewebegewicht, der Aufhellermischung enthaltend im Verhältnis 1 : 2 die Aufheller der Formel (2502) und der Formel

(A) 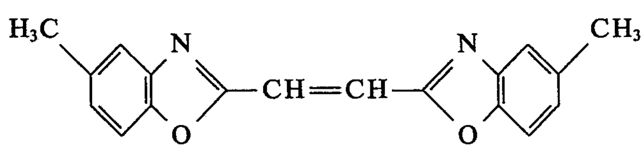

und

1 g/l des Adduktes von 35 Mol Äthylenoxid an 1 Mol Stearylalkohol

enthält. Die Applikation erfolgt gemäß folgendem Temperaturprogramm:

von 40 auf 120°C in 30 Minuten; bei 120°C während 30 Minuten
von 120 auf 40°C in 15 Minuten.

Das Gewebe wird dann in fließendem deionisiertem Wasser gespült und mit einem 180–190°C warmem Bügeleisen getrocknet. Das so behandelte Gewebe weist einen höheren Aufhelleffekt auf als die einzelnen Mischungskomponenten.

Eine Mischung enthaltend die Verbindung (2502) und die Verbindung (A) im Verhältnis 2 : 1 bewirkt ebenfalls einen höheren Aufhelleffekt.

Beispiel 41

Man foulardiert bei Raumtemperatur ein Polyestergewebe (Terylen® 540) mit einer wäßrigen Flotte, die

1 g/l einer Aufhellermischung, bestehend aus den Aufhellern der Formel (1601) und der Formel

(B) 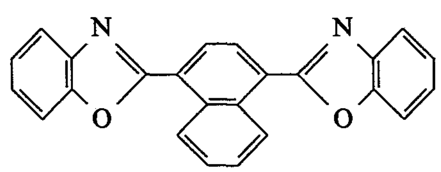

im Verhältnis 1 : 2 bzw. 2 : 1 und

1 ml/l des Adduktes von 8–9 Mol Äthylenoxid an 1 Mol p-tert. Octylphenol

enthält, bis zu einer Flottenaufnahme von 80%. Anschließend wird 10 Minuten lang bei 80°C getrocknet und 30 Sekunden bei 180°C thermofixiert. Das so behandelte Gewebe weist einen höheren Aufhelleffekt auf als die einzelnen Mischungskomponenten.

**Patentansprüche**

1. 4-Heterocyclyl-4'-vinyl-stilbene der Formel

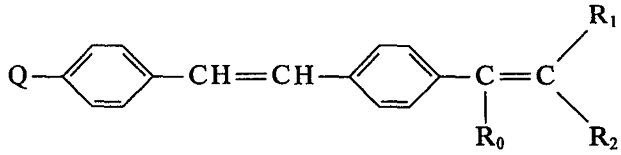

worin

Q einen monocyclischen 5- oder 6gliedrigen unsubstituierten oder nicht-chromophor substituierten, 0, 1 oder 2 ankondensierte Benzolringe aufweisenden aromatischen heterocyclischen Ring oder einen bicyclischen 9gliedrigen aromatischen heterocyclischen Ring,
$R_0$ Wasserstoff, unsubstituiertes oder nicht-chromophor substituiertes Alkyl,
$R_1$ Wasserstoff, unsubstituiertes oder nicht-chromophor substituiertes Alkyl, Alkoxycarbonyl, Carbamoyl oder Sulfonamido; Alkenyl, Carboxy, Alkyl-, Aryl- oder Aralkylsulfonyl, Cyano, Sulfo oder Phosphonsäuredialkylester und
$R_2$ Wasserstoff, unsubstituiertes oder nicht-chromophor substituiertes Alkyl oder Alkenyl bedeuten, wobei höchstens eines der Symbole $R_1$ und $R_2$ Wasserstoff bedeutet.

2. 4-Heterocyclyl-4'-vinyl-stilbene gemäß Anspruch 1 der Formel

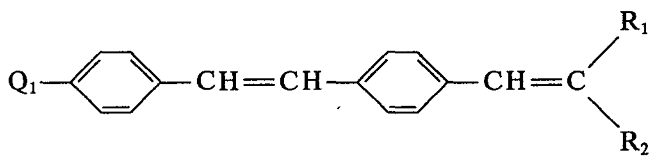

worin

$R_1$ und $R_2$ die in Anspruch 1 angegebene Bedeutung haben und

$Q_1$ einen Rest der Formel

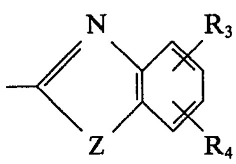
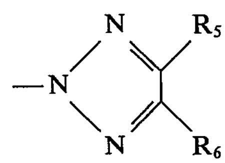
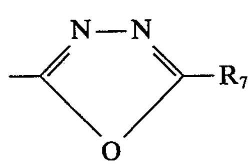
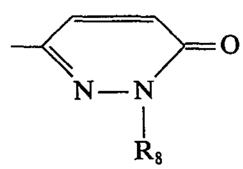

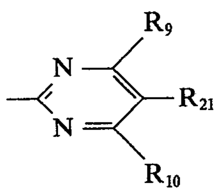
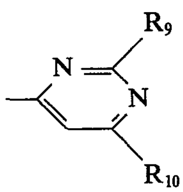
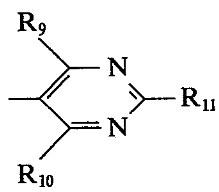
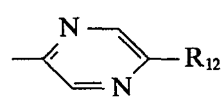

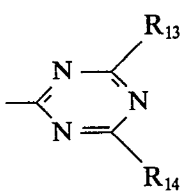
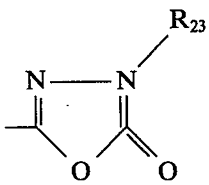
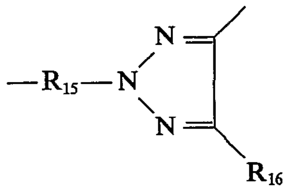
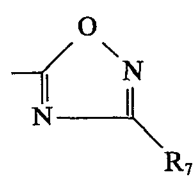

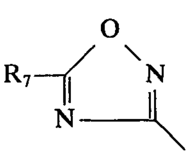
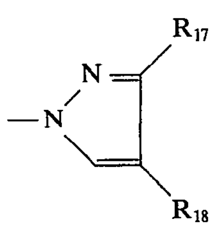
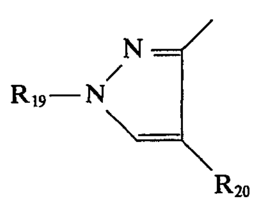
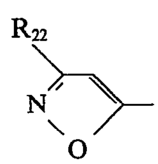

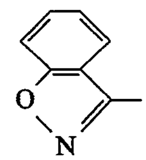 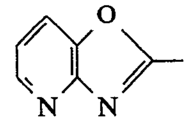 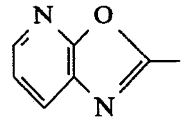 oder 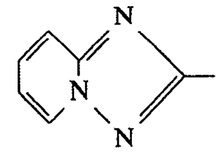

bedeuten, in welchen Resten

$R_3$ Wasserstoff, Chlor, $C_1$—$C_4$-Alkyl, Phenyl-$C_1$—$C_3$-Alkyl, Cyclohexyl, Phenyl, $C_1$—$C_4$-Alkoxy, $C_1$—$C_4$-Alkylsulfonyl, $C_1$—$C_4$-Alkoxycarbonyl, Cyano oder Carboxy oder zusammen mit $R_4$ einen gegebenenfalls durch 1 bis 4 Methylgruppen substituierten ankondensierten 1-Cyclopenten-, 1-Cyclohexen- oder Benzolring,

$R_4$ Wasserstoff, Chlor oder $C_1$—$C_4$-Alkyl oder zusammen mit $R_3$ einen gegebenenfalls durch 1 bis 4 Methylgruppen substituierten ankondensierten 1-Cyclopenten-, 1-Cyclohexen- oder Benzolring,

$R_5$ Wasserstoff, $C_1$—$C_4$-Alkyl, Cyano, COOR, worin R für $C_1$—$C_4$-Alkyl steht, Phenyl oder Styryl oder zusammen mit $R_6$ einen gegebenenfalls durch $C_1$—$C_4$-Alkyl, $C_1$—$C_4$-Alkoxy oder Chlor substituierten ankondensierten Benzolring oder einen ankondensierten Naphthalinring,

$R_6$ Wasserstoff, $C_1$—$C_4$-Alkyl, Phenyl oder zusammen mit $R_5$ einen gegebenenfalls durch $C_1$—$C_4$-Alkyl, $C_1$—$C_4$-Alkoxy oder Chlor substituierten ankondensierten Benzolring oder einen ankondensierten Naphthalinring,

$R_7$ unsubstituiertes oder nicht-chromophor substituiertes $C_1$—$C_4$-Alkyl, gegebenenfalls durch $C_1$—$C_4$-Alkyl, $C_1$—$C_4$-Alkoxy, $C_1$—$C_4$-Alkoxycarbonyl, Cyano oder Chlor substituiertes Phenyl, Styryl, Biphenylyl oder Naphthyl,

$R_8$ Wasserstoff, $C_1$—$C_4$-Alkyl oder unsubstituiertes oder durch Chlor oder Methyl substituiertes Phenyl,

$R_9$ und $R_{10}$ unabhängig voneinander Wasserstoff, $C_1$—$C_4$-Alkyl, unsubstituiertes oder durch Chlor oder Methyl substituiertes Phenyl, $C_1$—$C_4$-Alkoxy, $C_3$—$C_8$-Alkoxyalkoxy, unsubstituiertes oder durch Chlor oder Methyl substituiertes Phenoxy, Chlor, $C_1$—$C_4$-Alkylthio, Phenylthio, $C_1$—$C_4$-Alkylamino, Di-$C_1$—$C_4$-alkylamino, Morpholino, Piperidino, Piperazino, Pyrrolidino oder Anilino,

$R_{11}$ Wasserstoff, $C_1$—$C_4$-Alkyl oder unsubstituiertes oder durch Chlor oder Methyl substituiertes Phenyl,

$R_{12}$ $C_1$—$C_4$-Alkoxy, $C_3$—$C_8$-Alkoxyalkoxy, $C_1$—$C_4$-Alkylthio, unsubstituiertes oder durch Chlor oder Methyl substituiertes Phenoxy, Cycloalkoxy, $C_1$—$C_4$-Alkylthio, unsubstituiertes oder durch Chlor oder Methyl substituiertes Phenylthio, $C_1$—$C_4$-Alkylamino, Di-$C_1$—$C_4$-alkylamino, Morpholino, Piperidino, Piperazino, Pyrrolidino oder Anilino,

$R_{13}$ und $R_{14}$ Wasserstoff, Halogen, $C_1$—$C_4$-Alkoxy, Phenyl, Aralkoxy, Cycloalkoxy, Aryloxy, $C_1$—$C_4$-Alkylmercapto, $C_1$—$C_4$-Alkylamino, Di-$C_1$—$C_4$-Alkylamino, Morpholino, Piperidino, Piperazino, Pyrrolidino, Arylamino oder $C_1$—$C_4$-Alkyl,

$R_{15}$ unsubstituiertes oder durch Chlor oder $C_1$—$C_4$-Alkyl substituiertes Phenyl,

$R_{16}$ Wasserstoff oder $C_1$—$C_4$-Alkyl,

$R_{17}$ unsubstituiertes oder durch Chlor oder $C_1$—$C_4$-Alkyl substituiertes Phenyl,

$R_{18}$ Wasserstoff oder $C_1$—$C_4$-Alkyl,

$R_{19}$ unsubstituiertes oder durch Chlor oder $C_1$—$C_4$-Alkyl substituiertes Phenyl,

$R_{20}$ Wasserstoff oder $C_1$—$C_4$-Alkyl,

$R_{21}$ Wasserstoff oder $C_1$—$C_4$-Alkyl,

$R_{22}$ unsubstituiertes oder durch $C_1$—$C_4$-Alkoxy substituiertes Phenyl,

$R_{23}$ Wasserstoff, $C_1$—$C_4$-Alkyl oder unsubstituiertes oder durch $C_1$—$C_4$-Alkyl, Halogen oder Cyano substituiertes Phenyl und

Z O, S oder NX bedeuten, worin X für Wasserstoff, $C_1$—$C_4$-Alkyl, Acetyl, Benzoyl oder Phenyl steht.

3. 4-Heterocyclyl-4'-vinyl-stilbene gemäß Anspruch 2 der Formel

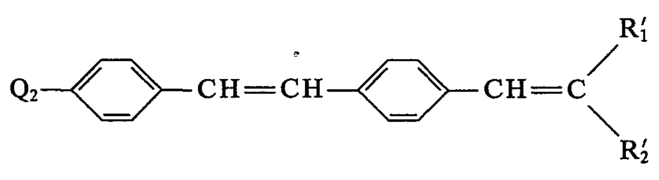

worin $R_1'$ Wasserstoff, $C_1$—$C_6$-Alkyl, durch $C_1$—$C_4$-Alkoxy oder $C_2$—$C_5$-Alkoxycarbonyl substituiertes $C_1$—$C_6$-Alkyl, $C_2$—$C_4$-Alkenyl, Cyano, $COOR^o$ worin $R^o$ für $C_1$—$C_4$-Alkyl, $C_1$—$C_4$-Alkoxy-$C_2$—$C_4$-alkyl, $C_1$—$C_4$-Alkoxy-$C_2$—$C_4$-alkoxy-$C_2$—$C_4$-alkyl, $C_1$—$C_4$-Alkoxy-$C_2$—$C_4$-alkoxy-$C_2$—$C_4$-alkoxy-$C_2$—$C_4$-alkyl, Tetrahydro-2-furyl-methyl, 1,4-Dioxa-2-cyclohexyl-methyl, Allyl, $C_1$—$C_4$-Alkylamino-$C_1$—$C_4$-alkyl oder Di-$C_1$—$C_4$-alkylamino-$C_1$—$C_4$-alkyl steht, —CON(R')(R''), worin R' für Wasserstoff, $C_1$—$C_6$-Alkyl, durch Hydroxy, $C_1$—$C_4$-Alkoxy, $C_2$—$C_6$-Hydroxyalkoxy, $-SO_3M$ oder Mono- oder Di-$C_1$—$C_4$-alkylamino substituiertes $C_2$—$C_6$-Alkyl, Cyclohexyl, Benzyl oder Phenäthyl und R'' für Wasserstoff, $C_1$—$C_6$-Alkyl, durch

Hydroxy, $C_1$—$C_4$-Alkoxy oder $C_2$—$C_6$- Hydroxyalkoxy substituiertes $C_2$—$C_6$-Alkyl stehen, $-SO_2N(R')(R'')$ worin R' und R'' die vorstehende Bedeutung haben, $-SO_3M$, $R_x-SO_2-$ worin $R_x$ für $C_1$—$C_6$-Alkyl, durch $C_1$—$C_4$-Alkoxy oder $C_1$—$C_4$-Alkoxy-$C_2$—$C_6$-alkoxy substituiertes $C_2$—$C_6$-Alkyl, Phenyl oder Benzyl steht, oder Phosphonsäuredialkylester,

$R'_2$ Wasserstoff oder $C_1$—$C_6$-Alkyl,

M Wasserstoff oder ein bei optischen Aufhellern übliches, nicht-chromophores Kation, und

$Q_2$ einen Rest der Formel

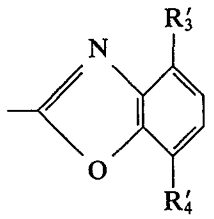 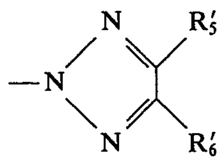 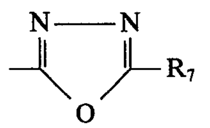 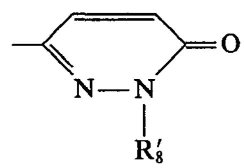

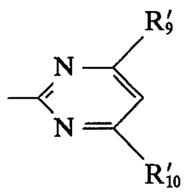 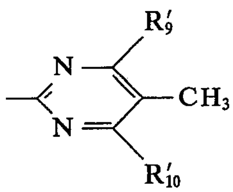 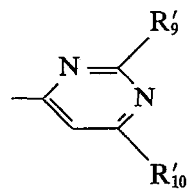 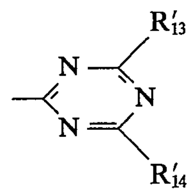

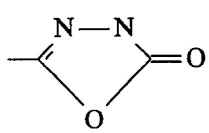 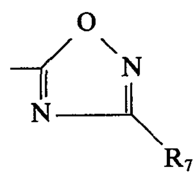 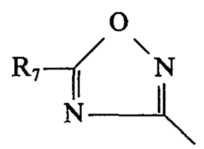 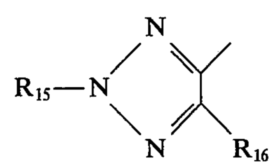

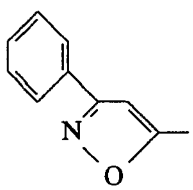 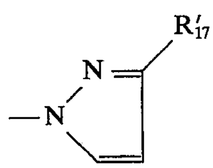 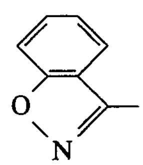 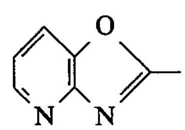 oder

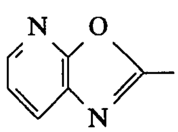

bedeuten, worin

$R'_3$ Wasserstoff, Methyl, Chlor oder $C_1$—$C_4$-Alkoxy,

$R'_4$ Wasserstoff, Chlor, $C_1$—$C_4$-Alkyl, Phenyl, $C_1$—$C_4$-Alkoxy oder Phenoxy,

$R'_5$ Wasserstoff, $C_1$—$C_4$-Alkyl, Phenyl oder Styryl oder zusammen mit $R'_6$ einen ankondensierten unsubstituierten oder durch $C_1$—$C_4$-Alkyl, $C_1$—$C_4$-Alkoxy oder Chlor substituierten Benzolring oder einen ankondensierten Naphthalinring,

$R'_6$ Wasserstoff, $C_1$—$C_4$-Alkyl, Phenyl oder zusammen mit $R'_5$ einen ankondensierten unsubstituierten oder durch $C_1$—$C_4$-Alkyl, $C_1$—$C_4$-Alkoxy oder Chlor substituierten Benzolring oder einen ankondensierten Naphthalinring,

$R'_7$ unsubstituiertes oder nicht-chromophor substituiertes $C_1$—$C_4$-Alkyl, gegebenenfalls durch $C_1$—$C_4$-Alkyl, $C_1$—$C_4$-Alkoxy, $C_1$—$C_4$-Alkoxycarbonyl, Cyano oder Chlor substituiertes Phenyl, Styryl, Biphenylyl oder Naphthyl,

$R'_8$ unsubstituiertes $C_1$—$C_4$-Alkyl oder Phenyl,

$R'_9$ Wasserstoff, unsubstituiertes $C_1$—$C_4$-Alkyl, unsubstituiertes oder durch Chlor oder Methyl substituiertes Phenyl, Chlor, $C_1$—$C_4$-Alkoxy, $C_3$—$C_5$-Alkoxyalkoxy, unsubstituiertes oder durch Chlor oder Methyl substituiertes Phenoxy, $C_1$—$C_4$-Alkylthio oder Phenylthio,

$R'_{10}$ Wasserstoff, unsubstituiertes $C_1$—$C_4$-Alkyl, $C_1$—$C_4$-Alkoxy, unsubstituiertes oder durch Chlor oder Methyl substituiertes Phenyl, unsubstituiertes oder durch Chlor oder Methyl substituiertes Phenoxy, $C_1$—$C_4$-Alkylthio, Phenylthio oder Chlor,

$R'_{13}$ und $R'_{14}$ Wasserstoff, $C_1$—$C_4$-Alkylamino, Di-$C_1$—$C_4$-alkylamino, Phenyl, Morpholino, Piperidino,

Phenylamino oder einen Rest $-(OCH_2-CH_2)_q-OY$ worin Y für Wasserstoff, $C_1-C_4$-Alkyl, Benzyl oder Phenyl und q für eine ganze Zahl von 0 bis 7 stehen,
$R_{15}$ unsubstituiertes oder durch Chlor oder $C_1-C_4$-Alkyl substituiertes Phenyl,
$R_{16}$ Wasserstoff oder $C_1-C_4$-Alkyl und
$R'_{17}$ $C_1-C_4$-Alkyl bedeuten.

4. 4-Heterocyclyl-4'-vinyl-stilbene gemäß Anspruch der Formel

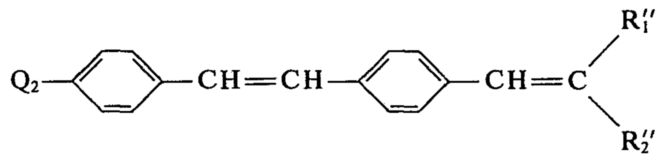

worin $R''_1$ Cyano, COOR worin R für $C_1-C_4$-Alkyl, $C_1-C_4$-Alkoxy-$C_2-C_4$-alkyl, $C_1-C_4$-Alkoxy-$C_2-C_4$-alkoxy-$C_2-C_4$-alkyl, $C_1-C_4$-Alkoxy-$C_2-C_4$-alkoxy-$C_2-C_4$-alkoxy-$C_2-C_4$-alkyl, 2,3,4,5-Tetrahydro-2-furyl-methyl, 1,4-Dioxa-2-cyclohexyl-methyl steht, CON(R')(R''), worin R' für Wasserstoff, $C_1-C_6$-Alkyl, durch Hydroxy, $C_1-C_4$-Alkoxy, $C_2-C_6$-Hydroxyalkoxy, $-SO_3M$ oder Mono- oder Di-$C_1-C_4$-alkylamino substituiertes $C_2-C_6$-Alkyl, Cycloalkyl, Benzyl oder Phenäthyl und R'' für Wasserstoff, $C_1-C_6$-Alkyl, durch Hydroxy, $C_1-C_4$-Alkoxy oder $C_2-C_6$-Hydroxyalkoxy substituiertes $C_2-C_6$-Alkyl stehen, $R_x-SO_2-$ worin $R_x$ für $C_1-C_6$-Alkyl, durch $C_1-C_4$-Alkoxy oder $C_1-C_4$-Alkoxy-$C_2-C_6$-alkoxy substituiertes $C_2-C_6$-Alkyl, Phenyl oder Benzyl steht oder Phosphonsäuredialkylester,
$R''_2$ Wasserstoff oder $C_1-C_4$-Alkyl,
M Wasserstoff oder ein bei optischen Aufhellern übliches, nicht-chromophores Kation bedeuten und
$Q_2$ die oben angegebene Bedeutung hat.

5. 4-Heterocyclyl-4'-vinyl-stilbene gemäß Anspruch 4 der Formel

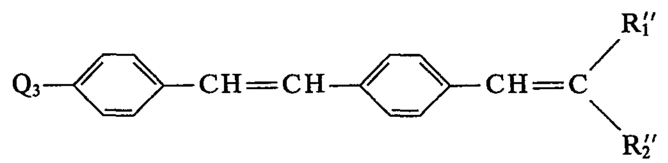

worin
$R''_1$ und $R''_2$ die in Anspruch 4 gegebene Bedeutung haben und

$Q_3$ einen Rest der Formel

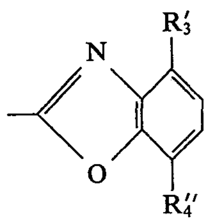

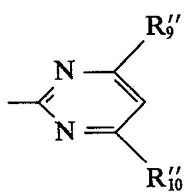

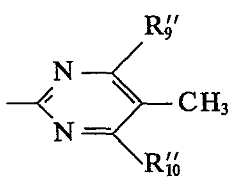

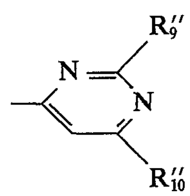

oder 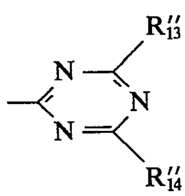

bedeuten, worin

$R'_3$ Wasserstoff, Methyl, Chlor oder $C_1-C_4$-Alkoxy,
$R''_4$ Wasserstoff, Methyl, Chlor oder $C_1-C_4$-Alkyl,
$R''_9$ Wasserstoff, Methyl, Phenyl, $C_1-C_3$-Alkoxy, Methoxyäthoxy oder Phenoxy,
$R''_{10}$ unsubstituiertes $C_1$- oder $C_2$-Alkyl, $C_1-C_3$-Alkoxy oder Phenoxy und
$R''_{13}$ und $R''_{14}$ je Wasserstoff oder einen Rest $-(OCH_2-CH_2)_r-OY'$ worin
Y' für $C_1-C_4$-Alkyl und r für eine ganze Zahl von 0 bis 2 stehen,

bedeuten.

6. 4-Heterocyclyl-4'-vinyl-stilbene gemäß Anspruch 5 der Formel

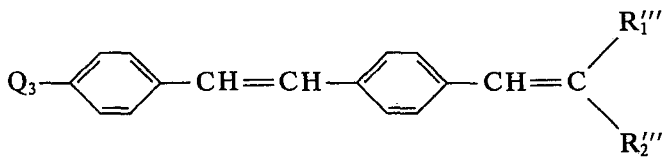

worin $R_1'''$ Cyano, COOR worin R für $C_1$—$C_4$-Alkyl, $C_1$—$C_4$-Alkoxy-$C_2$—$C_4$-alkyl, $C_1$—$C_4$-Alkoxy-$C_2$—$C_4$-alkoxy-$C_2$—$C_4$-alkyl, $C_1$—$C_4$-Alkoxy-$C_2$—$C_4$-alkoxy-$C_2$—$C_4$-alkoxy-$C_2$—$C_4$-alkyl, 2,3,4,5-Tetrahydro-2-furyl-methyl oder 1,4-Dioxa-2-cyclohexyl-methyl steht, oder $R_x$—$SO_2$—, worin $R_x$ für $C_1$—$C_4$-Alkyl, durch $C_1$—$C_4$-Alkoxy oder $C_1$—$C_4$-Alkoxy-$C_2$—$C_6$-Alkoxy substituiertes $C_2$—$C_6$-Alkyl, Phenyl oder Benzyl steht, und $R_2'''$ Wasserstoff oder $C_1$—$C_4$-Alkyl bedeutet, und $Q_3$ die in Anspruch 5 angegebene Bedeutung hat.

7. 4-Heterocyclyl-4'-vinyl-stilbene gemäß Anspruch 5 der Formel

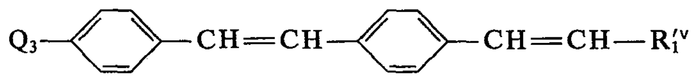

worin $R_1^{IV}$ Cyano oder COOR' bedeutet, worin R' $C_1$—$C_4$-Alkyl oder $C_1$—$C_4$-Alkoxy-$C_2$—$C_6$-alkyl ist und $Q_3$ die in Anspruch 5 angegebene Bedeutung hat.

8. Das 4-Heterocyclyl-4'-vinyl-stilben gemäß Anspruch 4 der Formel

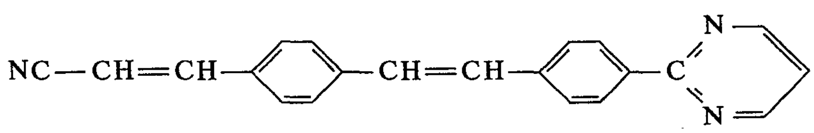

9. Das 4-Heterocyclyl-4'-vinyl-stilben gemäß Anspruch 6 der Formel

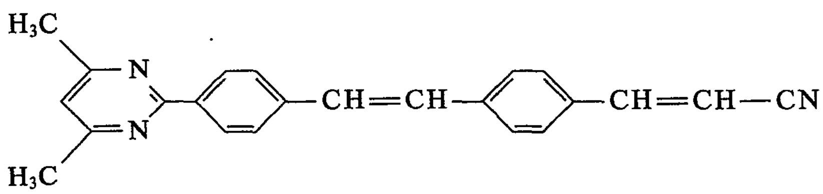

10. Das Heterocyclyl-4'-vinyl-stilben gemäß Anspruch 6 der Formel

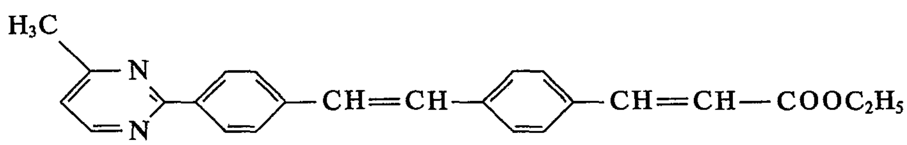

11. Das 4-Heterocyclyl-4'-vinyl-stilben gemäß Anspruch 6 der Formel

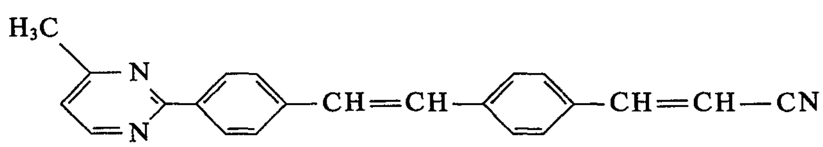

12. Das 4-Heterocyclyl-4'-vinyl-stilben gemäß Anspruch 6 der Formel

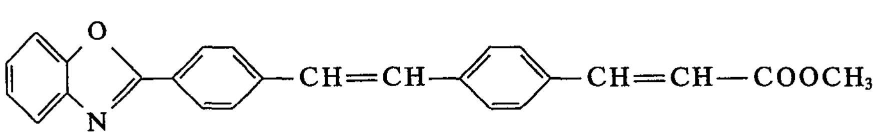

13. Verfahren zur Herstellung von 4-Heterocyclyl-4'-vinyl-stilbene der Formel

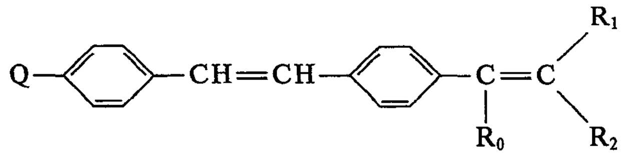

worin

Q einen monocyclischen 5- oder 6gliedrigen unsubstituierten oder nicht-chromophor substituierten, 0, 1 oder 2 ankondensierte Benzolringe aufweisenden aromatischen heterocyclischen Ring oder einen bicyclischen 9gliedrigen aromatischen heterocyclischen Ring,
$R_0$ Wasserstoff, unsubstituiertes oder nicht-chromophor substituiertes Alkyl,
$R_1$ Wasserstoff, unsubstituiertes oder nicht-chromophor substituiertes Alkyl, Alkoxycarbonyl, Carbamoyl, oder Sulfonamide; Alkenyl, Carboxy, Alkyl-, Aryl- oder Aralkylsulfonyl, Cyano, Sulfo oder Phosphonsäuredialkylester und
$R_2$ Wasserstoff, unsubstituiertes oder nicht-chromophor substituiertes Alkyl oder Alkenyl bedeuten, wobei höchstens eines der Symbole $R_1$ und
$R_2$ Wasserstoff bedeutet,

dadurch gekennzeichnet, daß man in Gegenwart basischer Kondensationsmittel eine Verbindung der Formel

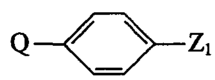

mit einer Verbindung der Formel

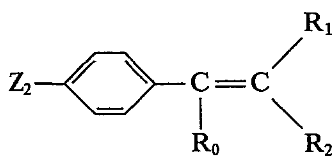

kondensiert, worin Q, $R_0$, $R_1$ und $R_2$ die oben angegebene Bedeutung haben und von $Z_1$ und $Z_2$ das eine die OHC-Gruppe und das andere eine Gruppierung der Formel

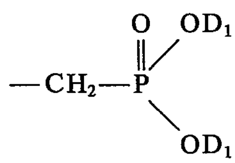 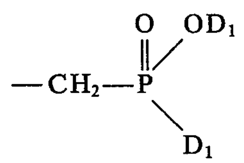 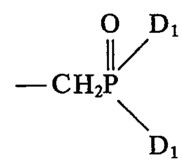 oder 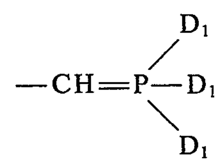

bedeuten, worin $D_1$ einen unsubstituierten oder substituierten Alkyl-, Aryl-, Cycloalkyl- oder Aralkylrest darstellt.

14. Verfahren zum optischen Aufhellen von hochmolekularen synthetischen, halbsynthetischen und natürlichen organischen Materialien, dadurch gekennzeichnet, daß man 4-Heterocyclyl-4'-vinyl-stilbene wie in einem der Ansprüche 1 bis 7 definiert, diesen Materialien in einer Menge von 0,001 bis 2%, bezogen auf das aufzuhellende Material, einverleibt oder auf deren Oberfläche aufbringt.

15. Verfahren gemäß Anspruch 14 zum optischen Aufhellen von hochmolekularen organischen Materialien.

16. Verfahren gemäß Anspruch 14 zum optischen Aufhellen von Polyestern.

17. Aufhellermischung enthaltend ein 4-Heterocyclyl-4'-vinyl-stilben gemäß den Ansprüchen 1 bis 7 und einen für Polyester geeigneten Aufheller im Verhältnis 1:9 bis 9:1.

18. Aufhellermischung gemäß Anspruch 17 enthaltend ein 4-Heterocyclyl-4'-vinyl-stilben der Formel

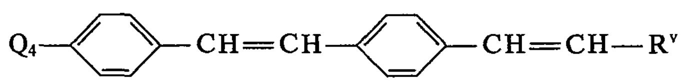

worin $Q_4$ den Benzoxazol-2-yl-, Pyrimidin-2-yl-, 4-Methyl-pyrimidin-2-yl- oder 4,6-Dimethylpyrimidin-2-ylrest und $R^v$ $C_2$—$C_4$-Alkoxycarbonyl oder Cyano bedeuten und als für Polyester geeigneten Aufheller das 1,4-Bis-(benzoxazol-2-yl)-naphthalin, das 4,4'-Bis-(äthoxycarbonyl-vinyl)-stilben, das 4,4'-Bis-(cyan-vinyl)-stilben, das 1,4-Bis-(2'-cyanostyryl)-benzol, das 1,5-Bis-(benzoxazol-2'-yl)-thiophen,

das 1-Phenyl-4-(5',7'-dimethylbenzoxazol-2'-yl)-stilben, das 1,2-Bis-(5'-methyl-benzoxazol-2'-yl)-vinylen, das 4-(Benzoxazol-2'-yl)-4'-(3"-methyl-1",2",4"-oxdiazol-5"-yl)-stilben oder das 2,4-Dimethoxytriazin-2-yl-pyren im Verhältnis 1 : 2 oder 2 : 1.

19. Aufhellermischung gemäß Anspruch 18 enthaltend den Aufheller der Formel

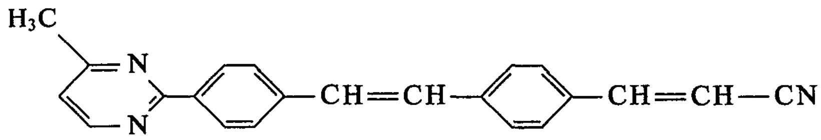

und den Aufheller der Formel

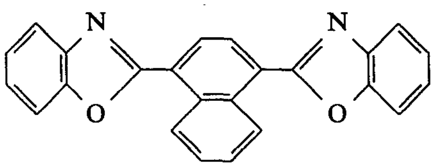

im Verhältnis 1 : 2 oder 2 : 1.

20. Aufhellermischung gemäß Anspruch 17 enthaltend den Aufheller der Formel

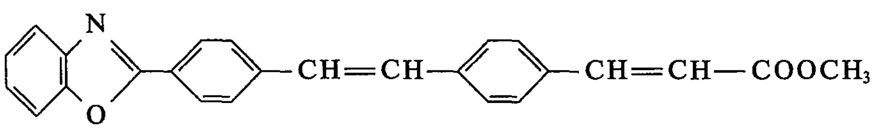

und den Aufheller der Formel

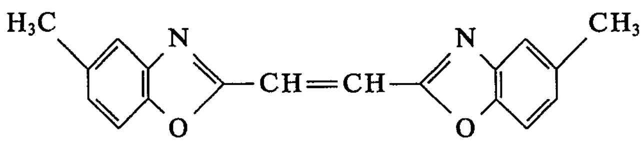

im Verhältnis 1 : 2 oder 2 : 1.

**Claims**

1. A 4-heterocyclyl-4'-vinylstilbene of the formula

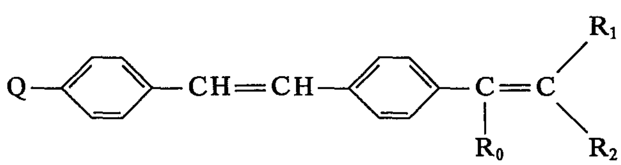

wherein Q is a monocyclic 5- or 6-membered aromatic heterocyclic ring which is unsubstituted or substituted by non-chromophoric groups and which contains no fused benzene rings or 1 or 2 fused benzene rings, or is a bicyclic 9-membered aromatic heterocyclic ring, $R_0$ is hydrogen, alkyl which is unsubstituted or substituted by non-chromophoric groups, $R_1$ is hydrogen, alkyl, alkoxycarbonyl, carbamoyl or sulfonamido, each of which is unsubstituted or substituted by non-chromophoric groups, or is alkenyl, carboxyl, alkylsulfonyl, arylsulfonyl, aralkylsulfonyl, cyano, sulfo or phosphonic acid dialkyl ester, and $R_2$ is hydrogen, alkyl or alkenyl,each of which is unsubstituted or substituted by non-chromophoric groups, with the proviso that at most one of $R_1$ and $R_2$ is hydrogen.

2. A 4-heterocyclyl-4'-vinylstilbene according to claim 1 of the formula

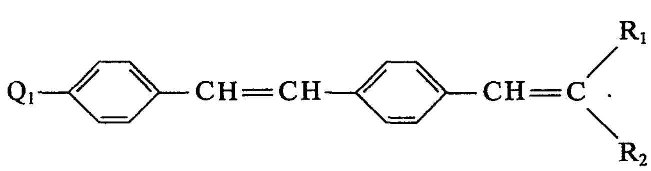

wherein $R_1$ and $R_2$ are as defined in claim 1, $Q_1$ is a radical of the formula

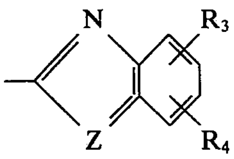 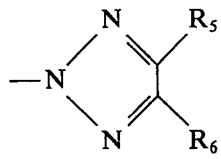 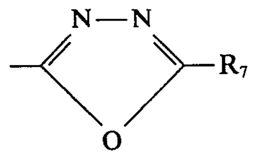 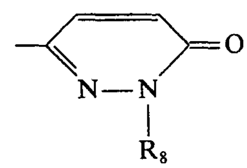

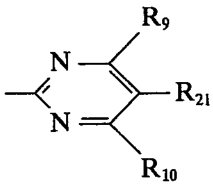 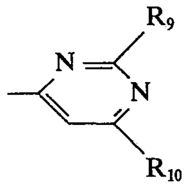 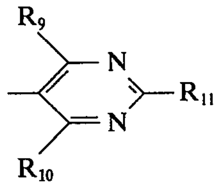 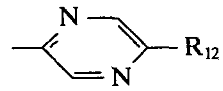

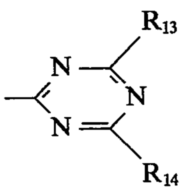 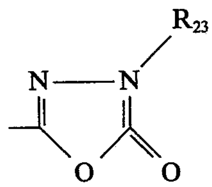 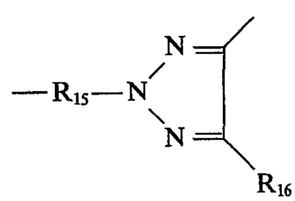 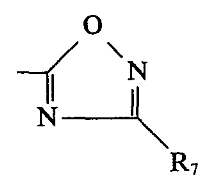

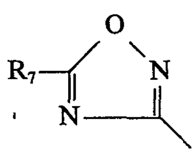 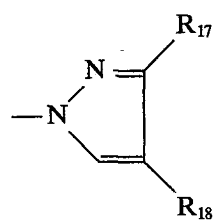 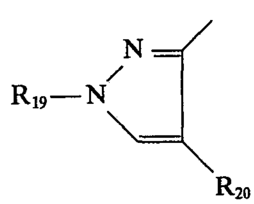 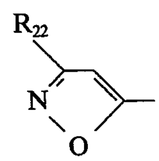

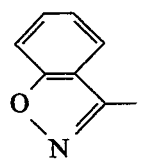 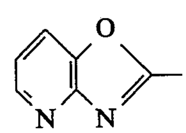 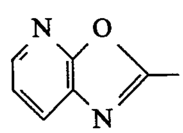 or 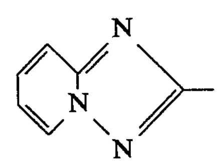

in which radicals

$R_3$ is hydrogen, chlorine, $C_1$—$C_4$alkyl, phenyl($C_1$—$C_3$)alkyl, cyclohexyl, phenyl, $C_1$—$C_4$alkoxy, $C_1$—$C_4$alkylsulfonyl, $C_1$—$C_4$alkoxycarbonyl, cyano or carboxy, or together with $R_4$ is a fused 1-cyclopentene, 1-cyclohexene or benzene ring, each of which is unsubstituted or substituted by 1 to 4 methyl groups,

$R_4$ is hydrogen, chlorine or $C_1$—$C_4$alkyl or, together with $R_3$, forms a 1-cyclopentene, 1-cyclohexene or benzene ring, each of which is unsubstituted or substituted by 1 to 4 methyl groups,

$R_5$ is hydrogen, $C_1$—$C_4$alkyl, cyano, COOR, wherein R is $C_1$—$C_4$alkyl, phenyl or styryl or, together with $R_6$, forms a fused benzene ring which is unsubstituted or substituted by $C_1$—$C_4$alkyl, $C_1$—$C_4$alkoxy or chlorine, or a fused naphthalene ring,

$R_6$ is hydrogen, $C_1$—$C_4$alkyl, phenyl or, together with $R_5$, forms a fused benzene ring which is unsubstituted or substituted by $C_1$—$C_4$alkyl, $C_1$—$C_4$alkoxy or chlorine, or a fused naphthalene ring,

$R_7$ is $C_1$—$C_4$alkyl which is unsubstituted or substituted by non-chromophoric groups, phenyl, styryl, biphenylyl or naphthyl, each of which is unsubstituted or substituted by $C_1$—$C_4$alkyl, $C_1$—$C_4$alkoxy, $C_1$—$C_4$alkoxycarbonyl, cyano or chlorine,

$R_8$ is hydrogen, $C_1$—$C_4$alkyl or phenyl which is unsubstituted or substituted by chlorine or methyl,

$R_9$ and $R_{10}$, each independently of the other, are hydrogen, $C_1$—$C_4$alkyl, phenyl which is unsubstituted or substituted by chlorine or methyl, or are $C_1$—$C_4$alkoxy, $C_3$—$C_8$alkoxyalkoxy, phenoxy which is unsubstituted or substituted by chlorine or methyl, or are chlorine, $C_1$—$C_4$alkylthio, phenylthio, $C_1$—$C_4$alkylamino, di($C_1$—$C_4$)alkylamino, morpholino, piperidino, piperazino, pyrrolidino or anilino,

$R_{11}$ is hydrogen, $C_1$—$C_4$alkyl or phenyl which is unsubstituted or substituted by chlorine or methyl,

$R_{12}$ is $C_1$—$C_4$alkoxy, $C_3$—$C_8$alkoxyalkoxy, $C_1$—$C_4$alkylthio, phenoxy which is unsubstituted or substituted by chlorine or methyl, or is cycloalkoxy, $C_1$—$C_4$alkylthio, phenylthio which is unsubstituted or substituted by chlorine or methyl, or is $C_1$—$C_4$alkylamino, di($C_1$—$C_4$)alkylamino, morpholino, piperidino, piperazino, pyrrolidino or anilino,

$R_{13}$ and $R_{14}$ are hydrogen, halogen, $C_1$—$C_4$alkoxy, phenyl, aralkoxy, cycloalkoxy, aryloxy, $C_1$—$C_4$alkyl-

mercapto, $C_1$—$C_4$alkylamino, di($C_1$—$C_4$)alkylamino, morpholino, piperidino, piperazino, pyrrolidino, arylamino or $C_1$—$C_4$alkyl,

$R_{15}$ is phenyl which is unsubstituted or substituted by chlorine or $C_1$—$C_4$alkyl,
$R_{16}$ is hydrogen or $C_1$—$C_4$alkyl,
$R_{17}$ is phenyl which is unsubstituted or substituted by chlorine or $C_1$—$C_4$alkyl,
$R_{18}$ is hydrogen or $C_1$—$C_4$alkyl,
$R_{19}$ is phenyl which is unsubstituted or substituted by chlorine or $C_1$—$C_4$alkyl,
$R_{20}$ is hydrogen or $C_1$—$C_4$alkyl,
$R_{21}$ is hydrogen or $C_1$—$C_4$alkyl,
$R_{22}$ is phenyl which is unsubstituted or substituted by $C_1$—$C_4$alkoxy,
$R_{23}$ is hydrogen, $C_1$—$C_4$alkyl or phenyl which is unsubstituted or substituted by $C_1$—$C_4$alkyl, halogen or cyano, and
Z is O, S or NX, wherein X is hydrogen, $C_1$—$C_4$alkyl, acetyl, benzoyl or phenyl.

3. A 4-heterocyclyl-4'-vinylstilbene according to claim 2 of the formula

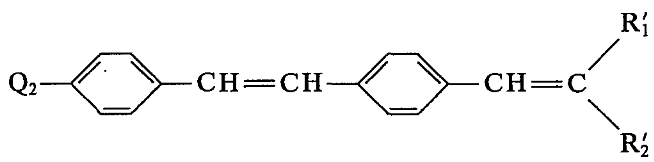

wherein $R_1'$ is hydrogen, $C_1$—$C_6$alkyl, $C_1$—$C_6$alkyl which is substituted by $C_1$—$C_4$alkoxy or $C_2$—$C_5$alkoxycarbonyl, or is $C_2$—$C_4$alkenyl, cyano, $COOR^0$, wherein $R^0$ is $C_1$—$C_4$alkyl, $C_1$—$C_4$alkoxy-$C_2$—$C_4$alkyl, $C_1$—$C_4$alkoxy-$C_2$—$C_4$alkoxy-$C_2$—$C_4$alkyl, $C_1$—$C_4$alkoxy-$C_2$—$C_4$alkoxy-$C_2$—$C_4$alkoxy-$C_2$—$C_4$alkyl, tetrahydro-2-furylmethyl, 1,4-dioxa-2-cyclohexylmethyl, allyl, $C_1$—$C_4$alkylamino-$C_1$—$C_4$alkyl or di($C_1$—$C_4$)-alkylamino-$C_1$—$C_4$alkyl, —CON(R')(R''), wherein R' is hydrogen, $C_1$—$C_6$alkyl, $C_2$—$C_6$alkyl which is substituted by hydroxy, $C_1$—$C_4$alkoxy, $C_2$—$C_6$hydroxyalkoxy, $-SO_3M$ or mono- or di($C_1$—$C_4$)alkylamino, or is cyclohexyl, benzyl or phenethyl, and R'' is hydrogen, $C_1$—$C_6$alkyl, $C_2$—$C_6$alkyl which is substituted by hydroxy, $C_1$—$C_4$alkoxy or $C_2$—$C_6$hydroxyalkoxy, $-SO_2N(R')(R'')$, wherein R' and R'' are as defined above, $-SO_3M$, $R_x-SO_2-$, wherein $R_x$ is $C_1$—$C_6$alkyl, $C_2$—$C_6$alkyl which is substituted by $C_1$—$C_4$alkoxy or $C_1$—$C_4$alkoxy-$C_2$—$C_6$alkoxy, or is phenyl or benzyl or phosphonic acid dialkyl ester;
$R_2'$ is hydrogen or $C_1$—$C_6$alkyl,
M is hydrogen or a non-chromophoric cation common to fluorescent whitening agents, and
$Q_2$ is a radical of the formula

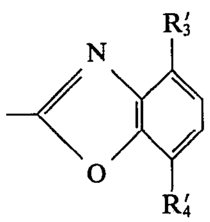 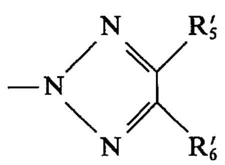 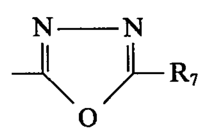 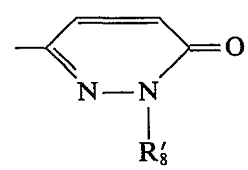

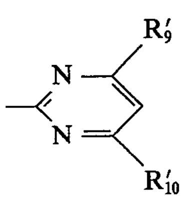 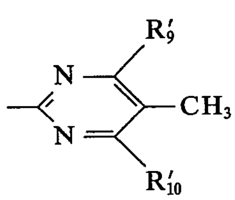 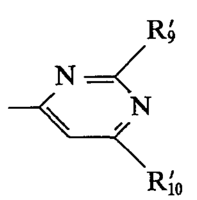 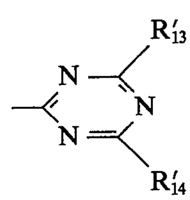

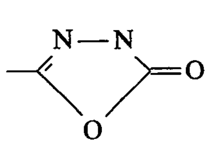 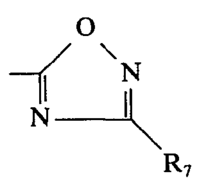 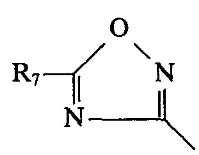 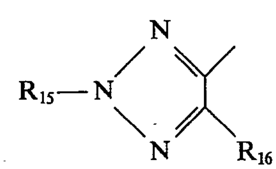

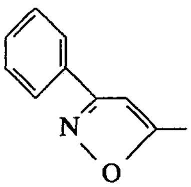 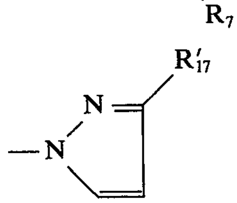 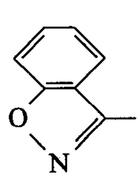 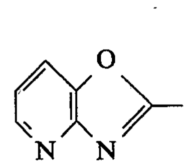 or

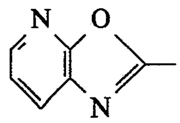

wherein

$R_3'$ is hydrogen, methyl, chlorine or $C_1$—$C_4$alkoxy,
$R_4'$ is hydrogen, chlorine, $C_1$—$C_4$alkyl, phenyl, $C_1$—$C_4$alkoxy or phenoxy,
$R_5'$ is hydrogen, $C_1$—$C_4$alkyl, phenyl or styryl or, together with $R_6$, forms a fused unsubstituted benzene ring or a fused benzene ring which is substituted by $C_1$—$C_4$alkyl, $C_1$—$C_4$alkoxy or chlorine, or a fused naphthalene ring,
$R_6'$ is hydrogen, $C_1$—$C_4$alkyl, phenyl or, together with $R_5'$, forms a fused unsubstituted benzene ring or a fused benzene ring which is substituted by $C_1$—$C_4$alkyl, $C_1$—$C_4$alkoxy or chlorine, or a fused naphthalene ring,
$R_7$ is $C_1$—$C_4$alkyl which is unsubstituted or substituted by non-chromophoric substituents, phenyl, styryl, biphenylyl or naphthyl, each of which is unsubstituted or substituted by $C_1$—$C_4$alkyl, $C_1$—$C_4$alkoxy, $C_1$—$C_4$alkoxycarbonyl, cyano or chlorine,
$R_8'$ is unsubstituted $C_1$—$C_4$alkyl or phenyl,
$R_9'$ is hydrogen, unsubstituted $C_1$—$C_4$alkyl, phenyl which is unsubstituted or substituted by chlorine or methyl, or is chlorine, $C_1$—$C_4$alkoxy, $C_3$—$C_5$alkoxyalkoxy, phenoxy which is unsubstituted or substituted by chlorine or methyl, or is $C_1$—$C_4$alkylthio or phenylthio,
$R_{10}'$ is hydrogen, unsubstituted $C_1$—$C_4$alkyl, $C_1$—$C_4$alkoxy, phenyl which is unsubstituted or substituted by chlorine or methyl, phenoxy which is unsubstituted or substituted by chlorine or methyl, or is $C_1$—$C_4$alkylthio, phenylthio or chlorine,
$R_{13}'$ and $R_{14}'$ are hydrogen, $C_1$—$C_4$alkylamino, di($C_1$—$C_4$)alkylamino, phenyl, morpholino, piperidino, phenylamino or a radical $-(OCH_2)_q-OY$, wherein Y is hydrogen, $C_1$—$C_4$alkyl, benzyl or phenyl, and q is an integer from 0 to 7,
$R_{15}$ is phenyl which is unsubstituted or substituted by chlorine or $C_1$—$C_4$alkyl,
$R_{16}$ is hydrogen or $C_1$—$C_4$alkyl, and
$R_{17}'$ is $C_1$—$C_4$alkyl.

4. A 4-heterocyclyl-4'-vinylstilbene according to claim 3 of the formula

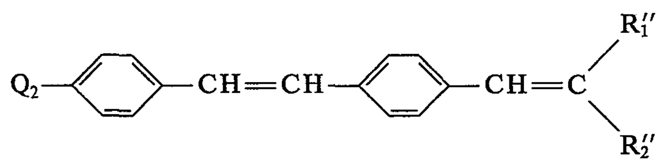 (5)

wherein $R_1''$ is cyano, COOR, wherein R is $C_1$—$C_4$alkyl, $C_1$—$C_4$alkoxy-$C_2$—$C_4$alkyl, $C_1$—$C_4$alkoxy-$C_2$—$C_4$alkoxy-$C_2$—$C_4$alkyl, $C_1$—$C_4$alkoxy-$C_2$—$C_4$alkoxy-$C_2$—$C_4$alkoxy-$C_2$—$C_4$alkyl, 2,3,4,5-tetrahydro-2-furylmethyl, 1,4-dioxa-2-cyclohexylmethyl, CON(R')(R''), wherein R' is hydrogen, $C_1$—$C_6$alkyl, $C_2$—$C_4$alkyl which is substituted by hydroxy, $C_1$—$C_4$alkoxy, $C_2$—$C_6$hydroxyalkoxy, $-SO_3M$ or mono- or di($C_1$—$C_4$)alkylamino, or is cycloalkyl, benzyl or phenethyl, and R'' is hydrogen, $C_1$—$C_6$alkyl, $C_2$—$C_6$alkyl which is substituted by hydroxy, $C_1$—$C_4$alkoxy or $C_2$—$C_6$hydroxyalkoxy, $R_x-SO_2-$, wherein $R_x$ is $C_1$—$C_6$alkyl, $C_2$—$C_6$alkyl which is substituted by $C_1$—$C_4$alkoxy or $C_1$—$C_4$alkoxy-$C_2$—$C_6$alkoxy, or is phenyl or benzyl or phosphonic acid dialkyl ester; $R_2''$ is hydrogen or $C_1$—$C_4$alkyl, M is hydrogen or a non-chromophoric cation common to fluorescent whitening agents, and $Q_2$ is as defined in claim 3.

5. A 4-heterocyclyl-4'-vinylstilbene according to claim 4 of the formula

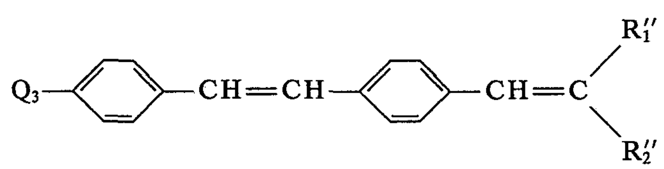

wherein $R_1''$ and $R_2''$ are as defined in claim 4 and $Q_3$ is a radical of the formula

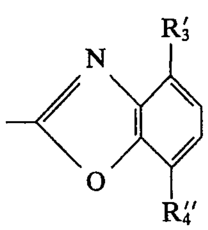 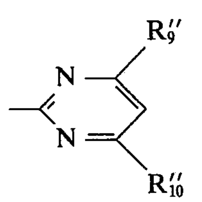 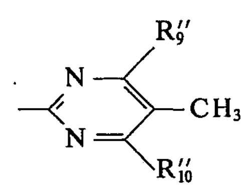 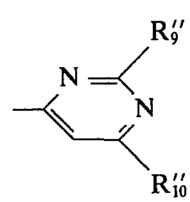

or 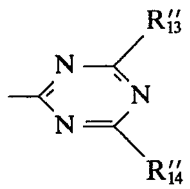

wherein $R_3'$ is hydrogen, methyl, chlorine or $C_1-C_4$alkoxy, $R_4''$ is hydrogen, methyl, chlorine or $C_1-C_4$alkyl, $R_9''$ is hydrogen, methyl, phenyl, $C_1-C_3$alkoxy, methoxyethoxy or phenoxy, $R_{10}''$ is unsubstituted alkyl of 1 or 2 carbon atoms, $C_1-C_3$alkoxy or phenoxy, and each of $R_{13}''$ and $R_{14}''$ is hydrogen or a radical $-(OCH_2-CH_2)_r-OY'$, wherein Y' is $C_1-C_4$alkyl and r is an integer from 0 to 2.

6. A 4-heterocyclyl-4'-vinylstilbene to claim 5 of the formula

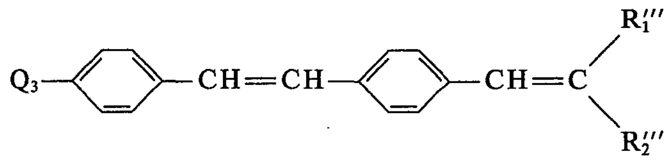

wherein $R_1'''$ is cyano, COOR, wherein R is $C_1-C_4$alkyl, $C_1-C_4$alkoxy-$C_2-C_4$alkyl, $C_1-C_4$alkoxy-$C_2-C_4$alkoxy-$C_2-C_4$alkyl, $C_1-C_4$alkoxy-$C_2-C_4$alkoxy-$C_2-C_4$alkoxy-$C_2-C_4$alkyl, 2,3,4,5-tetrahydro-2-furylmethyl or 1,4-dioxa-2-cyclohexylmethyl or $R_x-SO_2-$, wherein $R_x$ is $C_1-C_4$alkyl, $C_2-C_4$alkyl which is substituted by $C_1-C_4$alkoxy or $C_1-C_4$alkoxy-$C_2-C_6$alkoxy, or is phenyl or benzyl, and $R_2'''$ is hydrogen or $C_1-C_4$alkyl, and $Q_3$ is as defined in claim 5.

7. A 4-heterocyclyl-4'-vinylstilbene according to claim 5 of the formula

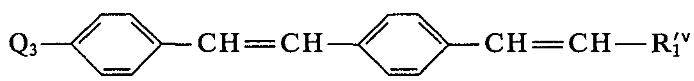

wherein $R_1^{IV}$ is cyano or COOR', wherein R' is $C_1-C_4$alkyl or $C_1-C_4$alkoxy-$C_2-C_6$alkyl, and $Q_3$ is as defined in claim 5.

8. The 4-heterocyclyl-4'-vinylstilbene according to claim 4 of the formula

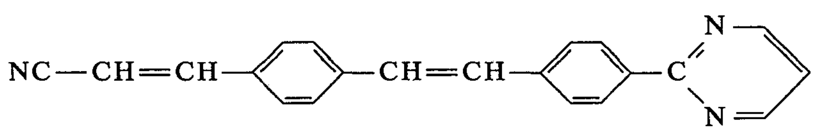

9. The 4-heterocyclyl-4'-vinylstilbene according to claim 6 of the formula

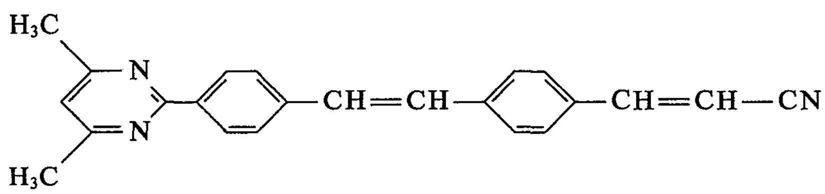

10. The 4-heterocyclyl-4'-vinylstilbene according to claim 6 of the formula

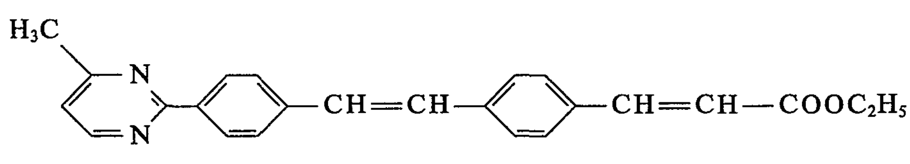

11. The 4-heterocyclyl-4'-vinylstilbene according to claim 6 of the formula

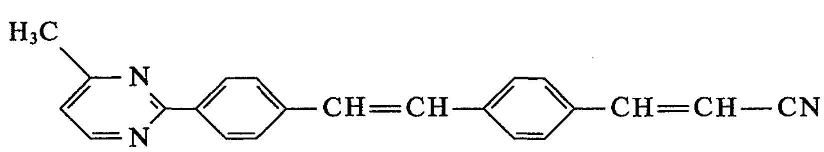

12. The 4-heterocyclyl-4'-vinylstilbene according to claim of the formula

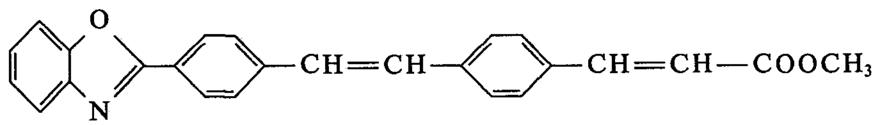

13. A process for the production of 4-heterocyclyl-4'-vinylstilbene of the formula

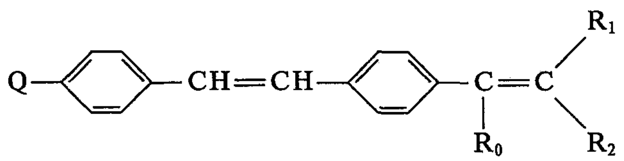

wherein Q is a monocyclic 5- or 6-membered aromatic heterocyclic ring which is unsubstituted or substituted by non-chromophoric groups and which contains no fused benzene rings or 1 or 2 fused benzene rings, or is a bicyclic 9-membered aromatic heterocyclic ring, $R_0$ is hydrogen, alkyl which is unsubstituted or substituted by non-chromophoric groups, $R_1$ is hydrogen, alkyl, alkoxycarbonyl, carbamoyl or sulfonamido, each of which is unsubstituted or substituted by non-chromophoric groups, or is alkenyl, carboxyl, alkylsulfonyl, arylsulfonyl, aralkylsulfonyl, cyano, sulfo or phosphonic acid dialkyl ester, and $R_2$ is hydrogen, alkyl or alkenyl, each of which is unsubstituted or substituted by non-chromophoric groups, with the proviso that at most one of $R_1$ and $R_2$ is hydrogen, which process comprises condensing, in the presence of a basic condensing agent, a compound of the formula

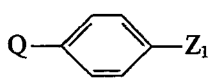

with a compound of the formula

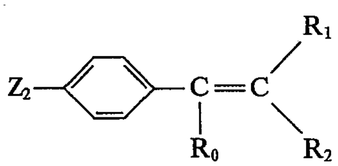

wherein Q, $R_0$, $R_1$ and $R_2$ are as defined above and one of $Z_1$ and $Z_2$ is the OHC group and the other is a grouping of the formula

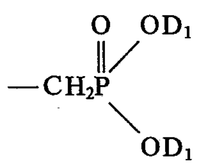 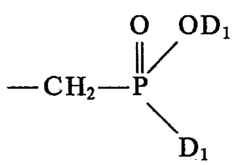 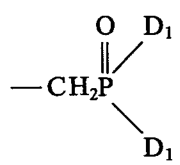 or 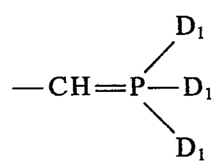

wherein $D_1$ is an unsubstituted or a substituted alkyl, aryl, cycloalkyl or aralkyl radical.

14. A process for whitening man-made, regenerated man-made and natural organic material of high molecular weight, which comprises incorporating in or applying to the surface of said material a 4-heterocyclyl-4'-vinylstilbene as defined in any one of claims 1 to 7, in an amount of 0.001 to 2%, based on the weight of said material.

15. A process according to claim 14, wherein the material to be whitened is organic material of high molecular weight.

16. A process according to claim 14, wherein the material to be whitened is polyester.

17. A mixture containing a 4-heterocyclyl-4'-vinylstilbene according to any one of claims 1 to 7, and a fluorescent whitening agent suitable for polyester, in the ratio of 1 : 9 to 9 : 1.

18. A mixture according to claim 17 containing a 4-heterocyclyl-4'-vinylstilbene of the formula

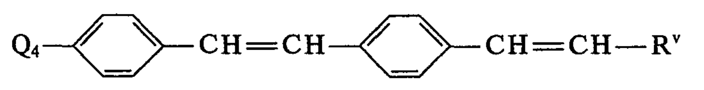

wherein $Q_4$ is a benzoxazol-2-yl radical, a pyrimidin-2-yl radical, a 4-methylpyrimidin-2-yl radical or a 4,6-dimethylpyrimidine-2-yl radical, and $R^V$ is $C_2$—$C_4$alkoxycarbonyl or cyano, and, as fluorescent whitening agent suitable for polyester, 1,4-bis(benzoxazol-2'-yl)naphthalene, 4,4'-bis(ethoxycarbonylvinyl)stilbene, 4,4'-bis(cyanovinyl)stilbene, 1,4-bis(2'-cyanostyryl)benzene, 1,5-bis(benzoxazole-2'-yl)thiophene, 1-phenyl-4(5',7'-dimethylbenzoxazol-2'-yl)stilbene, 1,2-bis(5'-methylbenzoxazol-2'-yl)vinylene, 4-(benzoxazol-2'-yl)-4'-(3"-methyl-1",2",4"-oxadiazol-5"-yl)stilbene or 2,4-dimethoxy-triazin-2-yl-pyrene in the ratio 1 : 2 or 2 : 1.

19. A mixture according to claim 18 containing the fluorescent whitening of the formula

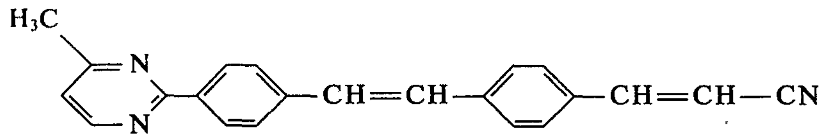

and the fluorescent whitening of the formula

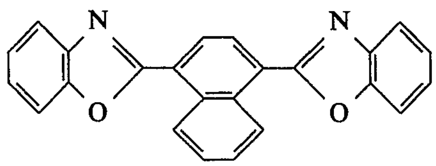

in the ratio 1 : 2 or 2 : 1.

20. A mixture according to claim 17 containing the fluorescent whitening agent of the formula

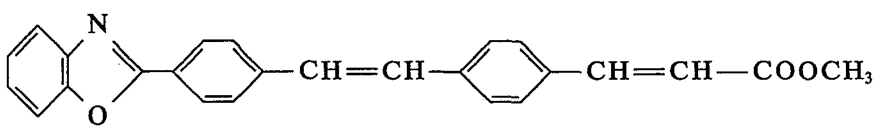

and the fluorescent whitening agent of the formula

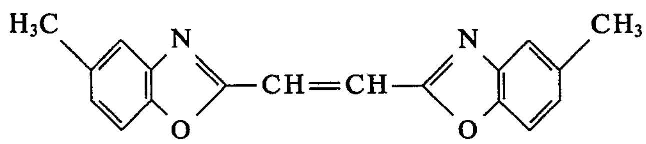

in the ratio 1 : 2 or 2 : 1.

**Revendications**

1. 4-hétérocyclyl-4'-vinyl-stilbènes répondant à la formule

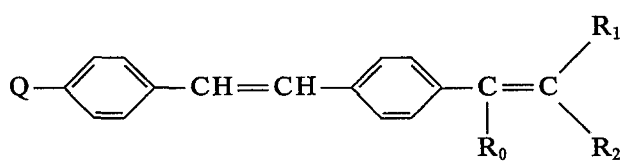

dans laquelle Q est un noyau hétérocyclique aromatique, monocyclique penta ou hexagonal, non substitué ou substitué par un groupe non-chromophore ayant 0, 1 ou 2 noyaux benzèniques fixés par condensation, ou bien un noyau hétérocyclique aromatique, bicyclique à neuf chainons, $R_0$ représente l'hydrogène, un alkyle non substitué ou substitué par un groupe non-chromophore; $R_1$ représente l'hydrogène, un alkyle non substitué ou substitué par un groupe non-chromophore, un alcoxycarbonyle, un carbamoyle ou un sulfonamido; un groupe alcényle, carboxyle, alkyl-, aryl- ou aralkyl-sulfonyle, cyano, sulfo ou ester dialkylique de l'acide phosphonique, et $R_2$ représente l'hydrogène, un alkyle non substitué ou substitué par un groupe non-chromophore ou un alcényle, étant entendu que $R_1$ et $R_2$ représentent l'hydrogène.

2. 4-hétérocyclyl-4'-vinyl-stilbènes selon la revendication 1 répondant à la formule

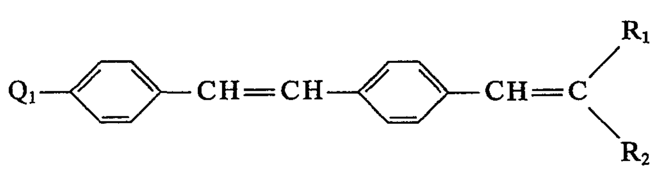

dans laquelle $R_1$ et $R_2$ sont tels que définis dans la revendication 1 et $Q_1$ représente un radical répondant à la formule suivante

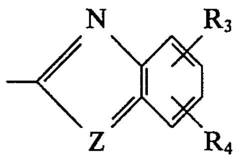 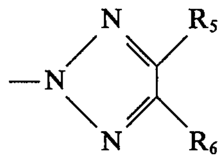 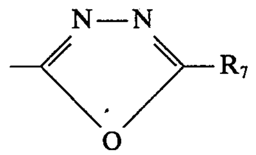 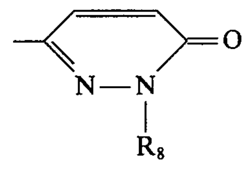

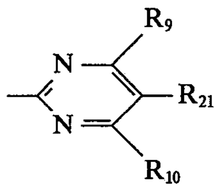 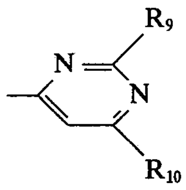 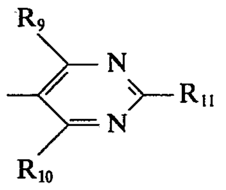 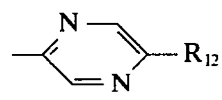

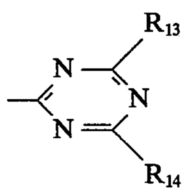 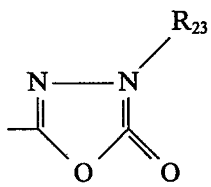 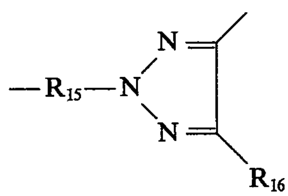 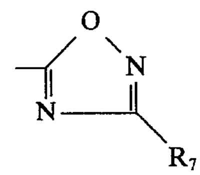

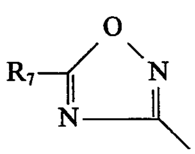 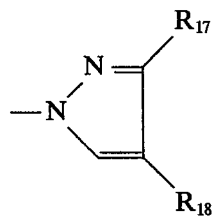 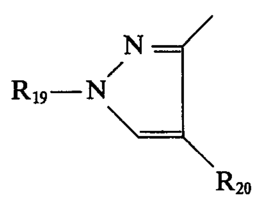 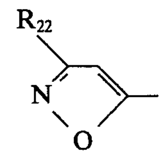

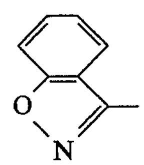 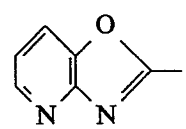 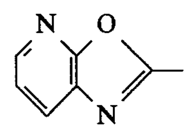 ou 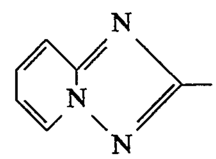

Dans ces radicaux,

$R_3$ représente l'hydrogène, le chlore, un groupe alkyle en $C_1$—$C_4$, phényl(alkyle en $C_1$—$C_3$), cyclohexyle, phényle, alcoxy en $C_1$—$C_4$, alkylsulfonyle en $C_1$—$C_4$, (alcoxy en $C_1$—$C_4$)carbonyle, cyano ou carboxyle, ou en association avec $R_4$, un noyau 1-cyclopentènique, 1-cyclohexènique ou benzènique obtenus par condensation éventuellement substitués par 1 à 4 groupes méthyle,

$R_4$ est l'hydrogène, le chlore ou un groupe alkyle en $C_1$—$C_4$ ou en association avec $R_3$, un noyau 1-cyclopentènique ou 1-cyclohexènique ou benzènique obtenus par condensation éventuellement substitués par 1 à 4 groupes méthyle,

$R_5$ est l'hydrogène, un groupe alkyle en $C_1$—$C_4$, cyano, COOR, où R représente un alkyle en $C_1$—$C_4$, phényle, ou styryle ou, en association avec $R_6$, un noyau benzènique obtenu par condensation éventuellement substitué par un groupe alkyle en $C_1$—$C_4$, alcoxy en $C_1$—$C_4$ ou du chlore, ou un noyau naphtalénique obtenu par condensation,

$R_6$ est l'hydrogène, un groupe alkyle en $C_1$—$C_4$, phényle, ou, en association avec $R_5$ un noyau benzènique obtenu par condensation substitué par un groupe alkyle en $C_1$—$C_4$, alcoxy en $C_1$—$C_4$ ou du chlore, ou un noyau naphtalènique obtenu par condensation,

$R_7$ est un alkyle en $C_1$—$C_4$ non substitué ou substitué par un groupe non-chromophore, un phényle éventuellement substitué par un groupe alkyle en $C_1$—$C_4$, alcoxy en $C_1$—$C_4$, (alcoxy en $C_1$—$C_4$)carbonyle, cyano, ou le chlore, un styryle, un biphénylyle ou un naphtalyle.

$R_8$ est l'hydrogène, un groupe alkyle en $C_1$—$C_4$ ou phényle non substitué ou substitué par du chlore ou du méthyle;

$R_9$ et $R_{10}$ sont indépendamment l'un de l'autre de l'hydrogène, un groupe alkyle en $C_1$—$C_4$, phényle non substitué ou substitué par du chlore ou du méthyle; alcoxy en $C_1$—$C_4$, alcoxyalcoxy en $C_3$—$C_8$, phénoxy non substitué ou substitué par du chlore ou du méthyle, le chlore, un groupe (alkyle en $C_1$—$C_4$)-thio, phénylthio, (alkyl en $C_1$—$C_4$)-amino, (dialkyl en $C_1$—$C_4$)-amino, morpholino, pipéridino, pipérazino, pyrrolidino, ou anilino,

$R_{11}$ est l'hydrogène, un alkyle en $C_1$—$C_4$ ou un phényle non substitué ou substitué par du chlore ou du méthyle,

$R_{12}$ est un groupe alcoxy en $C_1-C_4$, alcoxyalcoxy en $C_3-C_8$, (alkyle en $C_1-C_4$)-thio, phénoxy non substitué ou substitué par du chlore ou du méthyle, cycloalcoxy, phénylthio non substitué ou substitué par du chlore ou du méthyle, un groupe(alkyl en $C_1-C_4$)amino, (dialkyl en $C_1-C_4$)-amino, morpholino, pipéridino, pipérazino, pyrrolidino ou anilino,

$R_{13}$ et $R_{14}$ sont l'hydrogène, un halogène, un groupe alcoxy en $C_1-C_4$, phényle, aralcoxy, cycloalcoxy, aryloxy, (alkyle en $C_1-C_4$)-mercapto, (alkyle en $C_1-C_4$)-amino, (dialkyle en $C_1-C_4$)-amino, morpholino, pipéridino, piperazino, pyrrolidino, arylamino ou alkyle en $C_1-C_4$,

$R_{15}$ est un groupe phényle non substitué ou substitué par du chlore ou un alkyle en $C_1-C_4$,

$R_{16}$ est l'hydrogène ou un groupe alkyle en $C_1-C_4$

$R_{17}$ est un groupe phényle non substitué ou substitué par du chlore ou un alkyle en $C_1-C_4$,

$R_{18}$ est l'hydrogène ou un groupe alkyle en $C_1-C_4$,

$R_{19}$ est un groupe phényle non substitué ou substitué par du chlore ou un alkyle en $C_1-C_4$,

$R_{20}$ est l'hydrogène ou un groupe alkyle en $C_1-C_4$,

$R_{21}$ est l'hydrogène ou un groupe alkyle en $C_1-C_4$,

$R_{22}$ est un groupe phényle non substitué ou substitué par un alcoxy en $C_1-C_4$,

$R_{23}$ est l'hydrogène ou un groupe alkyle en $C_1-C_4$ ou un phényle non substitué ou substitué par un groupe alkyle en $C_1-C_4$, un halogène ou un groupe cyano, et

Z désigne O, S ou NX, où X représente l'hydrogène, un groupe alkyle en $C_1-C_4$, acétyle, benzoyle ou phényle.

3. 4-hétérocyclyl-4'-vinyl-stilbènes selon la revendication 2, répondant à la formule

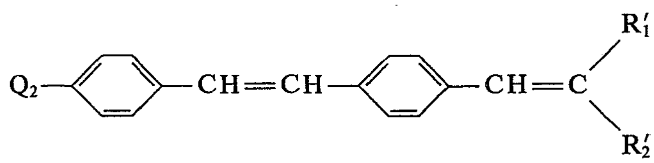

dans laquelle $R'_1$ représente l'hydrogène, un groupe alkyle en $C_1-C_6$, alkyle en $C_1-C_6$ substitué par un alcoxy en $C_1-C_4$ ou un groupe alcoxycarbonyle en $C_2-C_5$, un groupe alcényle en $C_2-C_4$, cyano, $COOR^0$ où $R^0$ représente un alkyle en $C_1-C_4$, un (alcoxy en $C_1-C_4$)-(alkyle en $C_2-C_4$), (alcoxy en $C_1-C_4$)-(alcoxy en $C_2-C_4$)-(alkyle en $C_2-C_4$), (alcoxy en $C_1-C_4$)-(alcoxy en $C_2-C_4$)-(alkoxy en $C_2-C_4$)-(alkyle en $C_2-C_4$), tétrahydro-2-furyl-méthyle, 1,4-dioxa-2-cyclohexyl-méthyle, allyle, (alkyl en $C_1-C_4$)-amino-(alkyle en $C_1-C_4$) ou di-(alkyl en $C_1-C_4$)-amino-(alkyle en $C_1-C_4$), $-CON(R')(R'')$ où R' représente l'hydrogène, un groupe alkyle en $C_1-C_6$, alkyle en $C_2-C_6$ substitué par un radical hydroxyle, alcoxy en $C_1-C_4$, hydroxyalcoxy en $C_2-C_6$, $-SO_3M$ ou mono- ou (dialkyl en $C_1-C_4$)-amino, un cyclohexyle, un benzyle, ou un phénéthyle; et R'' représente l'hydrogène, un groupe alkyle en $C_1-C_6$, alkyle en $C_2-C_6$ substitué par un radical hydroxyle, alcoxy en $C_1-C_4$ ou hydroxyalcoxy en $C_2-C_6$,

$-SO_2N(R')(R'')$ où R' et R'' sont tels que définis ci-dessus,

$-SO_3M$, $R_x-SO_2$ où $R_x$ représente un groupe alkyle en $C_1-C_6$, alkyle en $C_2-C_6$ substitué par un alcoxy en $C_1-C_4$ ou un (alcoxy en $C_1-C_4$)-(alcoxy en $C_2-C_6$), un groupe phényle ou benzyle, ou ester dialkylique d'acide phosphonique.

$R'_2$ est l'hydrogène ou un groupe alkyle en $C_1-C_6$,

M est l'hydrogène ou un cation non chromophore habituel dans les azurants optiques, et

$Q_2$ représente un radical répondant à la formule

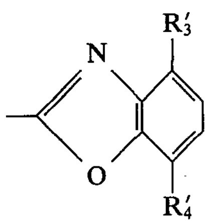 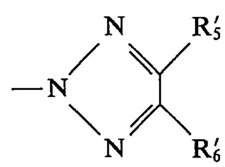 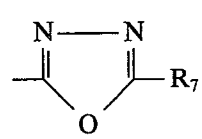 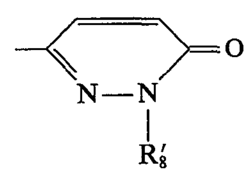

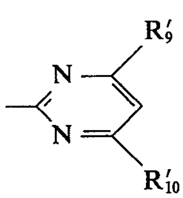 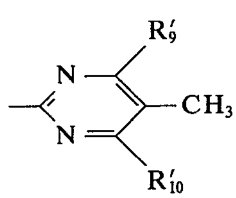 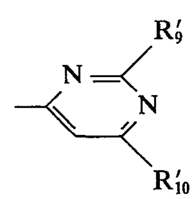 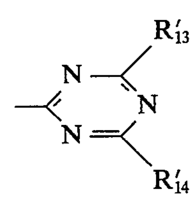

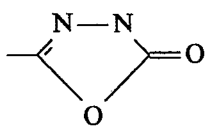 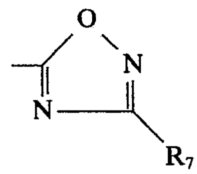 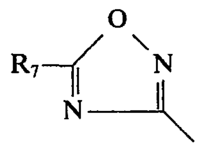 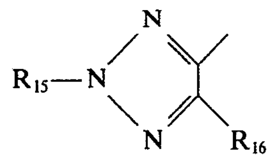

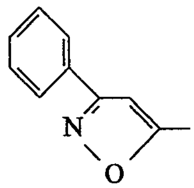 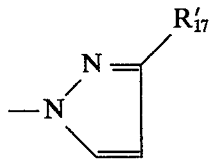 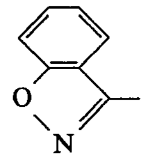 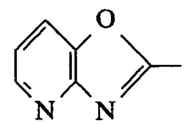 ou

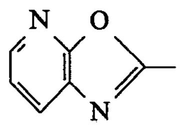

dans laquelle

$R'_3$ est l'hydrogène, un méthyle, le chlore ou un alcoxy en $C_1$—$C_4$,

$R'_4$ est l'hydrogène, le chlore, un groupe alkyle en $C_1$—$C_4$, phényle, alcoxy en $C_1$—$C_4$ ou phénoxy,

$R'_5$ est l'hydrogène, un groupe alkyle en $C_1$—$C_4$, phényle ou styryle ou en association avec $R'_6$ avec un noyau benzène obtenu par condensation, non substitué ou substitué par un groupe alkyle en $C_1$—$C_4$ ou du chlore ou un noyau naphtalénique obtenu par condensation,

$R'_6$ est l'hydrogène, un groupe alkyle en $C_1$—$C_4$, phényle ou en association avec $R'_5$, un noyau benzénique obtenu par condensation, non substitué ou substitué par un groupe alkyle en $C_1$—$C_4$, alcoxy en $C_1$—$C_4$ ou du chlore, ou un noyau naphtalénique obtenu par condensation,

$R_7$ est un groupe alkyle en $C_1$—$C_4$ non substitué ou substitué par un groupe non-chromophore, un groupe phényle éventuellement substitué par un radical alkyle en $C_1$—$C_4$, alcoxy en $C_1$—$C_4$, (alcoxy en $C_1$—$C_4$)carbonyle, cyano ou du chlore, un groupe styryle, biphénylyle ou naphtyle,

$R'_8$ est un groupe alkyle en $C_1$—$C_4$ non substitué ou phényle,

$R'_9$ est de l'hydrogène, un groupe alkyle en $C_1$—$C_4$ non substitué, phényle non substitué ou substitué par du chlore ou du méthyle, le chlore, un groupe alcoxy en $C_1$—$C_4$, alcoxyalcoxy en $C_3$—$C_5$, phénoxy non substitué, ou substitué par du chlore ou du méthyle, un groupe (alkyl en $C_1$—$C_4$)-thio ou phénylthio,

$R'_{10}$ est de l'hydrogène, un groupe alkyle en $C_1$—$C_4$ non substitué, alcoxy en $C_1$—$C_4$, phényle non substitué ou substitué par du chlore ou du méthyle, phénoxy non substitué ou substitué par du chlore ou du méthyle, (alkyle en $C_1$—$C_4$)-thio, phénylthio ou du chlore,

$R'_{13}$ et $R'_{14}$ sont l'hydrogène, un groupe (alkyl en $C_1$—$C_4$)-amino, di-(alkyl en $C_1$—$C_4$)-amino, phényle, morpholino, pipéridino, phénylamino, ou un radical $-(OCH_2-CH_2)_q-OY$ dans lequel Y est l'hydrogène, un groupe alkyle en $C_1$—$C_4$, benzyle, ou phényle et q est un entier compris entre 0 et 7,

$R_{15}$ est un phényle non substitué ou substitué par un groupe alkyle en $C_1$—$C_4$ ou du chlore,

$R_{16}$ est l'hydrogène ou un groupe alkyle en $C_1$—$C_4$, et

$R'_{17}$ est un groupe alkyle en $C_1$—$C_4$.

4. 4-hétérocyclyl-4'-vinyl-stilbènes selon la revendication 3 répondant à la formule

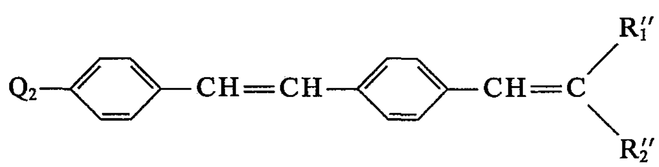

dans laquelle $R''_1$ est un groupe cyano, COOR où R est un groupe alkyle en $C_1$—$C_4$, (alcoxy en $C_1$—$C_4$)-(alkyle en $C_2$—$C_4$), (alcoxy en $C_1$—$C_4$)-(alcoxy en $C_2$—$C_4$)-(alkyle en $C_2$—$C_4$), (alcoxy en $C_1$—$C_4$)-(alcoxy en $C_2$—$C_4$)-(alcoxy en $C_2$—$C_4$)-(alkyle en $C_2$—$C_4$), 2,3,4,5-tétrahydro-2-furyl-méthyle, 1,4-dioxa-2-cyclohexyl-méthyle,

CON(R')(R''), où R' est l'hydrogène, un groupe alkyle en $C_1$—$C_6$, alkyle en $C_2$—$C_6$ substitué par un hydroxyle, un radical alcoxy en $C_1$—$C_4$, hydroxyalcoxy en $C_2$—$C_6$, $-SO_3M$ ou mono-ou di-(alkyl en $C_1$—$C_4$)-amino, un groupe cycloalkyle, benzyle ou phénéthyle, et R'' est l'hydrogène, un groupe alkyle en $C_1$—$C_6$, alkyle en $C_2$—$C_6$ substitué par un radical hydroxyle, alcoxy en $C_1$—$C_4$ ou hydroxyalcoxy en $C_2$—$C_6$, un radical $R_x-SO_2-$ où $R_x$ est un groupe alkyle en $C_1$—$C_6$, alkyle en $C_2$—$C_6$ substitué par un radical alcoxy en $C_1$—$C_4$ ou (alcoxy en $C_1$—$C_4$)-(alcoxy en $C_2$—$C_6$), un groupe phényle ou benzyle ou ester dialkylique de l'acide phosphonique,

$R_2''$ est l'hydrogène ou un groupe alkyle en $C_1$—$C_4$,
M est l'hydrogène ou un cation non chromophore habituel dans les azurants optiques, et
$Q_2$ est tel que défini ci-dessus.

5. 4-hétérocyclyl-4'-vinyl-stilbènes selon la revendication 4, répondant à la formule

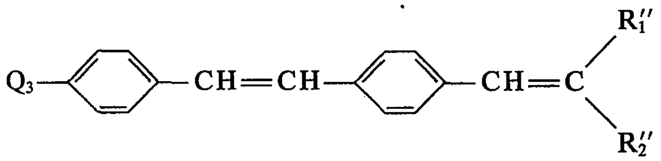

dans laquelle $R_1''$ et $R_2''$ sont tels que définis dans la revendication 4, et $Q_3$ est un radical de formule

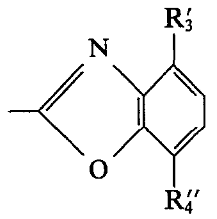
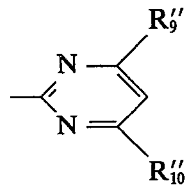
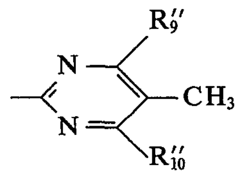
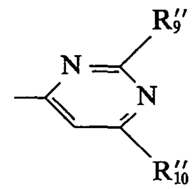

ou 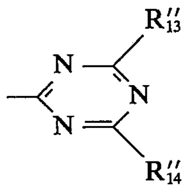

dans laquelle

$R_3'$ est l'hydrogène, un méthyle, le chlore ou un alcoxy en $C_1$—$C_4$,
$R_4''$ est l'hydrogène, un méthyle, le chlore ou un alkyle en $C_1$—$C_4$,
$R_9''$ est l'hydrogène, un groupe méthyle, phényle, alcoxy en $C_1$—$C_3$, méthoxyéthoxy ou phénoxy,
$R_{10}''$ est un groupe alkyle en $C_1$ ou en $C_2$ non substitué, alcoxy en $C_1$—$C_3$ ou phénoxy, et
$R_{13}''$ et $R_{14}''$ sont chacun l'hydrogène ou un radical $-(OCH_2-CH_2)_r-OY'$ dans lequel Y' est un groupe alkyle en $C_1$—$C_4$, et r est un entier compris entre 0 et 2,

6. 4-hétérocyclyl-4'-vinyl-stilbènes selon la revendication 5, répondant à la formule

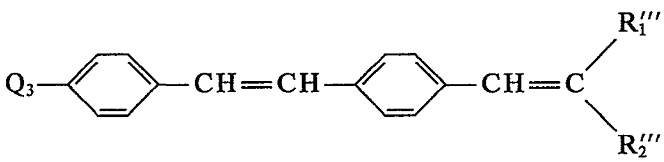

dans laquelle $R_1'''$ est un groupe cyano, COOR où R est un groupe alkyle en $C_1$—$C_4$, (alcoxy en $C_1$—$C_4$)-(alkyle en $C_2$—$C_4$), (alcoxy en $C_1$—$C_4$)-(alcoxy en $C_2$—$C_4$)-(alkyle en $C_2$—$C_4$), (alcoxy en $C_1$—$C_4$)-(alcoxy en $C_2$—$C_4$)-(alcoxy en $C_2$—$C_4$)-(alkyle en $C_2$—$C_4$), 2,3,4,5-tétrahydro-2-furyl-méthyle ou un 1,4-dioxa-2-cyclohexyl-méthyle,
ou un groupe $R_x-SO_2$, où $R_x$ est un groupe alkyle en $C_1$—$C_4$, alkyle en $C_2$—$C_6$ substitué par un radical alcoxy en $C_1$—$C_4$ ou (alcoxy en $C_1$—$C_4$)-(alcoxy en $C_2$—$C_6$), un groupe phényle ou benzyle, et $R_2'''$ est l'hydrogène ou un groupe alkyle en $C_1$—$C_4$, et $Q_3$ est tel que défini dans la revendication 5.

7. 4-hétérocyclyl-4'-vinyl-stilbènes selon la revendication 5, de formule

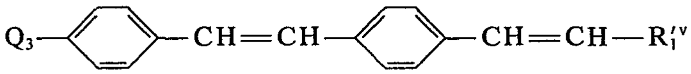

dans laquelle $R_1^{iv}$ est un groupe cyano ou COOR', où R' est un groupe alkyle en $C_1$—$C_4$ ou (alcoxy en $C_1$—$C_4$)-(alkyle en $C_2$—$C_6$), et $Q_3$ est tel que défini dans la revendication 5.

8. 4-hétérocyclyl-4'-vinyl-stilbène selon la revendication 4, de formule

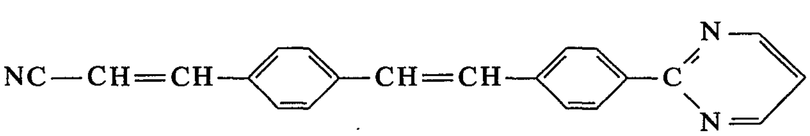

9. 4-hétérocyclyl-4'-vinyl-stilbène selon la revendication 6, de formule

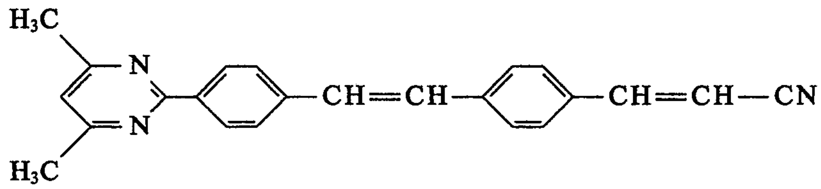

10. 4-hétérocyclyl-4'-vinyl-stilbène selon la revendication 6, de formule

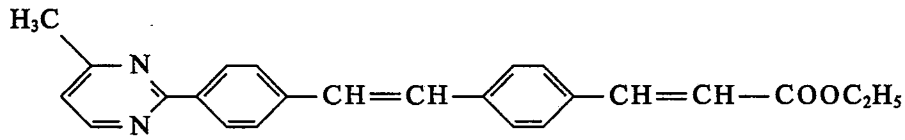

11. 4-hétérocyclyl-4'-vinyl-stilbène selon la revendication 6, de formule

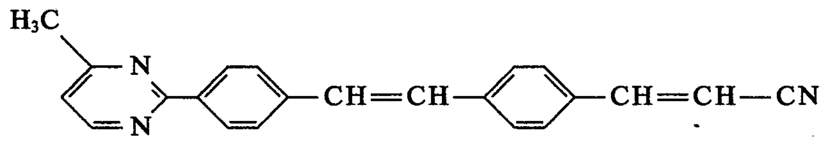

12. 4-hétérocyclyl-4'-vinyl-stilbène selon la revendication 6, de formule

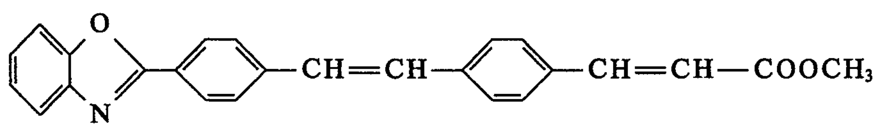

13. Procédé de préparation des 4-hétérocyclyl-4'-vinyl-stilbènes répondant à la formule

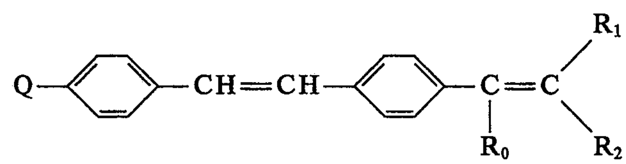

dans laquelle Q est un noyau hétérocyclique aromatique, monocyclique penta ou hexagonal, non substitué ou substitué par un groupe non-chromophore, ayant 0, 1 ou 2 noyaux benzèniques fixés par condensation, ou bien un noyau hétérocyclique aromatique, bicyclique à neuf chainons; $R_0$ représente l'hydrogène, un alkyle non substitué ou substitué par un groupe non-chromophore; $R_1$ représente l'hydrogène, un groupe alkyle non substitué ou substitué par un radical non-chromophore, un groupe alcoxycarbonyle, carbamoyle ou sulfonamido; alcényle, carboxyle, alkyl-, aryl- ou aralkyl-sulfonyle, cyano, sulfo ou ester dialkylique de l'acide phosphonique, et $R_2$ représente l'hydrogène, un alkyle non substitué ou substitué par un groupe non-chromophore ou un alcényle, étant entendu que tout au plus un des $R_1$ et $R_2$ représentent l'hydrogène, caractérisé en ce que, en présence d'un agent de condensation basique, on condense un composé répondant à la formule

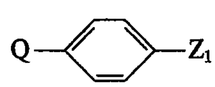

avec un composé de formule

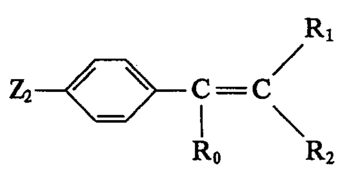

dans laquelle Q, $R_0$, $R_1$ et $R_2$ sont tels que définis ci-dessus, et dans laquelle $Z_1$ et $Z_2$ sont l'un le groupe OHC et l'autre un groupement de formule

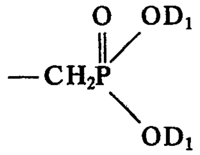 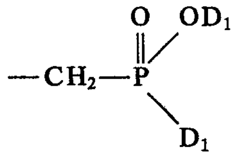 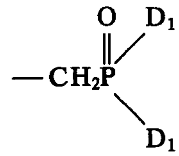 ou 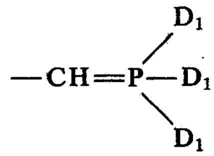

dans laquelle $D_1$ est un radical alkyle, aryle, cycloalkyle ou aralkyle non substitués ou substitués.

14. Procédé d'azurage optique de matières organiques naturelles, semi-synthétiques, et synthétiques à poids moléculaire élevé, caractérisé en ce que des 4-hétérocyclyl-4'-vinyl-stilbènes, selon la définition dans l'une des revendications 1 à 7, sont incorporés dans ces matières ou appliqués sur leurs surfaces dans une proportion de 0,001 à 2% par rapport à la matière à blanchir.

15. Procédé selon la revendication 14, destiné à l'azurage optique de matières organiques à poids moléculaire élevé.

16. Procédé selon la revendication 14, destiné à l'azurage optique de polyesters.

17. Mélange d'azurants contenant un 4-hétérocyclyl-4'-vinyl-stilbène selon les revendications 1 à 7, et un azurant approprié pour polyester dans une proportion de 1 : 9 à 9 : 1.

18. Mélange d'azurants selon la revendication 17, contenant un 4-hétérocyclyl-4'-vinyl-stilbène de formule

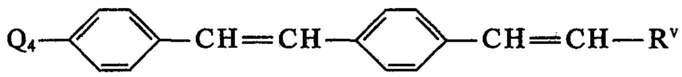

dans laquelle $Q_4$ est un radical Benzoxazol-2-yle, pyrimidin-2-yle, 4-méthyl-pyrimidin-2-yle- ou 4,6-diméthyl-pyrimidin-2-yle, et $R^V$ est un alcoxycarbonyle en $C_2$—$C_4$ ou un groupe cyano, ainsi que en qualité d'azurants appropriés pour le polyester, le 1,4-bis-(benzoxazol-2'-yl)-naphtalène, le 4,4'-bis-(éthoxycarbonyl-vinyl)-stilbène, le 4,4'-bis-(cyanovinyl)-stilbène, le 1,4-bis-(2'-cyano-styryl)-benzène, le 1,5-bis-(benzoxazol-2'-yl)-thiophène, le 1-phényl-4-(5',7'-diméthyl-benzoxazol-2'-yl)-stilbène, le 1,2-bis-(5'-méthyl-benzoxazol-2'-yl)-vinylène, le 4-(benzoxazol-2'-yl)-4'-(3"-méthyl-1",2",4"-oxadiazol-5"-yl)-stilbène et le 2,4-diméthoxytriazin-2-yl-pyrène dans une proportion de 1 : 2 ou de 2 : 1.

19. Mélange d'azurants selon la revendication 18, contenant l'azurant de formule

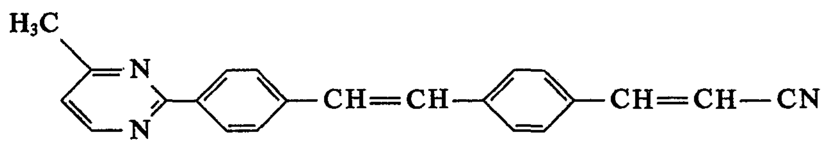

et l'azurant de formule

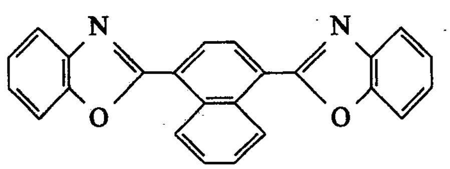

dans une proportion de 1 : 2 ou 2 : 1.

20. Mélange d'azurants selon la revendication 17, contenant l'azurant de formule

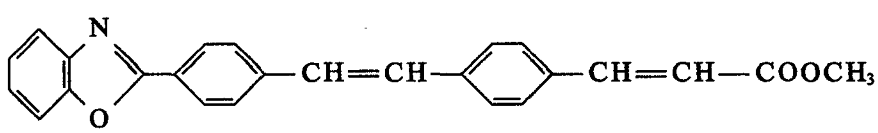

et l'azurant de formule

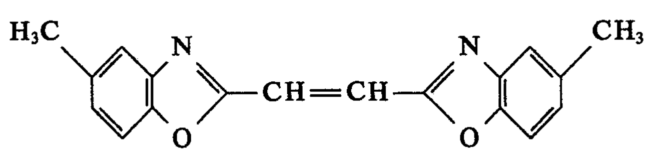

dans une proportion de 1 : 2 ou 2 : 1.